(19) Europäisches Patentamt
European Patent Office
Office européen des brevets

(11) Publication number : **0 609 625 A1**

(12) # EUROPEAN PATENT APPLICATION

(21) Application number : **93310355.8**

(22) Date of filing : **20.12.93**

(51) Int. Cl.$^5$ : **C07D 217/06,** C07D 401/12, C07D 217/14, C07D 215/12, C07D 241/42, C07D 213/40, A61K 31/50, A61K 37/02

(30) Priority : **22.12.92 US 995621**

(43) Date of publication of application : **10.08.94 Bulletin 94/32**

(84) Designated Contracting States : **AT BE CH DE DK ES FR GB GR IE IT LI LU NL PT SE**

(71) Applicant : **ELI LILLY AND COMPANY**
**Lilly Corporate Center**
**Indianapolis Indiana 46285 (US)**

(72) Inventor : **Dressman, Bruce Anthony**
**4337 Caledonia Way**
**Indianapolis, Indiana 46254 (US)**
Inventor : **Hammond, Marlys**
**2042 Layton Street**
**Pasadena, California 91104 (US)**
Inventor : **Kaldor, Stephen Warren**
**7435 Burkwood Way**
**Indianapolis, Indiana 46254 (US)**

(74) Representative : **Tapping, Kenneth George et al**
**Erl Wood Manor**
**Windlesham Surrey, GU20 6PH (GB)**

(54) **Inhibitors of HIV protease useful for the treatment of AIDS.**

(57) The present invention provides novel HIV protease inhibitors, pharmaceutical formulations containing those compounds and methods of treating and/or preventing HIV infection and/or AIDS.

EP 0 609 625 A1

The present invention relates to compounds of formula I, below, and pharmaceutically acceptable salts thereof that inhibit the protease encoded by human immunodeficiency virus (HIV) type 1 (HIV-1) or type 2 (HIV-2). These compounds are useful in the treatment or prevention of infection by HIV and the treatment or prevention of the resulting acquired immune deficiency syndrome (AIDS). The compounds, their pharmaceutically acceptable salts, and the pharmaceutical compositions of the present invention can be used alone or in combination with other antivirals, immunomodulators, antibiotics or vaccines. Methods of treating or preventing AIDS, methods of treating or preventing HIV infection and methods of inhibiting HIV replication are disclosed.

A retrovirus designated human immuno-deficiency virus (HIV) is the causative agent of the complex disease termed Acquired Immune Deficiency Syndrome (AIDS), and is a member of the lentivirus family of retroviruses. M. A. Gonda, F. Wong-Staal, R. C. Gallo, "Sequence Homology and Morphological Similarity of HTLV III And Visna Virus, A Pathogenic Lentivirus", Science, 227, 173, (1985); P. Sonigo, N. Alizon, et al., "Nucleotide Sequence of the Visna Lentivirus: Relationship to the AIDS Virus", Cell, 42, 369, (1985). The complex disease AIDS includes progressive destruction of the immune system and degeneration of the central and peripheral nervous systems. The HIV virus was previously known or referred to as LAV, HTLV-III or ARV.

A common feature of retrovirus replication is the post-translational processing of precursor polyproteins by a virally encoded protease to generate mature viral proteins required for viral assembly and function. Interruption of this processing appears to prevent the production of normally infectious virus. Unprocessed structural proteins also have been observed in clones of non-infectious HIV strains isolated from human patients. The results suggest that the inhibition of HIV protease represents a viable method for the treatment or prevention of AIDS and/or the treatment or prevention of infection by HIV.

The HIV genome encodes structural protein precursors known as gag and pol, which are processed to afford the protease, reverse transcriptase and endonuclease/integrase. The protease further cleaves gag and gag-pol polyproteins to yield mature structural proteins of the virus core.

Considerable efforts are being directed toward the control of HIV by means of the structural protein precursors which are processed to yield the retroviral protease, reverse transcriptase and endonuclease/integrase. For example, a currently used therapeutic, AZT, is an inhibitor of the viral reverse transcriptase. H. Mitsuya, NS. Broder, "Inhibition of the In Vitro Infectivity in Cytopathic Effects of HTLV III", Proc. Natl. Acad. Sci. USA, 83, 1911 (1986).

Research efforts have also been directed toward HIV protease inhibitors. For example, European Patent Application 346 847 discloses compounds which are said to be useful as HIV protease inhibitors.

Unfortunately, many of the known HIV protease inhibitors suffer from toxicity problems, lack of bioavailability or short *in vivo* half-lives. In particular, it is believed that oral bioavailability is a necessary characteristic of an HIV protease inhibitor due to the chronic nature of the disease. However, peptides and peptide mimetics are notorious for their inability to be orally absorbed. Thus, despite the recognized therapeutic potential associated with a protease inhibitor and the research efforts expended thus far, a viable therapeutic agent has not yet emerged.

Accordingly, a primary object of the present invention is to provide novel HIV protease inhibitors which are useful in the treatment or prevention of HIV infection and/or the resulting acquired immune deficiency syndrome (AIDS).

The present invention provides a compound of formula I

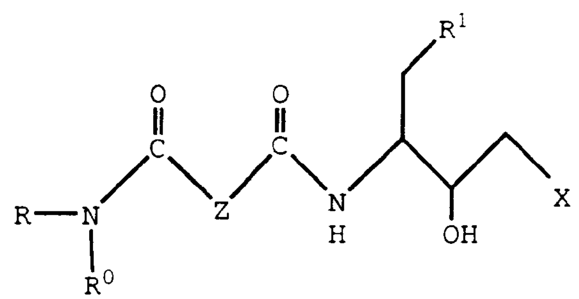

I

wherein:

R and $R^0$ are independently selected from hydrogen, $C_1$-$C_6$ alkyl, aryl($C_1$-$C_4$)alkyl, heterocycle($C_1$-$C_4$)alkyl or unsaturated heterocycle($C_1$-$C_4$)alkyl;

or R and $R^0$ are combined with the nitrogen atom to which they are attached to form a heterocycle or unsaturated heterocycle with the proviso that the nitrogen atom may not be quaternized;

Z is a group having the structure:

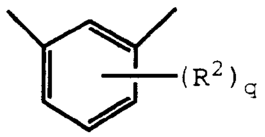 , 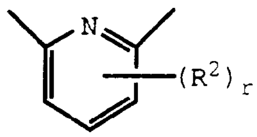 , 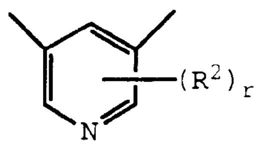 ,

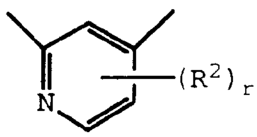 , 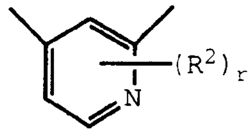 , 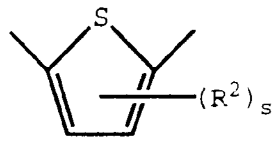 ,

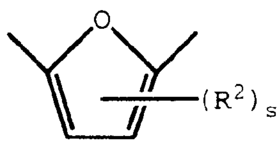 or 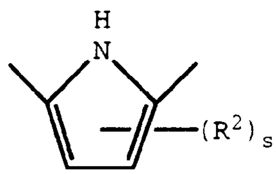 ;

q is 0, 1, 2, 3 or 4;

r is 0, 1, 2 or 3;

s is 0, 1 or 2;

$R^2$ is hydrogen, halo, $C_1$-$C_6$ alkyl, aryl($C_1$-$C_4$)alkyl, $C_1$-$C_6$ alkoxy, $C_1$-$C_6$ alkylthio, amino, $C_1$-$C_4$ alkylamino, di($C_1$-$C_4$)alkylamino, carbamoyl or N-($C_1$-$C_4$)alkylcarbamoyl;

$R^1$ is aryl, heterocycle, unsaturated heterocycle, $C_5$-$C_7$ cycloalkyl or -S-$R^{1a}$, where $R^{1a}$ is aryl, heterocycle, unsaturated heterocycle or $C_5$-$C_7$ cycloalkyl;

X is a group having the structure:

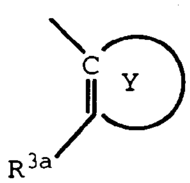 , 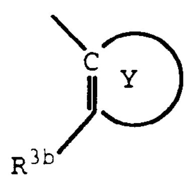 or 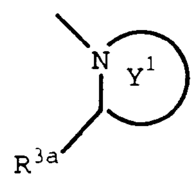 ;

Y is aryl or unsaturated heterocycle;

$Y^1$ is heterocycle;

$R^{3a}$ is a group having the structure:

1) -C(O)-$NR^4R^4$,

2) 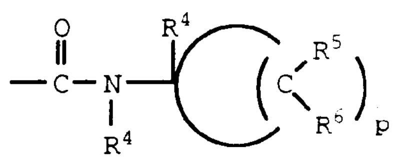 ,

or

3) 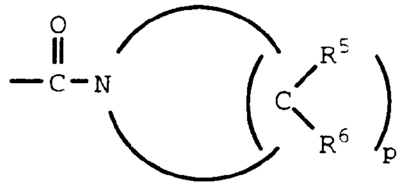 ;

$R^{3b}$ is a group having the structure:

1) 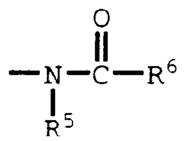 ,

2) 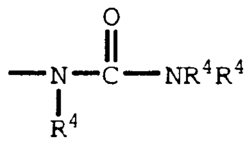 ,

or

3) 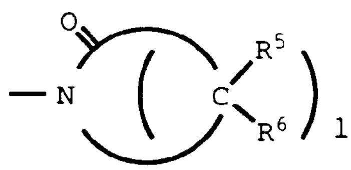 ;

p is 4 or 5;

l is 3, 4 or 5;

$R^4$ at each occurrence is independently hydrogen, $C_1$-$C_6$ alkyl or hydroxy($C_1$-$C_4$)alkyl; and

$R^5$ and $R^6$ are independently selected from hydrogen, hydroxy, $C_1$-$C_6$ alkyl, $C_1$-$C_6$ alkoxy, amino, $C_1$-$C_4$ alkylamino, hydroxy($C_1$-$C_4$)alkyl, carboxy, $C_1$-$C_4$ alkoxycarbonyl, carbamoyl, N-($C_1$-$C_4$)alkylcarbamoyl, aryl, heterocycle or unsaturated heterocycle;

or a pharmaceutically acceptable salt thereof.

All temperatures stated herein are in degrees Celsius (°C). All units of measurement employed herein are in weight units except for liquids which are in volume units.

As used herein, the term "$C_1$-$C_6$ alkyl" represents a straight or branched alkyl chain having from one to six carbon atoms. Typical $C_1$-$C_6$ alkyl groups include methyl, ethyl, propyl, isopropyl, butyl, isobutyl, *sec*-butyl, *t*-butyl, pentyl, *neo*-pentyl, hexyl and the like. The term "$C_1$-$C_6$ alkyl" includes within its definition the term "$C_1$-$C_4$ alkyl".

"Halo" represents chloro, fluoro, bromo or iodo.

"Halo($C_1$-$C_4$)alkyl" represents a straight or branched alkyl chain having from one to four carbon atoms with 1-3 halogen atoms attached to it. Typical halo($C_1$-$C_4$)alkyl groups include chloromethyl, 2-bromoethyl, 1-chloroisopropyl, 3-fluoropropyl, 2,3-dibromobutyl, 3-chloroisobutyl, iodo-*t*-butyl, trifluoromethyl and the like.

"Hydroxy($C_1$-$C_4$)alkyl" represents a straight or branched alkyl chain having from one to four carbon atoms with a hydroxy group attached to it. Typical hydroxy($C_1$-$C_4$)alkyl groups include hydroxymethyl, 2-hydroxyethyl, 3-hydroxypropyl, 2-hydroxyisopropyl, 4-hydroxybutyl and the like.

"$C_1$-$C_6$ alkylthio" represents a straight or branched alkyl chain having from one to four carbon atoms attached to a sulfur atom. Typical $C_1$-$C_6$ alkylthio groups include methylthio, ethylthio, propylthio, isopropylthio, butylthio, *sec*-butylthio, *t*-butylthio, pentylthio, hexylthio and the like.

"$C_1$-$C_4$ alkylamino" represents a straight or branched alkyl chain having from one to four carbon atoms attached to an amino group. Typical $C_1$-$C_4$ alkylamino groups include methylamino, ethylamino, propylamino, isopropylamino, butylamino, *sec*-butylamino and the like.

"Di($C_1$-$C_4$)alkylamino" represents two straight or branched alkyl chains having from one to four carbon atoms attached to a common amino group. Typical di($C_1$-$C_4$)alkylamino groups include dimethylamino, ethylmethylamino, methylpropylamino, ethylisopropylamino, butylmethylamino, sec-butylethylamino and the like.

"$C_1$-$C_6$ alkoxy" represents a straight or branched alkyl chain having from one to six carbon atoms attached to an oxygen atom. Typical $C_1$-$C_6$ alkoxy groups include methoxy, ethoxy, propoxy, isopropoxy, butoxy, *sec*-butoxy, *t*-butoxy, pentoxy, hexoxy and the like. The term "$C_1$-$C_6$ alkoxy" includes within its definition the term "$C_1$-$C_4$ alkoxy".

"$C_1$-$C_4$ alkoxycarbonyl" represents a straight or branched alkoxy chain having from one to four carbon atoms attached to a carbonyl moiety. Typical $C_1$-$C_4$ alkoxycarbonyl groups include methoxycarbonyl, ethoxycarbonyl, propoxycarbonyl, isopropoxycarbonyl, butoxycarbonyl and the like.

"N-($C_1$-$C_4$)alkylcarbamoyl" represents a straight or branched alkyl chain having from one to four carbon atoms attached to the nitrogen atom of a carbamoyl moiety. Typical N-($C_1$-$C_4$)alkylcarbamoyl groups include N-methylcarbamoyl, N-ethylcarbamoyl, N-propylcarbamoyl, N-isopropylcarbamoyl, N-butylcarbamoyl and N-*t*-butylcarbamoyl and the like.

"$C_5$-$C_7$ cycloalkyl" represents a saturated hydrocarbon ring structure containing from five to seven carbon atoms which is unsubstituted or substituted with 1, 2 or 3 substituents independently selected from halo, halo($C_1$-$C_4$)alkyl, $C_1$-$C_4$ alkyl, $C_1$-$C_4$ alkoxy, carboxy, $C_1$-$C_4$ alkoxycarbonyl, carbamoyl, N-($C_1$-$C_4$)alkylcarbamoyl, amino, $C_1$-$C_4$ alkylamino, di($C_1$-$C_4$)alkylamino or a group having the structure $-(CH_2)_a-R^7$ where a is 1, 2, 3 or 4 and $R^7$ is hydroxy, $C_1$-$C_4$ alkoxy, carboxy, $C_1$-$C_4$ alkoxycarbonyl, amino, carbamoyl, $C_1$-$C_4$ alkylamino or di($C_1$-$C_4$)alkylamino. Typical cycloalkyl groups include cyclopentyl, cyclohexyl, cycloheptyl, 3-methylcyclopentyl, 4-ethoxycyclohexyl, 5-carboxycyclo-heptyl, 6-chlorocyclohexyl and the like.

The term "heterocycle" represents an unsubstituted or substituted stable 5- to 7-membered monocyclic or 7- to 10-membered bicyclic heterocyclic ring which is saturated and which consists of carbon atoms and from one to three heteroatoms selected from the group consisting of nitrogen, oxygen or sulfur, and wherein any nitrogen and sulfur heteroatoms may optionally be oxidized, and any nitrogen heteroatom may optionally be quaternized and including a bicyclic group in which any of the above-defined heterocyclic rings is fused to a benzene ring. The heterocyclic ring may be attached at any heteroatom or carbon atom which affords a stable structure. The heterocycle is unsubstituted or substituted with 1, 2 or 3 substituents independently selected from halo, halo($C_1$-$C_4$)alkyl, $C_1$-$C_4$ alkyl, $C_1$-$C_4$ alkoxy, carboxy, $C_1$-$C_4$ alkoxycarbonyl, carbamoyl, N-($C_1$-$C_4$)alkylcarbamoyl, amino, $C_1$-$C_4$ alkylamino, di($C_1$-$C_4$)alkylamino or a group having the structure $-(CH_2)_a-R^7$ where a is 1, 2, 3 or 4 and $R^7$ is hydroxy, $C_1$-$C_4$ alkoxy, carboxy, $C_1$-$C_4$ alkoxycarbonyl, amino, carbamoyl, $C_1$-$C_4$ alkylamino or di($C_1$-$C_4$)alkylamino.

The term "unsaturated heterocycle" represents an unsubstituted or substituted stable 5- to 7-membered monocyclic or 7- to 10-membered bicyclic heterocyclic ring which has one or more double bonds and which consists of carbon atoms and from one to three heteroatoms selected from the group consisting of nitrogen, oxygen or sulfur, and wherein the nitrogen and sulfur heteroatoms may optionally be oxidized, and the nitrogen heteroatom may optionally be quarternized and including a bicyclic group in which any of the above-defined heterocyclic rings is fused to a benzene ring. The unsaturated heterocyclic ring may be attached at any heteroatom or carbon atom which affords a stable structure. The unsaturated heterocycle is unsubstituted or substituted with 1, 2 or 3 substituents independently selected from halo, halo($C_1$-$C_4$)alkyl, $C_1$-$C_4$ alkyl, $C_1$-$C_4$ alkoxy, carboxy, $C_1$-$C_4$ alkoxycarbonyl, carbamoyl, N-($C_1$-$C_4$)alkylcarbamoyl, amino, $C_1$-$C_4$ alkylamino, di($C_1$-$C_4$)alkylamino or a group having the structure $-(CH_2)_a-R^7$ where a is 1, 2, 3 or 4 and $R^7$ is hydroxy, $C_1$-$C_4$ alkoxy, carboxy, $C_1$-$C_4$ alkoxycarbonyl, amino, carbamoyl, $C_1$-$C_4$ alkylamino or di($C_1$-$C_4$)alkylamino.

Examples of such heterocycles and unsaturated heterocycles include piperidinyl, piperazinyl, azepinyl, pyrrolyl, pyrrolidinyl, pyrazolyl, pyrazolidinyl, imidazolyl, imidazolinyl, imidazolidinyl, pyridyl, pyrazinyl, pyrimidinyl, pyridazinyl, oxazolyl, oxazolidinyl, isoxazolyl, isoxazolidinyl, morpholinyl, thiazolyl, thiazolidinyl, isothiazolyl, quinuclidinyl, isothiazolidinyl, indolyl, quinolinyl, isoquinolinyl, benzimidazolyl, thiadiazolyl, benzopyranyl, benzothiazolyl, benzoazolyl, furyl, tetrahydrofuryl, tetrahydropyranyl, thienyl, benzothienyl, thiamorpholinyl, thiamorpholinylsulfoxide, thiamorpholinylsulfone, oxadiazolyl, triazolyl, tetrahydroquinolinyl, tetrahydrisoquinolinyl, 3-methylimidazolyl, 3-methoxypyridyl, 4-chloroquinolinyl, 4-aminothiazolyl, 8-methylquinolinyl, 6-chloroquinoxalinyl, 3-ethylpyridyl, 6-methoxybenzimidazolyl, 4-hydroxyfuryl, 4-methylisoquinolinyl, 6,8-dibromoquinolinyl, 4,8-dimethylnaphthyl, 2-methyl-1,2,3,4-tetrahydroisoquinolinyl, N-methyl-quinolin-2-yl, 2-t-butoxycarbonyl-1,2,3,4- isoquinolin-7-yl and the like.

"Heterocycle($C_1$-$C_4$)alkyl" represents a straight or branched alkyl chain having from one to four carbon atoms with a heterocycle group attached to it. "Unsaturated heterocycle($C_1$-$C_4$)alkyl" represents a straight or branched alkyl chain having from one to four carbon atoms with an unsaturated heterocycle group attached to it. Typical heterocycle($C_1$-$C_4$)alkyl and unsaturated heterocycle($C_1$-$C_4$)alkyl groups include pyrrolylmethyl, quinolinylmethyl, 1-indolylethyl, 2-furylethyl, 3-thien-2-ylpropyl, 1-imidazolylisopropyl, 4-thiazolylbutyl and the

like.

"Aryl" represents a phenyl or naphthyl ring which is optionally substituted with 1, 2 or 3 substituents independently selected from halo, morpholino($C_1$-$C_4$)alkoxycarbonyl, pyridyl($C_1$-$C_4$)alkoxycarbonyl, halo($C_1$-$C_4$)alkyl, $C_1$-$C_4$ alkyl, $C_1$-$C_4$ alkoxy, carboxy, $C_1$-$C_4$ alkoxycarbonyl, carbamoyl, N-($C_1$-$C_4$)alkylcarbamoyl, amino, $C_1$-$C_4$ alkylamino, di($C_1$-$C_4$)alkylamino or a group of the formula $-(CH_2)_a-R^7$ where a is 1, 2, 3 or 4; and $R^7$ is hydroxy, $C_1$-$C_4$ alkoxy, carboxy, $C_1$-$C_4$ alkoxycarbonyl, amino, carbamoyl, $C_1$-$C_4$ alkylamino or di($C_1$-$C_4$)alkylamino.

"Aryl($C_1$-$C_4$)alkyl" represents a straight or branched alkyl chain having from one to four carbon atoms with an aryl group attached to it. Typical aryl($C_1$-$C_4$)alkyl groups include phenylmethyl, 2-phenylethyl, 3-naphthylpropyl, 1-naphthylisopropyl, 4-phenylbutyl and the like.

The term "amino-protecting group" as used in the specification refers to substituents of the amino group commonly employed to block or protect the amino functionality while reacting other functional groups on the compound. Examples of such amino-protecting groups include formyl, trityl, phthalimido, trichloroacetyl, chloroacetyl, bromoacetyl, iodoacetyl; or urethane-type blocking groups such as benzyloxycarbonyl, 4-phenylbenzyloxycarbonyl, 2-methylbenzyloxycarbonyl, 4-methoxybenzyloxycarbonyl, 4-fluorobenzyloxycarbonyl, 4-chlorobenzyloxycarbonyl, 3-chlorobenzyloxycarbonyl, 2-chlorobenzyloxycarbonyl, 2,4-dichlorobenzyloxycarbonyl, 4-bromobenzyloxycarbonyl, 3-bromobenzyloxycarbonyl, 4-nitrobenzyloxycarbonyl, 4-cyanobenzyloxycarbonyl, 2-(4-xenyl)isopropoxycarbonyl, 1,1-diphenyleth-1-yloxycarbonyl, 1,1-diphenylprop-1-yloxycarbonyl, 2-phenylprop-2-yloxycarbonyl, 2-(*p*-toluyl)prop-2-yloxycarbonyl, cyclopentanyloxycarbonyl, 1-methylcyclopentanyloxycarbonyl, cyclohexanyloxycarbonyl, 1-methylcyclohexanyloxycarbonyl, 2-methylcyclohexanyloxycarbonyl, 2-(4-toluylsulfonyl)ethoxycarbonyl, 2-(methylsulfonyl)ethoxycarbonyl, 2-(triphenylphosphino)ethoxycarbonyl, fluorenylmethoxycarbonyl ("FMOC"), 2-(trimethylsilyl)ethoxycarbonyl, allyloxycarbonyl, 1-(trimethylsilylmethyl)prop-1-enyloxycarbonyl, 5-benzisoxalylmethoxycarbonyl, 4-acetoxybenzyloxycarbonyl, 2,2,2-trichloroethoxycarbonyl, 2-ethynyl-2-propoxycarbonyl, cyclopropylmethoxycarbonyl, 4-(decyloxy)benzyloxycarbonyl, isobornyloxycarbonyl, 1-piperidyloxycarbonyl and the like; or benzoylmethylsulfonyl, 2-nitrophenylsulfenyl, diphenylphosphine oxide and like amino-protecting groups. The species of amino-protecting group employed is not critical so long as the derivatized amino group is stable to the condition of subsequent reaction(s) on other positions of the intermediate molecule and can be selectively removed at the appropriate point without disrupting the remainder of the molecule including any other amino-protecting group(s). Preferred amino-protecting groups are *t*-butoxycarbonyl (*t*-Boc) and benzyloxycarbonyl (CbZ). Further examples of groups referred to by the above terms are described by J. W. Barton, "Protective Groups in Organic Chemistry", J. G. W. McOmie, Ed., Plenum Press, New York, N.Y., 1973, Chapter 2, and T. W. Greene, "Protective Groups in Organic Synthesis", John Wiley and sons, New York, N.Y., 1981, Chapter 7.

The term "carboxy-protecting group" as used in the specification refers to substituents of the carboxy group commonly employed to block or protect the carboxy functionality while reacting other functional groups on the compound. Examples of such carboxy-protecting groups include methyl, *p*-nitrobenzyl, *p*-methylbenzyl, *p*-methoxybenzyl, 3,4-dimethoxybenzyl, 2,4-dimethoxybenzyl, 2,4,6-trimethoxybenzyl, 2,4,6-trimethylbenzyl, pentamethylbenzyl, 3,4-methylene-dioxybenzyl, benzhydryl, 4,4'-dimethoxybenzhydryl, 2,2,4,4'-tetramethoxybenzhydryl, *t*-butyl, *t*-amyl, trityl, 4-methoxytrityl, 4,4'-dimethoxytrityl, 4,4',4"-trimethoxytrityl, 2-phenylprop-2-yl, trimethylsilyl, *t*-butyldimethylsilyl, phenacyl, 2,2,2-trichloroethyl, β-(di(*n*-butyl)methylsilyl)ethyl, *p*-toluenesulfonylethyl, 4-nitrobenzylsulfonylethyl, allyl, cinnamyl, 1-(trimethylsilylmethyl)prop-1-en-3-yl and like moieties. A preferred carboxy-protecting group is benzhydryl. Further examples of these groups are found in E. Haslam, "Protective Groups in Organic Chemistry", J.G.W. McOmie, Ed., Plenum Press, New York, N.Y., 1973, Chapter 5, and T.W. Greene, "Protective Groups in Organic Synthesis", John Wiley and Sons, New York, N.Y., 1981, Chapter 5.

The compounds of the present invention have at least two asymmetric centers denoted by an asterisk in the formula below:

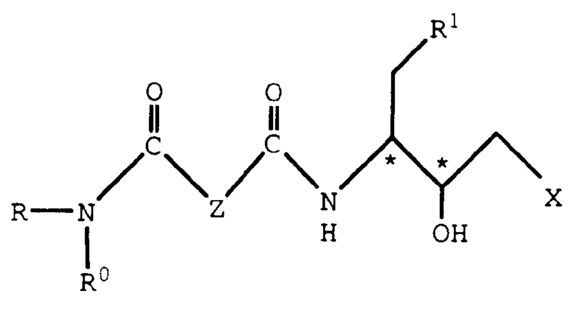

As a consequence of these asymmetric centers, the compounds of the present invention can occur as mixtures of diastereomers, racemic mixtures and as individual enantiomers. All asymmetric forms, individual isomers and combinations thereof, are within the scope of the present invention.

As mentioned above, the invention includes the pharmaceutically acceptable salts of the compounds defined by formula I. Although generally neutral, a compound of this invention may possess a sufficiently acidic, a sufficiently basic, or both functional groups, and accordingly react with any of a number of inorganic bases, and inorganic and organic acids, to form a pharmaceutically acceptable salt.

The term "pharmaceutically acceptable salt" as used herein, refers to salts of the compounds of the above formula which are substantially non-toxic to living organisms. Typical pharmaceutically acceptable salts include those salts prepared by reaction of the compounds of the present invention with a mineral or organic acid or an inorganic base. Such salts are known as acid addition and base addition salts.

Acids commonly employed to form acid addition salts are inorganic acids such as hydrochloric acid, hydrobromic acid, hydroiodic acid, sulfuric acid, phosphoric acid, and the like, and organic acids such as p-toluenesulfonic, methanesulfonic acid, oxalic acid, p-bromophenylsulfonic acid, carbonic acid, succinic acid, citric acid, benzoic acid, acetic acid, and the like.

Examples of such pharmaceutically acceptable salts are the sulfate, pyrosulfate, bisulfate, sulfite, bisulfite, phosphate, monohydrogenphosphate, dihydrogenphosphate, metaphosphate, pyrophosphate, chloride, bromide, iodide, acetate, propionate, decanoate, caprylate, acrylate, formate, isobutyrate, caproate, heptanoate, propiolate, oxalate, malonate, succinate, suberate, sebacate, fumarate, maleate, butyne-1,4-dioate, hexyne-1,6-dioate, benzoate, chlorobenzoate, methylbenzoate, dinitrobenzoate, hydroxybenzoate, methoxybenzoate, phthalate, sulfonate, xylenesulfonate, phenylacetate, phenylpropionate, phenylbutyrate, citrate, lactate, γ-hydroxybutyrate, glycollate, tartrate, methanesulfonate, propanesulfonate, naphthalene-1-sulfonate, napththalene-2-sulfonate, mandelate and the like. Preferred pharmaceutically acceptable acid addition salts are those formed with mineral acids such as hydrochloric acid and hydrobromic acid, and those formed with organic acids such as maleic acid and methanesulfonic acid.

Base addition salts include those derived from inorganic bases, such as ammonium or alkali or alkaline earth metal hydroxides, carbonates, bicarbonates, and the like. Such bases useful in preparing the salts of this invention thus include sodium hydroxide, potassium hydroxide, ammonium hydroxide, potassium carbonate, sodium carbonate, sodium bicarbonate, potassium bicarbonate, calcium hydroxide, calcium carbonate and the like. The potassium and sodium salt forms are particularly preferred.

It should be recognized that the particular counterion forming a part of any salt of this invention is not of a critical nature, so long as the salt as a whole is pharmacologically acceptable and as long as the counterion does not contribute undesired qualities to the salt as a whole.

Preferred compounds of this invention are those compounds of formula I where:

Z is

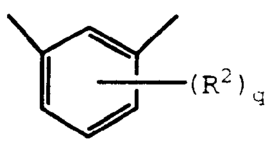

;

$R^1$ is aryl or -S-$R^{1a}$ where $R^{1a}$ is aryl;

$R^2$ is hydrogen or $C_1$-$C_6$ alkyl;

q is 0 or 1;

X is

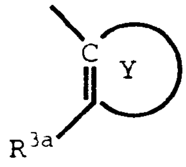

or

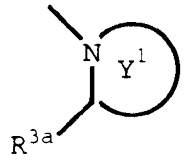

;

and

$R^{3a}$ is -C(O)-N$R^4R^4$, where $R^4$ is as defined above; or a pharmaceutically acceptable salt thereof.

Of these preferred compounds, more preferred are those compounds where:

Y is phenyl;

$Y^1$ is decahydro-(4aS, 8aS)-isoquinolinyl; and

$R^3$ is -C(O)NH(*t*-butyl);

or a pharmcaceutically acceptable salt thereof.

Of these preferred compounds, especially preferred are those compounds wherein:

R and $R^0$ are combined with the nitrogen atom to which they are attached to form a heterocycle or unsaturated heterocycle with the proviso that the nitrogen atom is not quaternized;

or a pharmaceutically acceptable salt thereof.

The most preferred compounds are:

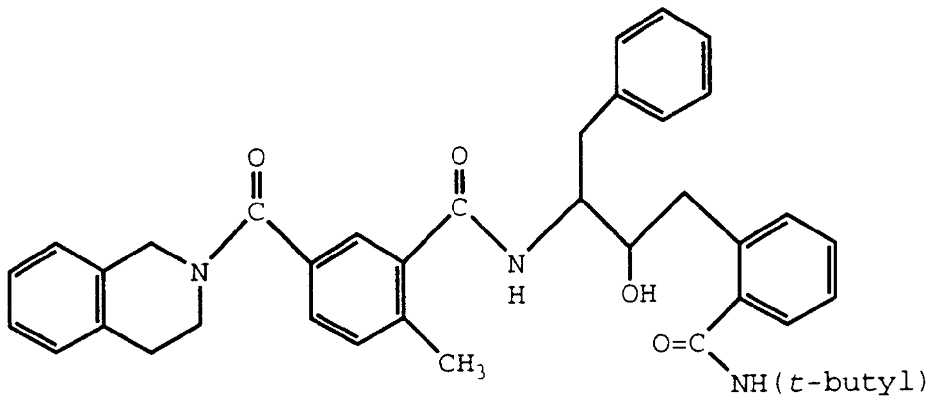

[3'''S-*(3'''R*, 4'''S*)]*-N-[1'-oxo-1'-(3''-[1'''-oxo-2'''-aza-3'''-phenylmethyl-4'''-hydroxy-5'''-(2''''-N-*t*-butylcarbamido)phenyl]pentyl-4''-methyl)phenyl]methyl-1,2,3,4-tetrahydroisoquinoline;

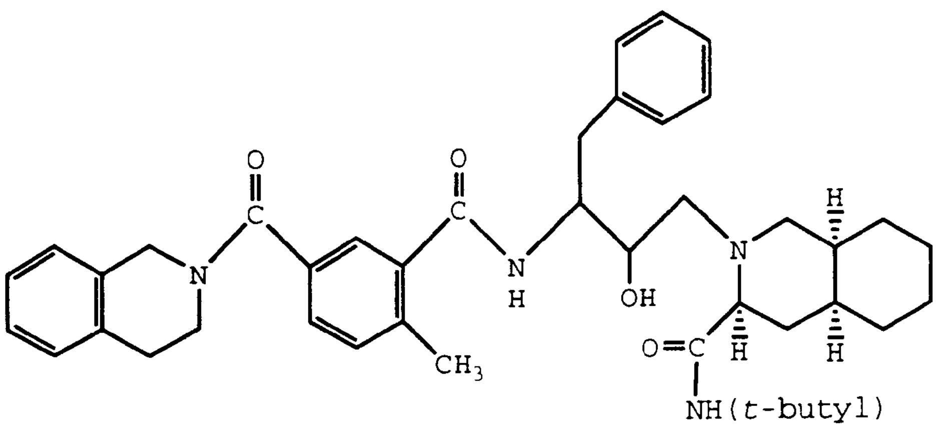

*[3S-(3R*,4aR*,8aR*,2'S*,3'R*)]*-2-[2'-hydroxy-3'-phenylmethyl-4'-aza-5'-oxo-5'-(3''-[1''',2''',3''',4'''-tetrahydroisoquinolin-2'''-ylcarbonyl]-6''- methyl)phenyl]pentyl-decahydroisoquinoline-3-N-*t*-butylcarboxamide; and

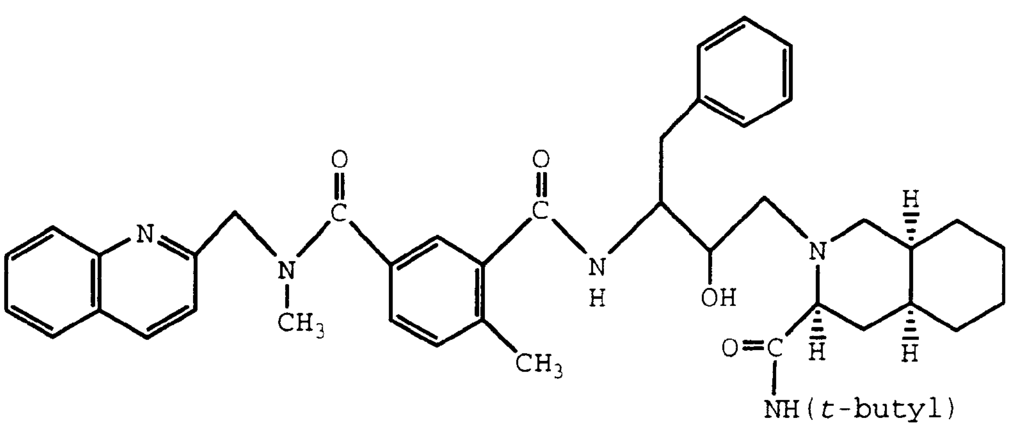

[3S-(3R*,4aR*,8aR*,2'S*,3'R*)]-2-[2'-hydroxy-3'-phenylmethyl-4'-aza-5'-oxo-5'-(3"-[1'"-oxo-2'"-N-methylaza-3'"-quinolin-2""-ylpropyl]-6"-methyl)phenyl]pentyl-decahydroisoquinoline-3-N-t-butylcarboxamide; or a pharmaceutically acceptable salt thereof.

The following list of compounds is provided to further illustrate compounds of formula I included within the scope of the invention:

[3'"S-(3'"R*,4'"S*)]-N-[1'-oxo-1'-(3"-[1'"-oxo-2'"-aza-3'"-phenylmethyl-4'"-hydroxy-5'"-(2""-N-t-butylcarbamido)phenyl]pentyl-4"-bromo]phenyl)methyl]-1,2,3,4-tetrahydroisoquinoline;

[3'"S-(3'"R*,4'"S*)]-N-[1'-oxo-1'-(3"-[1'"-oxo-2'"-aza-3'"-naphthylmethyl-4'"-hydroxy-5'"-(2""-N-t-butylcarbamido)phenyl]pentyl-4"-methyl]phenyl)methyl]1,2,3,4-tetrahydroisoquinoline;

[3'"S-(3'"R*,4'"S*)]-N-[1'-oxo-1'-(3"-[1'"-oxo-2'"-aza-3'"-naphthylthiomethyl-4'"-hydroxy-5'"-(2""-N-t-butylcarbamido)phenyl]pentyl-4"-methyl]phenyl)methyl]-1,2,3,4-tetrahydroisoquinoline;

[3'"S-(3'"R*, 4'"S*)]-N-[1'-oxo-1'-(3"-[1'"-oxo-2'"-aza-3'"-phenylmethyl-4'"-hydroxy-5'"-(2""-N-t-butylcarbamido)phenyl]pentyl-4"-methoxy]phenyl)methyl]-1,2,3,4-tetrahydroisoquinoline;

[3'"S-(3'"R*,4'"S*)]-N-[1'-oxo-1'-(3"-[1'"-oxo-2'"-aza-3'"-phenylmethyl-4'"-hydroxy-5'"-(2""-N-t-butylcarbamido)phenyl]pentyl-4"-methylthio]phenyl)methyl]-1,2,3,4-tetrahydroisoquinoline;

[3'"S-(3'"R*,4'"S*)] -N-[1'-oxo-1'-(3"-[1'"-oxo-2'"-aza-3'"-cyclohexylmethyl-4'"-hydroxy-5'"-(2""-N-t-butyl-carbamido)phenyl]pentyl-4"-methylthio]phenyl)methyl]-1,2,3,4-tetrahydroisoquinoline;

[3'"S-(3'"R*, 4'"S*)]-N-[1'-oxo-1'-(3"-[1'"-oxo-2'"-aza-3'"-phenylmethyl-4'"-hydroxy-5'"-(2""-N-t-butylcarbamido)phenyl]pentyl-4"-amino]phenyl)methyl]-1,2,3,4-tetrahydroisoquinoline;

[3'"S-(3'"R*,4'"S*)]-N-[1'-oxo-1'-(3"-[1'"-oxo-2'"-aza-3'"-phenylmethyl-4'"-hydroxy-5'"-(2""-N-t-butylcarbamido)phenyl]pentyl-4"-carbamoyl]phenyl)methyl]-1,2,3,4-tetrahydroisoquinoline;

[3S-(3R*, 4aR*, 8aR*,2'S*,3'R*)]-2-[2'-hydroxy-3'-naphth-2-ylthiomethyl-4∍-aza-5'-oxo-5'-(3"-(1'",2'",3'",4'"-tetrahydroisoquinolin-2'"-ylcarbonyl)-6"-methyl)phenyl]pentyl decahydroisoquinoline-3-N-t-butylcarboxamide;

[3S-(3R*,4aR*,8aR*,2'S*,3'R *)]-2-[2'-hydroxy-3'-phenylthiomethyl-4'-aza-5'-oxo-5'-(3"-(1'",2'",3'",4'"-tetrahydroisoquinolin-2'"-ylcarbonyl)-6"-methyl)phenyl]pentyl decahydroisoquinoline-3-N-t-butylcarboxamide;

[3S-(3R*,4aR*,8aR*,2'S*,3'R*)]-2-[2'-hydroxy-3'-phenylmethyl-4'-aza-5'-oxo-5'-(3"-(8'"-methyl-1'",2'",3'",4'"-tetrahydroisoquinolin-2'"-ylcarbonyl)-6"-methyl)phenyl]pentyldecahydroisoquinoline-3-N-t-butylcarboxamide;

[3S-(3R*,4aR*,8aR*,2'S*,3'S*)]-2- [2'-hydroxy-3'-phenylmethyl-4'-aza-5'-oxo-5'-(3"-(1'",2'",3'", 4'"-tetrahydroisoquinolin-2'"-ylcarbonyl)-6"-chloro)phenyl]pentyl decahydroisoquinoline-3-N-t-butylcarboxamide;

[3S-(3R*,4aR*,8aR*,2'S*,3'R*)]-2-[2'-hydroxy-3'-phenylmethyl-4'-aza-5'-oxo-5'-(3"-(1'",2'",3'", 4'"-tetrahydroisoquinolin-2'"-ylcarbonyl)-6"-methoxy)phenyl]pentyl decahydroisoquinoline-3-N-t-butylcarboxamide;

[3S-(3R*,4aR*,8aR*,2'S*,3'R*)]-2-[2'-hydroxy-3'-phenylmethyl-4'-aza-5'-oxo-5'-(3"-(1'",2'",3'",4'"-tetrahydroisoquinolin-2'"-ylcarbonyl)-6-trifluoromethyl)phenyl]pentyl decahydroisoquinoline-3-N-t-butylcarboxamide;

[3S-(3R*,4aR *,8aR*,2'S*,3'R*)]-2-[2'-hydroxy-3'-phenylmethyl-4'-aza-5'-oxo-5'-(3"-(1'",2'",3'", 4'"-tetrahydroisoquinolin-2'"-ylcarbonyl)-6"-methylthio)phenyl] pentyl decahydroisoquinoline-3-N-t-butylcarboxamide

[3S-(3R*,4aR*,8aR*,2'S*,3R*)]-2-[2'-hydroxy-3'-phenylmethyl-4'-aza-5'-oxo-5'-(3"-[1'"-oxo-2'"-N-methylaza-3'"-quinolin-2""-ylpropyl]-6"-bromo)phenyl]pentyl decahydroisoquinoline-3-N-t-butylcarboxamide;

[3S-(3R*,4aR*,8aR*,2'S*,3'R*)]-2-[2'-hydroxy-3'-phenylmethyl-4'-aza-5'-oxo-5'-(3"-[1'"-oxo-2'"-N-methylaza-3'"-quinolin-2""-ylpropyl]-6"-ethoxy)phenyl]pentyl decahydroisoquinoline-3-N-t-butylcarboxamide;

[3S-(3R*,4aR*,8aR*,2'S*,3'R*)]-2-[2'-hydroxy-3'-naphth-1-ylthiomethyl-4'-aza-5'-oxo-5'-(3"-[1'"-oxo-2'"-N-methylaza-3'"-quinolin-2""-ylpropyl]-6"-bromo)phenyl]pentyl decahydroisoquinoline-3-N-t-butylcarboxamide;

[3S-(3R*,4aR*,8aR*,2'S*,3'R*)]-2-[2'-hydroxy-3'-phenylmethyl-4'-aza-5'-oxo-5'-(3"-[1'"-oxo-2'"-N-ethylaza-3'"-quinolin-2""-ylpropyl]-6"-bromo)phenyl]pentyl decahydroisoquinoline-3-N-t-butylcarboxamide;

[3S-(3R*,4aR*,8aR*,2'S*,3'R*)]-2-[2'-hydroxy-3'-phenylmethyl-4'-aza-5'-oxo-5'-(3"-[1'"-oxo-2'"-N-methylaza-4'"-quinolin-2""-ylbutyl]-6"-bromo)phenyl]pentyl decahydroisoquinoline-3-N-t-butylcarboxamide;

[3S-(3R*,4aR*,8aR*,2'S*,3'R*)]-2-[2'-hydroxy-3'-phenylmethyl-4'-aza-5'-oxo-5'-(3"-[1'"-oxo-2'"-N-methylaza-3'"-quinolin-8""-ylpropyl]-6"-methyl)phenyl]pentyl decahydroisoquinoline-3-N-t-butylcarboxamide;

[3S-(3R*,4aR*,8aR*,2'S*,3'R*)]-2-[2'-hydroxy-3'-naphth-2-ylmethyl-4'-aza-5'-oxo-5'-(3"-[1'"-oxo-2'"-N-methylaza-3'"-quinolin-2""-ylpropyl]-6"-bromo)phenyl]pentyl decahydroisoquinoline-3-N-t-butylcarboxamide;

[3S-(3R*,4aR*,8aR*,2'S*,3'R*)]-2-[2'-hydroxy-3'-phenylmethyl-4'-aza-5'-oxo-5'-(3"-[1'"-oxo-2'"-aza-3'"-quinolin-2""-ylpropyl]-6"-bromo)phenyl]pentyl decahydroisoquinoline-3-N-t-butylcarboxamide;

[3S-(3R*,4aR*,8aR*,2'S*,3'R*)]-2-[2'-hydroxy-3'-phenylmethyl-4'-aza-5'-oxo-5'-(3"-[1'"-oxo-2'"-aza-3'"-naphth-2""-ylpropyl]-6"-methyl)phenyl]pentyl decahydroisoquinoline-3-N-t-butylcarboxamide;

[2'R-(2'R*,3'S*)]-2-[2'-hydroxy-3'-phenylmethyl-4'-aza-5'-oxo-5'-(3"-(1'"-oxo-2'"-aza-3'"-naphth-2""-yl)propyl-6"-methyl)phenyl]pentyl-N-t-butylbenzamide;

[2'R-(2'R*,3'S*)]-2-[2'-hydroxy-3'-phenylmethyl-4'-aza-5'-oxo-5'-(3"-(1'"-oxo-2'"-N-methylaza-3'"-quinolin-2’’’’-yl)propyl-6’’-methyl)phenyl]pentyl-N-*t*-butylbenzamide;

[2’R-*(2’R*,3’S*)*]-2-[2’-hydroxy-3’-naphthylmethyl-4’-aza-5’-oxo-5’-(3’’-(1’’’-oxo-2’’’-N-methylaza-3’’’-quinolin-2’’’’-yl)propyl-6’’-methyl)phenyl]pentyl-N-*t*-butylbenzamide;

[2’R-*(2’R*,3’S*)]*-2-[2’-hydroxy-3’-phenylmethyl-4’-aza-5’-oxo-5’-(3’’-(1’’’-oxo-2’’’-N-methylaza-3’’’-(8’’’’-methylquinolin-2’’’’-yl))propyl-6’’-methyl)phenyl]pentyl-N-*t*-butylbenzamide;

[2’R-*(2’R*,3’S*)*]-2-[2’-hydroxy-3’-phenylmethyl-4’-aza-5’-oxo-5’-(3’’-(1’’’-oxo-2’’’-aza-3’’’-quinolin-2’’’’-yl)propyl-6’’-chloro)phenyl]pentyl-N-*t*-butylbenzamide;

[2’R-*(2’R*,3’S*)*]-2-[2’-hydroxy-3’-phenylmethyl-4’-aza-5’-oxo-5’-(3’’-(1’’’-oxo-2’’’-aza-3’’’-quinoxalin-2’’’’-yl)propyl-6’’-methyl)phenyl]pentyl-N-*t*-butylbenzamide;

[2’R-*(2’R*,3’S*)*]-2-[2’-hydroxy-3’-phenylmethyl-4’-aza-5’-oxo-5’-(3’’-(1’’’-oxo-2’’’-aza-3’’’-naphth-2’’’’-yl)propyl-6’’-methyl)phenyl]pentyl-4-methyl-N-*t*-butylbenzamide;

[2’R-*(2’R*,3’S*)]-2-[2’-hydroxy -3’-phenylmethyl-4’-aza-5’-oxo-5’-(3’’-(1’’’-oxo-2’’’-N-methylaza-3’’’-pyrid-2’’’’-yl)propyl-6’’-methyl)phenyl]pentyl-N-*t*-butylbenzamide;

[2’R- *(2’R*,3’S*)*]-2-[2’-hydroxy-3’-phenylmethyl-4’-aza-5’-oxo-5’-(3’’-(1’’’-oxo-2’’’-aza-3’’’-quinolin-2’’’’-yl)propyl-6’’-methyl)phenyl]pentyl-N-*t*-butylprolinamide;

[3S-*(3R*,4aR*,8aR*,2’S*,3’R*)*]-2-[2’-hydroxy-3’-phenylmethyl-4’-aza-5’-oxo-5’-(3’’-[1’’’-oxo-2’’’-N-methylaza-3’’’-pyrid-2’’’’-ylpropyl]-6’’-methyl)phenyl]pentyl-decahydroisoquinoline-3-N-*t*-butylcarboxamide;

[2’R-*(2’R*,3’S*)*]-2-[2’-hydroxy-3’-phenylmethyl-4’-aza-5’-oxo-5’-(3’’-(1’’’-oxo-2’’’-N-methylaza-3’’’-pyrid-3’’’’-yl)-propyl-6’’-methyl)phenyl]pentyl-N-*t*-butylbenzamide;

[2’R-*(2’R*,3’S*)*]-2-[2’-hydroxy-3’-phenylmethyl-4’-aza-5’-oxo-5’-(3’’-(1’’’-oxo-N-methylaza-3’’’-pyrid-4’’’’-yl)propyl-6’’-methyl)phenyl]pentyl-N-*t*-butylbenzamide;

[2’R-*(2’R*,3’S*)*]-2-[2’-hydroxy-3’-phenylmethyl-4’-aza-5’-oxo-5’-(3’’-(1’’’-oxo-2’’’-N-methylaza-3’’’-phenyl-propyl-6’’-methyl)phenyl]pentyl-N-*t*-butylbenzamide;

[2’R-*(2’R*,3’S*)*]-2-[2’-hydroxy-3’-phenylmethyl-4’-aza-5’-oxo-5’-(3’’-(1’’’-oxo-2’’’-N-methylaza-3’’’-naphth-2’’’’-yl)-propyl-6’’-methyl)phenyl]pentyl-N-*t*-butylbenzamide;

or a pharmaceutically acceptable salt thereof.

The compounds of formula I can be prepared according to the following Reaction I.

Reaction I

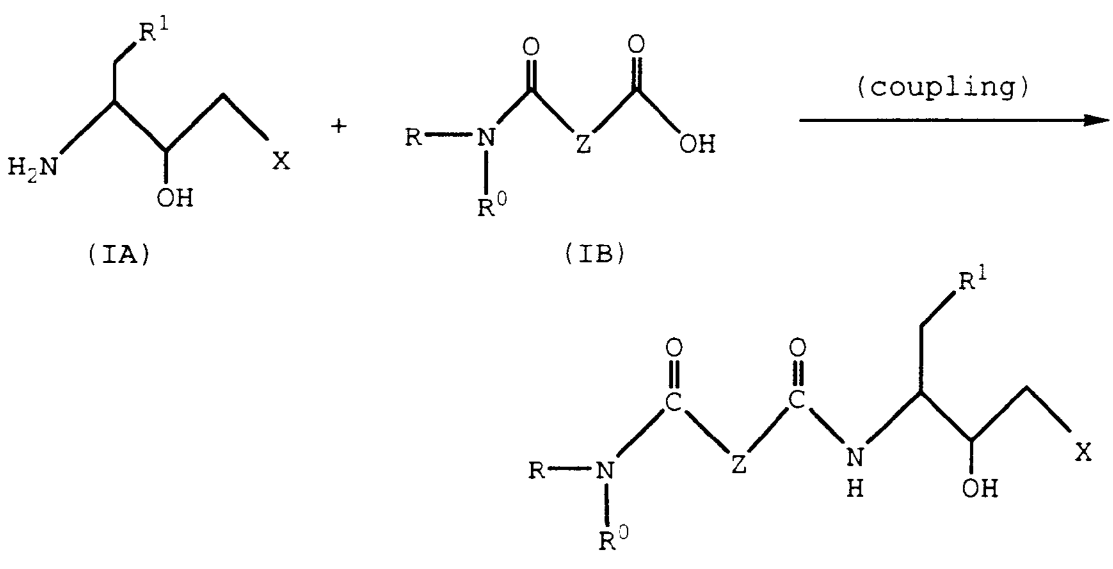


(IA) (IB) (I)

where R, $R^0$, $R^1$, X and Z are as defined above.

Reaction I is a standard coupling reaction commonly employed in the synthesis of peptides which is carried out by reacting a appropriately substituted amine of formula IA, with an appropriately substituted carboxylic acid reactant of formula IB, or an activated derivative thereof, in an aprotic solvent or mixture of solvents. The reaction is preferably carried out in the presence of a coupling reagent. Typical aprotic solvents for this reaction are tetrahydrofuran and dimethylformamide, preferably a mixture of such solvents. The reaction is carried out

at a temperature from about -30°C to about 25°C. The amine reactant is generally employed in equimolar proportions relative to the carboxylic acid reactant, in the presence of an equimolar quantity to a slight excess of the coupling reagent. Typical coupling reagents include the carbodiimides such as dicyclohexylcarbodiimide (DCC) and N,N'-diethylcarbodiimide; the imidazoles such as carbonyldiimidazole; as well as reagents such as bis(2-oxo-3-oxazolidinyl)phosphinic chloride (BOP-Cl) or N-ethoxycarbonyl-2-ethoxy-1,2-dihydroquinoline (EEDQ). A preferred coupling reagent for this reaction is DCC. A promoting agent maybe included for this reaction; a preferred promoting agent is hydroxybenzo-triazole hydrate (HOBT·$H_2O$).

Once the reaction is complete, the compound may be isolated, if desired, by procedures known in the art, for example, the compound may be crystallized and then collected by filtration, or the reaction solvent may be removed by extraction, evaporation or decantation. The compound may be further purified, if desired, by common techniques such as crystallization or chromatography over solid supports such as silica gel or alumina.

The compounds of the formula IA where X is a group having the formula

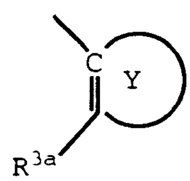 ;

can be prepared according to the procedures shown below in Reaction Scheme A.

Reaction Scheme A

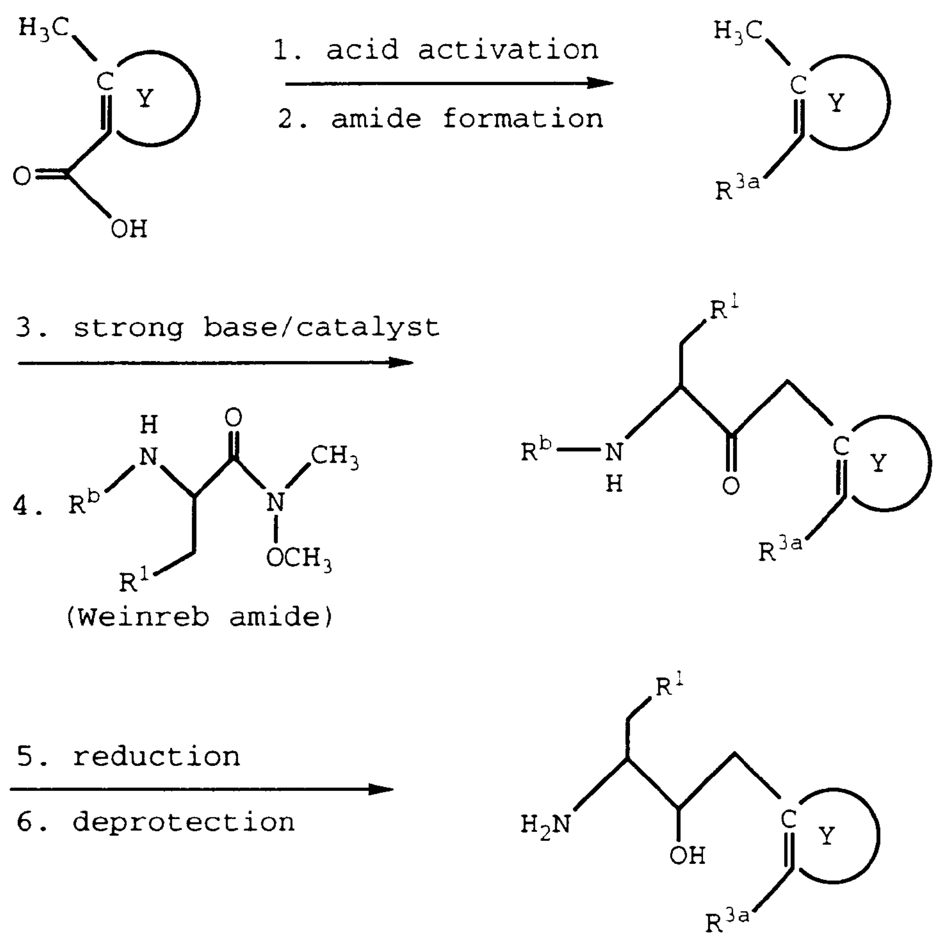


where:

$R^b$ is an amino-protecting group; and

$R^1$, $R^{3a}$ and Y are as defined above.

Reaction Scheme A, above, is accomplished by carrying out reactions 1-6 in sequential order. Once a reaction is complete, the intermediate compound may be isolated, if desired by procedures known in the art, for example, the compound may be crystallized and then collected by filtration, or the reaction solvent may be removed by extraction, evaporation or decantation. The intermediate compound may be further purified, if desired, by common techniques such as crystallization or chromatography over solid supports such as silica gel or alumina, before carrying out the next step of the reaction scheme.

In Reaction A.1, the reaction is typically carried out by activating, that is, converting, a suitably substituted aryl or unsaturated heterocycle carboxylic acid to the corresponding acyl chloride or acyl bromide by reaction with thionyl chloride, thionyl bromide, phosphorous trichloride, phosphorous tribromide, phosphorous pentabromide or phosphorous pentachloride according to procedures and under conditions known in the art. Suitable aryl, heterocycle or unsaturated heterocycle carboxylic acid compounds are commercially available or prepared by standard procedures known in the art.

In Reaction A.2, the acyl chloride or acyl bromide, prepared in Reaction A.1, is reacted with ammonia or a primary or secondary amine having the formula

$$H\text{-}NR^4R^4,$$

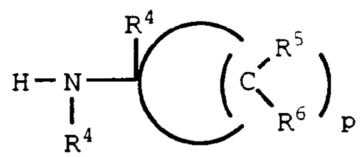

,

or

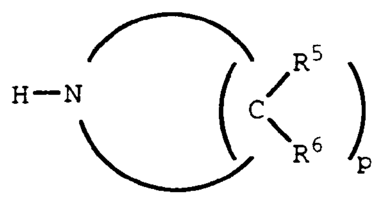

;

where $R^4$, $R^5$, $R^6$ and p are as defined above for formula I, in a nonpolar aprotic solvent or mixture of solvents in the presence or absence of an acid scavenger to afford the corresponding amide. The reaction is typically carried out at a temperature of from about -20°C to about 25°C. Typical solvents for this reaction include ethers and chlorinated hydrocarbons, preferably diethylether, chloroform or methylene chloride. Preferably, this reaction is carried out in the presence of an acid scavenger such as a tertiary amine, preferably triethylamine.

In Reaction A.3, the amide prepared in Reaction A.2, is reacted with a strong base in the presence of a solubilizing agent to afford the corresponding anion which is then reacted in Reaction A.4 with a Weinreb amide to afford a ketone. Reaction A.3 is carried out in an aprotic solvent at a temperature of from about -78°C to about 0°C. Typical bases used in Reaction A.3 include lithium amide bases and alkyl lithium bases, preferably $C_1$-$C_4$ alkyl lithium bases and lithium di($C_1$-$C_4$)alkylamide bases. Typical solubilizing agents for Reaction 3 are tetramethyl($C_1$-$C_4$)alkylenediamines, preferably tetramethylethylenediamine. Reaction A.4 is carried out in an aprotic solvent at a temperature from about -80°C to about -40°C. Typical solvents for Reactions A.3 and A.4 include ethers, preferably tetrahydrofuran. In Reaction A.4, the anion is generally employed in an amount ranging from about equimolar proportions to about a three molar excess of the anion, preferably in about a two molar excess of the anion relative to the Weinreb amide reactant.

In Reaction A.5, the ketone prepared in Reaction A.3, is reduced to the corresponding alcohol using a suitable reducing agent. The reaction is carried out in a protic solvent at a temperature of from about -25°C to about 25°C. Typical reducing agents for this reaction include sodium borohydride, lithium borohydride, diisobutylaluminum hydride, and sodium bis(2-methoxyethoxy)aluminum hydride. A preferred reducing agent is sodium borohydride. Typical protic solvents for this reaction include alcohols, preferably ethanol.

Reaction A.6 is a standard amino deprotection reaction using procedures and methods known in the art to afford the corresponding amine which is used in Reaction I above. This amine may be reacted without purification, but it is preferably purified first.

The compounds of formula IA where X is a group having the structure

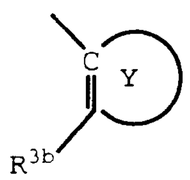 ;

are prepared according to the procedures shown below in Reaction Scheme B.

Reaction Scheme B

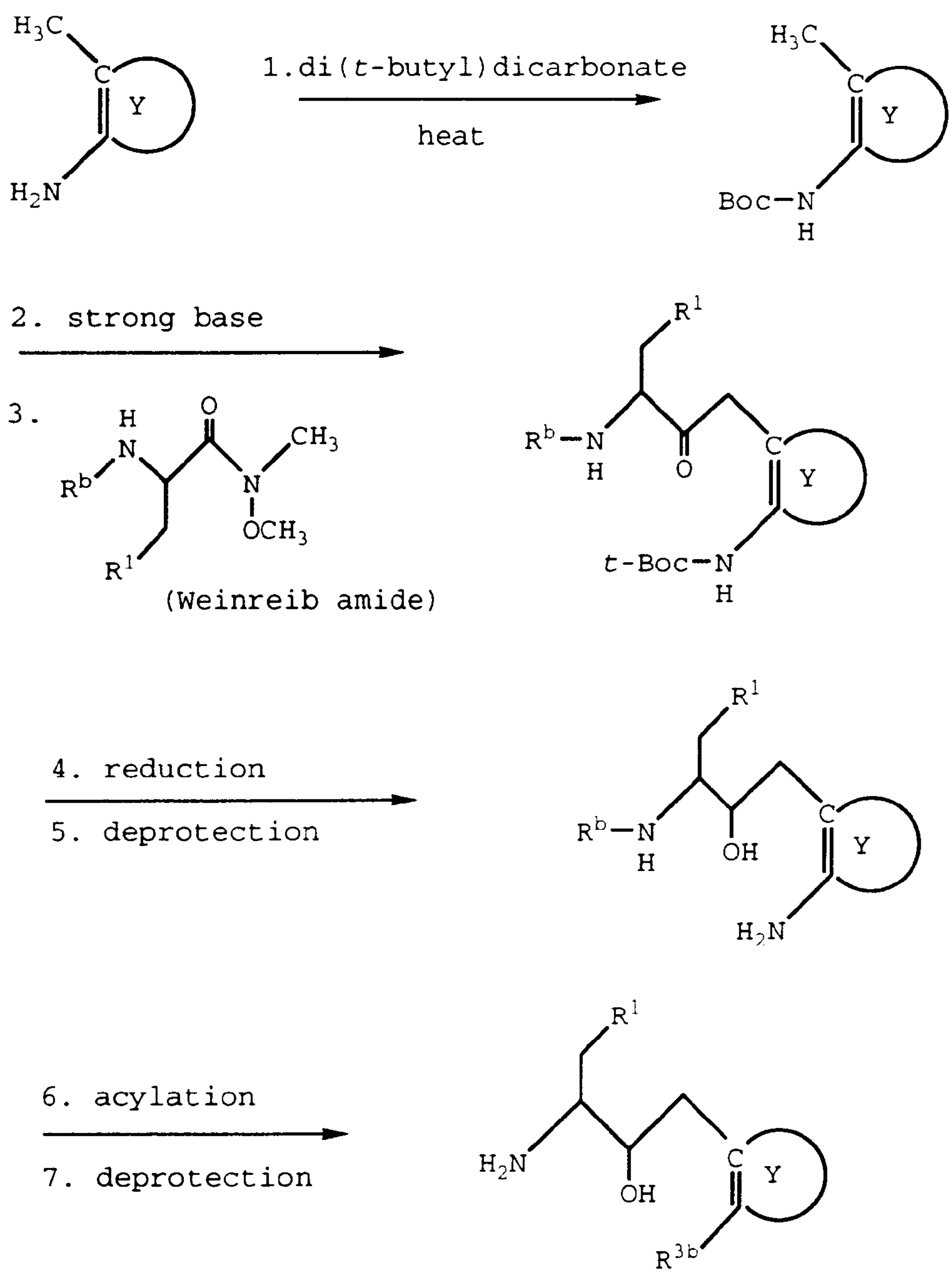


where $R^b$, $R^1$, Y and $R^{3b}$ are as defined above.

Reaction Scheme B, above, is accomplished by carrying out reactions 1-7 in sequential order. Once a

reaction is complete, the intermediate compound may be isolated by procedures known in the art, for example, the compound may be crystallized and then collected by filtration, or the reaction solvent may be removed by extraction, evaporation or decantation. The intermediate compound may be further purified, if desired, by common techniques such as crystallization or chromatography over solid supports such as silica gel or alumina, before carrying out the next step of the reaction scheme.

In Reaction B.1, a suitably substituted aryl or unsaturated heterocycle amine is protected, under standard conditions used with amino-protecting groups known in the art. Reactions B.2 through B.5 are carried out substantially as described above in Reaction Scheme A.3-A.6, with the exception that in Reaction Scheme B, an additional deprotection reaction, Reaction B.5, is necessary to remove the amino-protecting group introduced in Reaction B.1. This is a standard amino deprotection reaction using procedures and methods known in the art. For example, the *t*-Boc group illustrated in Reaction Scheme B.1 may be removed using a strong acid, preferably trifluoroacetic acid.

In Reaction B.6, the illustrated intermediate is acylated with a suitable acyl halide, isocyanate or chloroformate, preferably in the presence of an acid scavenger such as a tertiary amine, preferably triethylamine. The reaction is carried out at a temperature of from about -20°C to about 25°C. Typical solvents for this reaction include ethers and chlorinated hydrocarbons, preferably diethylether, chloroform or methylene chloride.

Reaction B.7 is a standard amino deprotection reaction using procedures and methods known in the art to afford the corresponding amine which is used in Reaction I, above. This amine may be reacted without purification, but it is preferably purified first.

The compounds of formula I where X is a group having the structure

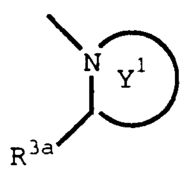 ;

are prepared according to the procedures shown below in Reaction Scheme C.

Reaction Scheme C

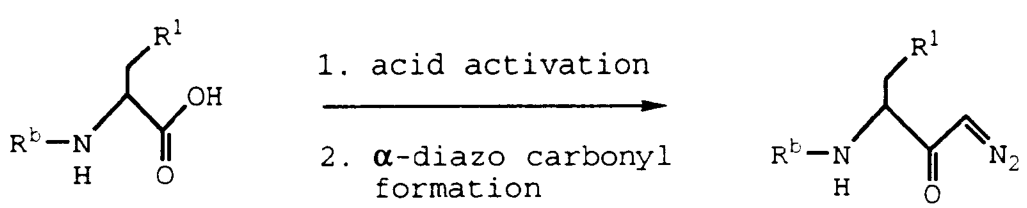

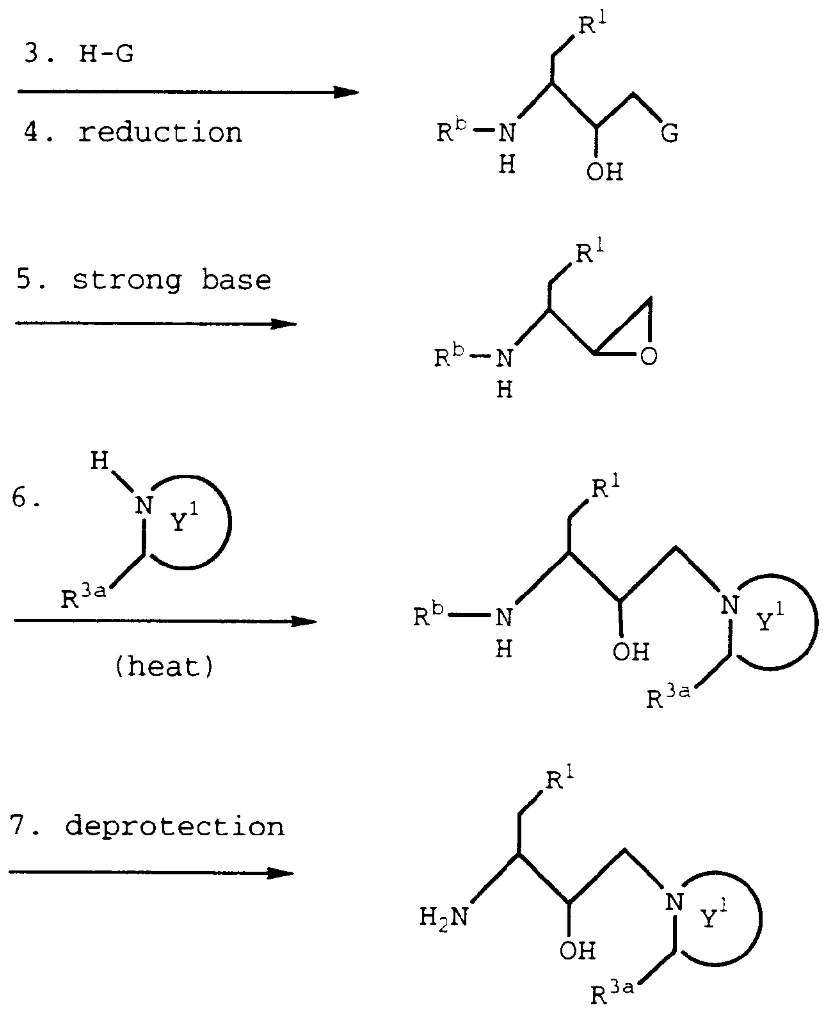


(IA)

where:

$R^1$, $R^{3a}$ and $R^b$ are as defined above; and

G is halo.

Reaction Scheme C, above, is accomplished by carrying out reactions 1-7 in sequential order. Once a reaction is complete, the intermediate compound may be isolated, if desired, by procedures known in the art, for example, the compound may be crystallized and then collected by filtration, or the reaction solvent may be removed by extraction, evaporation or decantation. The intermediate compound may be further purified, if desired, by common techniques such as crystallization or chromatography over solid supports such as silica gel or alumina, before carrying out the next step of the reaction scheme.

Reaction C.1 is carried out by activating, that is, converting, an amino-protected carboxylic acid reactant having the structure:

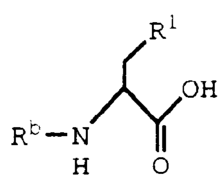

to the corresponding mixed anhydride under conditions known in the art. For example, the amino-protected carboxylic acid reactant may be reacted with a $C_1$-$C_6$ alkylchloroformate, such as isobutylchloroformate preferably in the presence of an acid scavenger. Preferred acid scavengers are the trialkylamines, preferably triethylamine. The reaction is typically carried out in an aprotic solvent such as ethyl acetate. Solvent choice is not critical so long as the solvent employed is inert to the ongoing reaction and the reactants are sufficiently solubilized to effect the desired reaction. The resulting mixed anhydride reactant is preferably used in Reaction

C.2 without further isolation or purification.

Reaction C.2 is accomplished in two steps. First, a solution of sodium hydroxide, covered with a layer of an ether solvent, preferably diethylether, is reacted with a large excess of N-methyl-N-nitro-N-nitrosoguanidine to form a diazomethane reactant. The sodium hydroxide is preferably used as an aqueous solution having about four to six mol/liter of sodium hydroxide. Once this reaction is substantially complete, the organic layer is dried over a dessicant such as potassium hydroxide. This solution is then reacted with the mixed anhydride from Reaction C.1, above, to form the corresponding α-diazo carbonyl compound. The diazomethane reactant is preferably used in this reaction without isolation or purification. The reaction is typically carried out at a temperature of from about -50°C to about -20°C, preferably about -30°C.

In Reaction C.3, the α-diazo carbonyl compound prepared in Reaction C.2 is reacted with an acid of the formula H-G where G is halo, in an aprotic solvent such as diethylether to form an α-halo carbonyl compound. A preferred acid reactant is hydrochloric acid which provides the corresponding α-chloro carbonyl compound. The reaction is typically carried out at a temperature from about -30°C to about 0°C. Solvent choice is not critical so long as the solvent employed is inert to the ongoing reaction and the reactants are sufficiently solubilized to effect the desired reaction. The acid reactant is typically added in the form of an anhydrous gas in small increments until the reaction appears substantially complete. The reaction can be monitored by thin layer chromatography (TLC).

In Reaction C.4, the carbonyl moiety on the compound prepared in Reaction C.3 is reduced using standard conditions known in the art to form the corresponding α-chloro hydroxy compound. For example, the compound prepared in Reaction C.3 is combined with a reducing agent in a mixture of solvents. Typical reducing agents include sodium borohydride, lithium borohydride, zinc borohydride, diisobutylaluminum hydride and sodium bis(2-methoxyethoxy)aluminum hydride. A preferred reducing agent is sodium borohydride. Typical solvent mixtures include a protic and aprotic mixture such as tetrahydrofuran/water. Solvent choice is not critical so long as the solvent employed is inert to the ongoing reaction and the reactants are sufficiently solubilized to effect the desired reaction. The reaction is typically carried out at a temperature from about -10°C to about 10°C, preferably about 0°C.

In Reaction C.5, the α-chloro hydroxy compound prepared in Reaction C.4 is treated with a strong base to form the corresponding epoxide under standard conditions known in the art. For example, the α-chloro hydroxy compound may be reacted with a potassium hydroxide/ethanol mixture in an organic solvent such as ethyl acetate . Solvent choice is not critical so long as the solvent employed is inert to the ongoing reaction and the reactants are sufficiently solubilized to effect the desired reaction. The reaction is typically carried out at a temperature from about 0°C to about the reflux temperature of the solvent. Preferably the reaction is carried out at room temperature.

In Reaction C.6, the epoxide prepared in Reaction C.5 is reacted with a heterocyclic reactant of the formula.

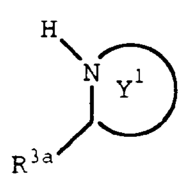 ;

in a protic solvent at a temperature of from about 70°C to 100°C. Solvent choice is not critical so long as the solvent employed is inert to the ongoing reaction and the reactants are sufficiently solubilized to effect the desired reaction. Typical solvents for this reaction include the alcohols, preferably ethanol. The reaction is preferably carried out at a temperature of about 80°C.

Reaction C.7 is a standard amino deprotection reaction using procedures and methods known in the art to afford the corresponding amine which is used in Reaction I, above. This amine may be reacted without purification, but it is preferably purified first.

The Weinreb amide used as a reactant in Reaction A.4 and B.3 is prepared by reacting an amino-protected amino acid with N-methoxy-N-methyl-amine in the presence of a promoting agent, an acid scavenger, and a coupling agent. The reaction is carried out in an aprotic solvent or mixture of solvents at a temperature of from about -25°C to 25°C. A preferred promoting agent for this reaction is HOBT·$H_2O$. Preferred acid scavengers are the tertiary alkylamines, preferably triethylamine or N-methylmorpholine. A preferred coupling reagent is ethyl dimethylaminopropylcarbodiimide hydrochloride. The Weinreb amide afforded by this reaction is preferably isolated prior to its use in Reactions A.4 and B.3.

The compounds of formula IA, where $R^1$ is a group having the structure S-$R^{1a}$, where $R^{1a}$ is aryl or $C_5$-$C_7$

cycloalkyl, are prepared using an Weinreb amide having the following structure:

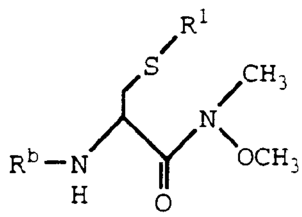

in Reactions A.4 and B.3. This Weinreb amide is prepared by first reacting amino-protected serine with triphenylphosphine and diethylazodicarboxylate (DEAD) in an aprotic solvent at a temperature of from about -80°C to 0°C to form the corresponding β-lactone. The reaction is typically carried out in an ether, such as tetrahydrofuran at a temperature of from about -80°C to -50°C. Next, the lactone ring is opened to provide a compound having the structure:

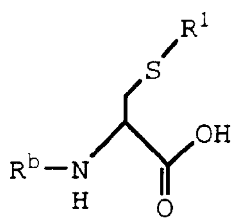

by reacting the lactone with an appropriately substituted thioanion having the structure, $-S-R^1$, where $R^1$ is as defined above for formula I. The thioanion compound is preferably formed by reacting the corresponding thiol with a strong base, such as sodium hydride or potassium hydride. This reaction is typically carried out in an aprotic solvent at a temperature from about 0°C to about 40°C and under an inert atmosphere, such as nitrogen. Typical solvents for this reaction include ethers, preferably tetrahydrofuran. The desired amide reactant is then formed by reacting the resulting carboxylic acid reactant with N-methoxy-N-methyl-amine in the presence of a promoting agent, an acid scavenger and a coupling agent substantially as described above.

The heterocyclic reactants, used in Reaction C.6 above, of the formula

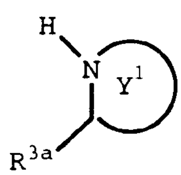 ;

can be prepared using procedures and methods known in the art. For example, the heterocyclic reactants were typically prepared from the corresponding amino-protected amino acids by acid activation followed by treatment with an alkylamine. This reaction is typically carried out in the presence of an acid scavenger, such as N-methylmorpholine. Removal of the amino-protecting group using standard chemical deprotecting techniques then provided the heterocyclic reactants used above in Reaction C.8.

For example, the [3S-(*3R*,4aR*,8aR**)]decahydroiso-quinoline-3-N-*t*-butoxycarboxamide was prepared using (2S)-1,2,3,4-tetrahydro-3-isoquinolinecarboxylic acid by the following procedure:

1) amino-protection (*t*-Boc);
2) acid activation/reaction with *t*-butylamine;
3) catalytic hydrogenation;
4) amino-deprotection.

The piperazine reactants may be prepared by converting an appropriately substituted pyrazine compound to the corresponding piperazine compound using procedures known in the art, preferably using catalytic hydrogenation. For example, the hydrogenation may be accomplished by combining the pyrazine reactant with a catalyst under a hydrogen atmosphere in an aprotic solvent at a temperature from about 0°C to about 60°C. Suitable catalysts include palladium-on-carbon, platinum metal, platinum oxide and the like. A preferred catalyst is platinum oxide. Typical solvents for this reaction include tetrahydrofuran, dimethylformamide or a mixture of tetrahydrofuran and dimethylformamide.

The nitrogen atom on the resultant piperazine reactant may be alkylated using procedures known in the art. For example, the piperazine reactant may be reacted with a halo($C_1$-$C_4$)alkyl, or halomethylpyridine, such as methyl iodide or chloromethylpyridine. Preferred halo substituents include chloro, bromo and iodo. The reaction is carried out at temperatures of from about 0°C to 60°C in a mutually inert solvent and in the presence of an acid scavenger. A preferred acid scavenger is potassium carbonate. Typical solvents include a mixture of a protic and aprotic solvents such as acetonitrile and water. Solvent choice is not critical so long as the solvent employed is inert to the ongoing reaction and the reactants are sufficiently solubilized to effect the desired reaction.

Alternatively, the alkylated piperazine reactant may be prepared using reductive amination. For example, the piperazine reactant prepared above may be reacted with an aldehyde (for example, 3-pyridine carboxylic aldehyde, ethanal, propanal) or a ketone in the presence of a reducing agent and an acid. The reaction is typically carried out in an alcoholic solvent such as methanol, ethanol or isopropanol. Typical reducing agents include sodium borohydride, lithium cyanoborohydride, sodium cyanoborohydride, and the like. A preferred reducing agent is sodium cyanoborohydride. Typical acids include any protic acid such as hydrochloric acid, sulfuric acid, methanesulfonic acid, or acetic acid. A preferred acid is acetic acid.

The carboxylic acid reactant, IB, used in Reaction I, to the extent not commercially available, can be prepared using known procedures. More particularly, compounds of the formula:

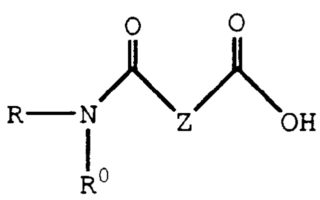

(IB)

are prepared according to the procedures shown below in Reaction Scheme II.

Reaction Scheme II

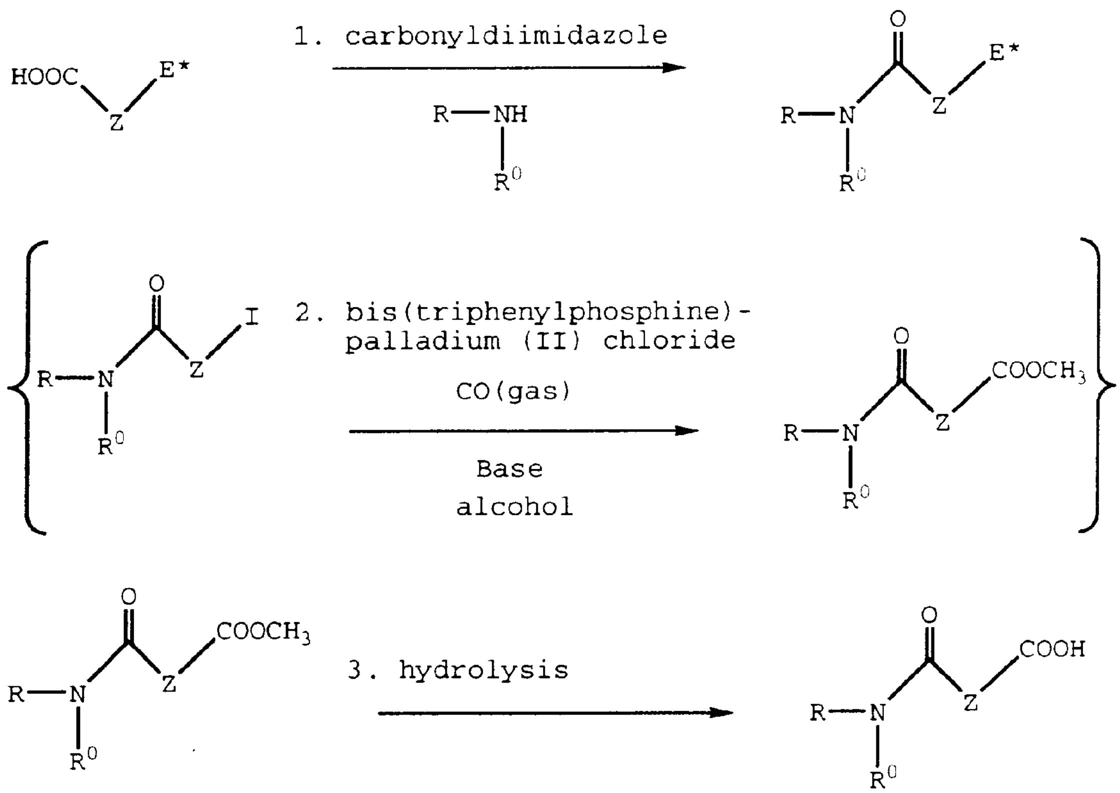


where:

Z, R and $R^0$, are as defined above for formula I;

and

E* is iodo or $C_1$-$C_4$ alkoxycarbonyl.

Where E* is iodo, Reaction Scheme II, above, is accomplished by carrying out Reactions 1-3 in sequential order. Once a reaction is complete, the intermediate compound may be isolated by procedures known in the art, for example, the compound may be crystallized and then collected by filtration, or the reaction solvent may be removed by extraction, evaporation or decantation. The intermediate compound may be further purified, if desired, by common techniques such as crystallization or chromatography over solid supports such as silica gel or alumina, before carrying out the next step of the reaction scheme.

Where E* is $C_1$-$C_4$ alkoxycarbonyl, the intermediate compound prepared in Reaction II.1 is reacted according to the procedure detailed in Reaction II.3 directly; Reaction II.2 is not necessary.

Reaction II.1 is a standard coupling reaction commonly employed in the synthesis of peptides and which is described above in the coupling reaction of Reaction I The reaction is carried out by simply combining an appropriately substituted amino moiety, $RR^0NH$, with a compound having the formula:

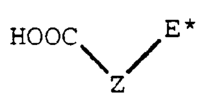

where E* is iodo or $C_1$-$C_4$ alkoxycarbonyl; and Z is as defined above for formula I. The reaction is carried out in a nonpolar aprotic solvent or mixture of solvents in the presence or absence of an acid scavenger to provide the corresponding amide. Typical solvents for this reaction include tetrahydrofuran and dimethylformamide, preferably tetrahydrofuran. The reaction is carried out at a temperature from about -30°C to about 25°C. The amine reactant is generally employed in equimolar proportions relative to the carboxylic acid reactant, in the

presence of an equimolar quantity to a slight excess of the coupling reagent.

Reaction II.2, is a carbonylation of the compound prepared in Reaction II.1, resulting in the replacement of the iodo substituent with a $C_1$-$C_4$ alkoxycarbonyl moiety, preferably methoxycarbonyl. The reaction is carried out by simply combining the iodo intermediate from Reaction II.2, above, a catalyst under a carbon monoxide atmosphere and in an alcoholic solvent, preferably methanol, to provide the alkyl ester of the desired compound. This reaction is carried out in the presence of an acid scavenger, preferably dicyclohexylamine. The reaction is carried out at a temperature from about 0°C to about 25°C. A preferred catalyst is bis(triphenylphospine)palladium (II) chloride.

Reaction II.3 is a standard hydrolysis reaction of the alkyl ester to provide the corresponding carboxylic acid reactant which is used in Reaction I. The reaction is carried out by combining the alkyl ester reactant with lithium hydroxide in a suitable solvent followed by acidification of the reaction solution to pH 2-3 to provide the desired carboxylic acid reactant. Typical solvents include a mixture of an ether and water, preferably a tetrahydrofuran/water mixture. The reaction is carried out at a temperature from about 0°C to about 35°C. The lithium hydroxide reactant is generally employed in equimolar proportions to about a two molar excess relative to the ester reactant, preferably in about a one molar excess.

Alternatively, a compound of the formula

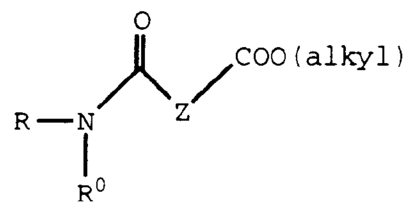

may be prepared by reacting an amino moiety of the formula $RR^0NH$ with a compound of the formula

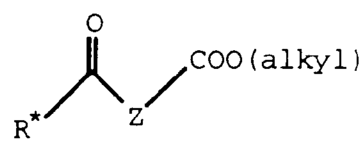

where R* is an activating group such as pentafluorophenol using procedures known in the art. The reaction is typically carried out in an aprotic solvent such as tetrahydrofuran, dimethylformamide, or methylene chloride at a temperature from about 0°C to about 35°C. A preferred solvent is methylene chloride. An acid scavenger such as triethylamine or N-methylmorpholine is preferably included. The resultant compound may then be hydrolyzed to provide the desired carboxylic acid reactant (used in Reaction Scheme I, above.

The amino moiety, $RR^0NH$, where R and $R^0$ are independently selected from $C_1$-$C_6$ alkyl, aryl($C_1$-$C_4$)alkyl, heterocycle($C_1$-$C_4$)-alkyl or unsaturated heterocycle($C_1$-$C_4$)alkyl, which is used above may be prepared using procedures known in the art. For example, amino-protected methylamine may be reacted with R-X (or $R^0$-X), where X is halo, in the presence of a promoting agent, for example, sodium iodide or potassium iodide, and a strong base such as sodium hydride, or *n*-butyl lithium, to provide a compound of the formula $CH_3$-N($R^b$)-R (or $CH_3$-N($R^b$)-$R^0$), where $R^b$ is an amino-protecting group and R and $R^0$ are defined above. Typical solvents include aprotic solvents such as dimethylformamide, or tetrahydrofuran, preferably dimethylformamide. The reaction is carried out at a temperature from about -10°C to about 10°C, preferably at about 0°C. The resultant protected amine may then be deprotected using procedure known in the art to provide the free amine which may be used as described above. To facilitate isolation of the free amine, a salt may be formed for example by exposing the deprotected amine to an acid such as hydrochloric acid.

Another procedure for preparing the amine compound of formula $RR^0NH$ involves reacting an amino-protected compound of formula R-NH-$R^b$ may be reacted with an appropriately substituted alkyl iodide or alkyl bromide ($R^0$-I or $R^0$-Br), such as methyl iodide or ethyl bromide, to provide an amino-protected compound of formula $RR^0NH$-$R^b$. This compound may then be deprotected and isolated for use in the above reactions.

As noted above, all asymmetric forms, individual isomers and combinations thereof are considered part of this invention. Such isomers may be prepared from their respective precursors by the procedures described above, by resolving the racemic mixtures, or by separating the diastereomers. The resolution can be carried out in the presence of a resolving agent, by chromatography or by repeated crystallization or by some combination of these techniques which are known in the art. Further details regarding resolutions can be found in

Jacques et al., Enantiomers, Racemates, and Resolutions, John Wiley & Sons 1981.

The compounds employed as initial starting materials in the synthesis of the compounds of this invention are known and, to the extent not commercially available are readily synthesized by standard procedures commonly employed by those in the art.

The pharmaceutically acceptable salts of the invention are typically formed by reacting a compound of formula I with an equimolar or excess amount of acid or base. The reactants are generally combined in a mutual solvent such as diethylether or benzene, for acid addition salts, or water or alcohols for base addition salts. The salts normally precipitate out of solution within about one hour to about ten days and can be isolated by filtration or other conventional methods.

The following Preparations and Examples further illustrate specific aspects of the present invention. It is to be understood, however, that these examples are included for illustrative purposes only and are not intended to limit the scope of the invention in any respect and should not be so construed.

In the following Preparations and Examples, the terms melting point, nuclear magnetic resonance spectra, electron impact mass spectra, field desorption mass spectra, fast atom bombardment mass spectra, infrared spectra, ultraviolet spectra, elemental analysis, high performance liquid chromatography, and thin layer chromatography are abbreviated "m.p.", "NMR", "EIMS", "MS (FD)", "MS (FAB)", "IR", "UV", "Analysis", "HPLC", and "TLC", respectively. In addition, the absorption maxima listed for the IR spectra are only those of interest and not all of the maxima observed.

In conjunction with the NMR spectra, the following abbreviations are used: "s" is singlet, "d" is doublet, "dd" is doublet of doublets, "t" is triplet, "q" is quartet, "m" is multiplet, "dm" is a doublet of multiplets and "br.s", "br.d", "br.t", and "br.m" are broad singlet, doublet, triplet, and multiplet respectively. "J" indicates the coupling constant in Hertz (Hz). Unless otherwise noted, NMR data refers to the free base of the subject compound.

The NMR spectra were obtained on a Brüker Corp. 270 MHz instrument or on a General Electric QE-300 300 MHz instrument. The chemical shifts are expressed in delta (δ) values (parts per million downfield from tetramethylsilane). MS (FD) spectra were taken on a Varian-MAT 731 Spectrometer using carbon dendrite emitters. EIMS spectra were obtained on a CEC 21-110 instrument from Consolidated Electrodynamics Corporation. MS(FAB) spectra were obtained on a VG ZAB-3 Spectrometer. IR spectra were obtained on a Perkin-Elmer 281 instrument. UV spectra were obtained on a Cary 118 instrument. TLC was carried out on E. Merck silica gel plates. Melting points are uncorrected.

Preparation 1

A. N-(*t*-Butyl)-2-methylbenzamide

To a cold (0°C) solution of 139.2 g (0.9 mol) of o-toluoyl chloride in 1200 mL of methylene chloride at 25°C, under nitrogen, was slowly added 180.0 g (1.8 mol) of triethylamine followed by the dropwise addition of a solution containing 73.14 g (1.0 mol) of t-butylamine in 200 mL of methylene chloride. The resulting reaction mixture was warmed to room temperature and allowed to react for 2.5 hours. The reaction mixture was then diluted with 1800 mL of water. The resulting organic and aqueous layers were separated, and the organic layer was washed sequentially with 2N sodium hydroxide, 1.0N hydrochloric acid and brine, dried over magnesium sulfate, filtered and then reduced to dryness under reduced pressure to provide 167.6 g of the desired subtitled intermediate as an off-white solid (mp 77-78°C).

Yield: 97%.

$^1$H NMR ($CDCl_3$): δ 1.41 (s, 9H), 2.41 (s, 3H), 5.54 (br.s, 1H), 7.13-7.30 (m, 4H).

IR ($CHCl_3$): 3430, 3011, 2971, 2932, 1661, 1510, 1484, 1452, 1393, 1366, 1304, 1216, 876 $cm^{-1}$.

MS (FD): m/e 191 ($M^+$), 191 (100).

| Analysis for $C_{12}H_{17}NO$: | | | |
|---|---|---|---|
| Calcd: | C, 75.35; | H, 8.76; | N, 7.32; |
| Found: | C, 75.10; | H, 9.11; | N, 7.20. |

B. S-N-*t*-Butyl-2-(3-(N-benzyloxycarbonyl)amino-2-oxo-4-phenylbutyl)benzamide

To a solution of 7.0 g (36.5 mmol) of the subtitled intermediate of Preparation 1A in 200 mL of anhydrous tetrahydrofuran, was added 12.1 mL (80.3 mmol) of N,N,N',N'-tetramethylethylenediamine (TMEDA) was added via syringe. The resulting solution was cooled to -78°C and then 55.9 mL of *sec*-butyllithium was added dropwise via syringe while maintaining the temperature of the reaction under -60°C. The resulting reaction solution was then allowed to stir for approximately 1 hour at -78°C before the addition of a solution containing 5.00 g (14.6 mmol) of *(S)*-N-methoxy-N-methyl-2-(N-benzyloxycarbonyl)amino-3-phenylpropanamide in 50 mL of anhydrous tetrahydrofuran was added via cannula while maintaining the reaction temperature below -65°C. The resulting reaction mixture was warmed to -20°C, quenched using 20 mL of saturated ammonium chloride and then diluted with 200 mL of diethylether. The organic and aqeous layers were separated and the organic layer was washed sequentially with water, 0.2N sodiumhydrogensulfate and brine, dried over sodium sulfate, filtered and then reduced to dryness under reduced pressure to provide a colorless oil. This oil was purified using flash chromatography (eluent of 25% ethyl acetate in methylene chloride) to provide 6.08 g of a colorless foam.

Yield: 88%.

$[a]_D$ -289.26° (*c* 0.12, MeOH).

$^1$H NMR ($CDCl_3$) δ 1.38 (s, 9H), 2.99 (dd, J=15; 6 Hz, 1H), 3.24 (dd, J=15; 6 Hz, 1H), 3.89 (d, J=18 Hz, 1H), 4.16 (d, J=18 Hz, 1H), 4.72 (dd, J=15, 6 Hz, 1H), 5.00-5.09 (m, 2H), 5.56 (d, J=6 Hz, 1H), 5.93 (br.s, 1H), 7.03-7.40 (m, 14H).

IR ($CHCl_3$): 3431, 3027, 3012, 2973, 1713, 1658, 1511, 1454, 1383, 1366, 1307, 1231, 1046 $cm^{-1}$.

MS (FD) m/e 472 ($M^+$), 218 (100).

| Analysis for $C_{29}H_{32}N_2O_4$: | | | |
|---|---|---|---|
| Calcd: | C, 73.70; | H, 6.82; | N, 5.93; |
| Found: | C, 73.41; | H, 6.98; | N, 5.83. |

C. [R-*(R*,S*)*]-N-*t*-Butyl-2-(3-(N-benzyloxycarbonyl)amino-2-hydroxy-4-phenylbutyl)benzamide

To a solution of 6.96 g (14.7 mmol) of the subtitled intermediate of Preparation 1B in 200 mL of absolute ethanol, under nitrogen, was added 2.78 g (73.5 mmol) of sodium borohydride. When the reaction was substantially complete, as indicated by TLC, the reaction mixture was diluted with 200 mL of ethyl acetate and quenched by the dropwise addition of 20 mL of saturated ammonium chloride. The organic and aqueous layers were then separated and the organic layer was washed sequentially with 1N hydrochloric acid, saturated sodium bicarbonate solution and brine, dried over sodium sulfate, filtered and then reduced to dryness under reduced pressure to provide 6.4 g of a colorless oil. This oil was purified using flash chromatography (gradient eluent of 2-10% methylene chloride in ethyl acetate) to provide 5.12 g of the subtitled intermediate.

Yield: 74%.

$[a]_D$ +10.38° (*c* 0.10, MeOH).

$^1$H NMR ($CDCl_3$) : δ 1.40 (s, 9H), 2.79 (dd, J=12, 3 Hz, 1H), 2.90-2.98 (m, 2H), 3.04 (44, J=12, 3 Hz, 1H), 3.70-3.81 (m, 1H), 3.97 (m, 1H), 4.96-5.08 (m, 2H), 5.10 (d, J=9 Hz, 1H), 5.88 (d, J=6 Hz, 1H), 5.93 (s, 1H), 7.13-7.42(m, 14H).

IR ($CHCl_3$): 3431, 3028, 3012, 2971, 1773, 1643, 1515, 1454, 1367, 1229, 1028 $cm^{-1}$.

MS (FD): m/e 475 ($M^+$), 475 (100).

| Analysis for $C_{29}H_{34}N_2O_4$: | | | |
|---|---|---|---|
| Calcd: | C, 73.39; | H, 7.22; | N, 5.99; |
| Found: | C, 73.12; | H, 7.48; | N, 5.62. |

D. [R-*(R*,S*)*]-N-*t*-Butyl-2-(3-amino-2-hydroxy-4-phenylbutyl)benzamide

A suspension was prepared containing 41.0 g (120 mmol) of the subtitled intermediate of Preparation 1C and 500 mg of 10% palladium-on-carbon in 150 mL of absolute ethanol. This suspension was shaken under 60 psi hydrogen in a Parr shaker apparatus. The 10% palladium-on-carbon catalyst was then removed by filtration. The resultant filtrate was reduced to dryness under reduced pressure to provide 31.1 g of a light yellow foam. This compound was used without further purification.

Yield: 96%.

[a]$_D$ +34.68° (*c* 1.0, MeOH).

$^1$H NMR ($CDCl_3$): δ 1.46 (s, 9H), 2.71 (dd, J=13.7; 9.5 Hz, 1H), 2.84 (dd, J=13.3; 2.51 Hz, 1H), 2.95-3.06 (m, 2H), 3.23-3.29 (m, 1H), 3.84-3.90 (m, 1H), 6.23 (s, 1H), 7.19-7.37 (m, 12H).

IR ($CHCl_3$) : 3440, 3382, 3007, 2970, 2934, 1643, 1516, 1454, 1367, 1213 cm$^{-1}$.

MS (FD): m/e 341 (M$^+$), 341 (100).

Preparation 2

A. [3S-*(3R*,4aR*,8aR*,2'S*,3'R*)*]-2-[3'-N(Benzyloxycarbonyl)amino-2'-hydroxy-4'-phenyl]butyl-N(*t*-butyl)decahydroisoquinoline-3-carboxamide

A solution of *[1R-(1R*,3S*,1'S*,4aS*,8aS*)]-1*-[(1'-N-benzyloxycarbonylamino-2'-phenyl)ethyl]oxirane and decahydroisoquinoline-3-N-*t*-butylcarboxamide in absolute ethanol was heated at 80°C overnight. The reaction mixture was reduced to dryness under reduced pressure to provide a residue. This residue was purified using flash chromatography (gradient eluent of 10-50% ethyl acetate in methylene chloride) to provide 6.47 g of an off-white foam.

Yield: 75%.

$^1$H NMR ($CDCl_3$): δ 1.29 (s, 9H), 1.25-2.05 (m, 2H), 2.20-2.35 (m, 2H), 2.55-2.70 (m, 11H), 2.85-3.10 (m, 3H), 3.24 (br.s, 1H), 3.82 (br.s, 1H), 3.98 (br.s, 1H), 4.99 (br.s, 2H), 5.16-5.18 (m, 1H), 5.80 (br.s, 1H), 7.05-7.38 (m, 10H).

IR ($CDCl_3$): 3600-3100 (br.), 3031, 2929, 1714, 1673, 1512, 1455, 1368, 1232, 1199, 1047 cm$^{-1}$.

MS (FD): m/e 536 (M$^+$H), 1068 (100).

B. [3S-*(3R*,4aR*,8aR*,2'S*,3'R*)*]-2-[3'-Amino-2'-hydroxy-4'-phenyl)butyl-decahydroisoquinoline-3-N-*t*-butylcarboxamide

A rapidly stirring suspension of 6.37 g (11.91 mmol) of the subtitled intermediate of Preparation 2A and 1.2 g of 10% palladium-on-carbon in 200 mL of absolute ethanol was placed under an atmosphere of hydrogen. After approximately 48 hours, the reaction mixture was filtered through celite and reduced to dryness under reduced pressure to provide 5.09 g of the desired subtitled intermediate. This compound was used without further purification.

$^1$H NMR ($CDCl_3$): δ 1.33 (s, 9H), 1.40-1.95 (m, 10H), 2.25-2.48 (m, 2H), 2.59-2.75 (m, 3H), 2.80-3.40 (m, 7H), 3.75-3.90 (m, 1H), 6.19 (br.s, 1H), 7.18-7.35 (m, 5H).

IR ($CDCl_3$): 3600-3100 (br.), 2929, 2865, 1671, 1515, 1455, 1367, 1245, 1047 cm$^{-1}$.

MS (FD) m/e 402 (M$^+$, 100).

Preparation 3

A. 2R-N(Benzyloxycarbonyl)amino-3-naphth-2-ylthio propanoic acid

To a solution of 1.28 g (8.00 mmol) of naphthalene-2-thiol in 30 mL tetrahydrofuran, was slowly added 1.77 g (8.16 g) of 60% sodium hydride, under nitrogen. After stirring for approximately 15 minutes, N(benzyloxycarbonyl)serine-β-lactone in 20 mL of tetrahydrofuran was slowly added. The reaction mixture was allowed to react at room temperature for approximately one hour, and then was concentrated under reduced pressure to provide a residue. This residue was dissolved in ethyl acetate and washed sequentially with 0.5N sodium bisulfate and a saturated brine solution. The resulting layers were separated and the organic layer was dried over sodium sulfate, filtered, and then concentrated under reduced pressure to provide a residue. This residue was purified using flash chromatography to provide 2.08 g of a pale yellow solid.

Yield: 68%.

[α]$_D$ -55.72°(*c* 1.0, MeOH).

$^1$H NMR ($CDCl_3$) : δ 3.42-3.61 (br.m, 2H), 5.53-5.76 (br.s, 1H), 4.85-5.08 (br.m, 2H), 5.54-5.76 (br.s, 1H), 7.06-7.97 (m, 12H).

IR (KBr): 3348, 3048, 1746, 1715, 1674, 1560, 1550, 1269, 1200, 1060 cm$^{-1}$.

MS (FD): m/e 381 (M$^+$), 381 (100).

| Analysis for $C_{20}H_{19}NO_4S$: | | | |
|---|---|---|---|
| Calcd: | C, 66.12; | H, 5.02; | N, 3.67; |
| Found: | C, 66.22; | H, 5.04; | N, 3.86. |

B. 3R-Benzyl 2-aza-3-(naphth-2-ylthiomethyl)-4-oxo-5-diazo pentanoate

To a cold (-30°C) solution of 15.38 g (40.3 mmol) of the subtitled intermediate from Preparation 3A in 230 mL of ethyl acetate and under nitrogen, was slowly added 5.62 mL (40.3 mmol) of triethylamine, via syringe. To the resulting solution was then added 7.84 mL (60.5 mmol) of isobutyl chloroformate, via syringe. In a separate flask, 10 g of N(methyl)-N(nitro)-N(nitroso)guanidine was carefully added to a bilayer mixture of 170 mL of diethylether and 170 mL of a 5N sodium hydroxide solution, resulting in a large evolution of gas. When this reaction was substantially complete, the organic layer was decanted from the aqueous layer onto potassium hydroxide and dried. This diazomethane formation and addition was repeated using identical quantities of diethylether and sodium hydroxide and 30 g of N(methyl)-N(nitro)-N(nitroso)guanidine. The resultant diazomethane reactant was then added to the mixed anhydride solution prepared above and the reaction mixture was allowed to react cold (-30°C) for approximately 20 minutes. When the reaction was substantially complete, as indicated by TLC, nitrogen was bubbled through the solution using a fire polished Pasteur pipet to remove any excess diazomethane and then the solution was concentrated under reduced pressure to provide a residue. This residue was purified using flash chromatography (eluent of 10% ethyl acetate in methylene chloride) to provide 13.62 g of a yellow oil.

Yield: 83%.

$^1H$ NMR ($CDCl_3$): δ 3.32-3.46 (m, 2H), 4.40-4.67 (m, 1H), 5.00-5.09 (m, 2H), 5.44 (s, 1H), 5.76 (d, J=7.8 Hz, 1H), 7.25-7.86 (m, 12H).

C. 3R-Benzyl 2-aza-3-(naphth-2-ylthiomethyl)-4-oxo-5-chloro pentanoate

A short burst (about 2 seconds) of anhydrous hydrochloric acid (gas) was passed through a cold (-20°C) solution of 13.62 g (33.59 mmol) of the subtitled intermediate from Preparation 3B in 230 mL of diethylether, resulting in the evolution of a gas. This procedure was repeated taking care not to add excess hydrochloric acid. When the reaction was substantially complete, as indicated by TLC, the solution was concentrated under reduced pressure to provide a residue. This residue was purified using flash chromatography (eluent of 10% ethyl acetate in methylene chloride) to provide 12.05 g of a pale tan solid.

Yield: 87%.

$^1H$ NMR ($CDCl_3$): δ 3.41 (dd, J=12,6 Hz, 1H), 3.53 (dd, J=12,6 Hz, 1H), 4.18 (AB q, J=41.9 Hz, J=15.9 Hz, 2H), 4.77 (dd, J=9, 3 Hz, 1H), 5.04 (AB q, J=12 Hz, J=10.4 Hz, 2H), 5.59 (d, J=7 Hz, 1H), 7.24-7.85 (complex, 12H).

$[\alpha]_D$ -80.00° (*c* 1.0, MeOH).

IR ($CHCl_3$): 3426, 3031, 3012, 1717, 1502, 1340, 1230, 1228, 1045 $cm^{-1}$.

MS (FD): m/e 413 ($M^+$), 413 (100).

| Analysis for $C_{22}H_{20}NO_3SCl$: | | | |
|---|---|---|---|
| Calcd: | C, 63.84; | H, 4.87; | N, 3.38; |
| Found: | C, 64.12; | H, 4.95; | N, 3.54. |

D. [3R-(*3R*,4S**)] Benzyl 2-aza-3-(naphth-2-ylthiomethyl)-4-hydroxy-5-chloro pentanoate

To a cold (0°C) solution of 530 mg (1.28 mmol) of the subtitled intermediate of Preparation 3C, in 10 mL of tetrahydrofuran and 1 mL of water, was added 73 mg (1.92 mmol) of sodium borohydride. When the reaction was substantially complete as indicated by TLC, the solution was adjusted to pH 3 using 10 mL of an aqueous saturated ammonium chloride solution and 500 µL of a 5N hydrochloric acid solution. The resultant solution was extracted twice with methylene chloride and the combined organic layers were washed with water, dried over sodium sulfate, filtered and then concentrated under reduced pressure to provide a residue. This residue

was purified using radial chromatography (eluent of methylene chloride) to provide 212 mg of a tan solid.
Yield: 40%.
$^1$H NMR ($CDCl_3$): δ 3.40 (s, 2H), 3.61-3.71 (m, 2H), 3.97-3.99 (m, 2H), 4.99 (s, 2H), 5.16 (br.s, 1H), 7.21-7.83 (complex, 12H).
MS (FD): m/e 415 ($M^+$) , 415 (100).
$[\alpha]_D$ -47.67 (*c* 0.86, MeOH).
IR ($CHCl_3$): 3630, 3412, 3011, 1720, 1502, 1236, 1044 $cm^{-1}$.

| Analysis for $C_{22}H_{22}NO_3ClS$: | | | |
|---|---|---|---|
| Calcd: | C, 63.53; | H, 5.33; | N, 3.37; |
| Found: | C, 63.72; | H, 5.60; | N, 3.64. |

E. [3R-*(3R*,4S*)*] Benzyl 2-aza-3-oxiranyl-4-naphth-2-ylthio butanoate

A solution of 31 mg (0.55 mmol) of potassium hydroxide in 1 mL of ethanol was added to a solution of 190 mg (0.46 mmol) of the subtitled intermediate of Preparation 3D, in 6 mL of a 1:2 ethanol/ethyl acetate solution. When the reaction was substantially complete, as indicated by TLC, the reaction mixture was poured into a water/methylene chloride mixture. The resulting layers were separated, and the organic layer was washed with water, dried over sodium sulfate, filtered and then concentrated under reduced pressure to provide a residue. This residue was purified using radial chromatography (eluent of 10% ethyl acetate in methylene chloride) to provide 172 mg of a light tan solid.
Yield: 99%.
$^1$H NMR ($CDCl_3$): δ 2.76 (br.s, 2H) 3.01 (br.s, 1H), 3.31 (d, J=5 Hz, 2H), 3.77 (br.s, 1H), 5.05 (s, 2H), 5.22 (d, J=6 Hz, 1H), 7.25-7.85 (complex, 12H).
$[a]_D$ -125.42° (*c* 0.59, MeOH).
MS (FD): m/e 379 ($M^+$), 379 (100).
IR ($CHCl_3$) : 3640, 3022, 2976, 1720, 1502, 1235, 1045 $cm^{-1}$.

| Analysis for $C_{22}H_{21}NO_3S$: | | | |
|---|---|---|---|
| Calcd: | C, 69.63; | H, 5.58; | N, 3.69; |
| Found: | C, 69.41; | H, 5.53; | N, 3.64. |

F. [3R-*(3R*,4R*,3'S*,4a'S*,8a'S*)*] Benzyl, [2-Aza-3-naphth-2-ylthiomethyl)-4-hydroxy-5-(3'-1"-N(*t*-butyl)amino-1"-oxomethyl)decahydroisoquinolin-2'-yl)] pentanoate

A solution was prepared containing 165 mg (0.40 mmol) of the subtitled intermediate of Preparation 3E and 94 mg (0.43 mmol) of 3-(1-N(*t*-butyl)amino-1-oxomethyl)- decahydro-(2H)-isoquinoline in 5 mL of ethanol. The resulting reaction mixture was allowed to react at 80°C for approximately 19 hours. The solution was then cooled to room temperature and concentrated under reduced pressure to provide a residue. This residue was purified using radial chromatography (eluent of 10% ethyl acetate in methylene chloride) to provide 103 mg of an off-white foam.
Yield: 42%.
$^1$H NMR ($CDCl_3$) : δ 1.10-1.73 (m, 20H), 2.13-2.31 (m, 2H), 2.44-2.53 (m, 1H), 2.56-2.68 (m, 1H), 2.86-2.97 (m, 1H), 3.52 (br.s, 2H), 4.02 (br.s, 2H), 4.98 (s, 2H), 5.65 (s, 1H), 5.94 (s, 1H), 7.25-7.83 (complex, 13H).
MS (FD): m/e 629 ($M^+$) , 138 (100).
$[\alpha]_D$ -92.45° (*c* 1.06, MeOH).
IR ($CHCl_3$) : 3429, 3010, 2929, 1713, 1670, 1514, 1455, 1047 $cm^{-1}$.

| Analysis for $C_{35}H_{47}N_3O4S$: | | | |
|---|---|---|---|
| Calcd: | C, 69.98; | H, 7.67; | N, 6.80; |
| Found: | C, 69.86; | H, 7.78; | N, 6.58. |

G. [2R-*(2R*,3R*,3'S*,4a'S*,8a'S*)*]-N(*t*-Butyl)-2'-(2-hydroxy-3-amino-4-(naphth-2-ylthio)]butyl decahydroisoquinoline-3'-carboxamide

A solution was prepared containing 50 mg (0.081 mmol) of the subtitled intermediate of Preparation 3F and 1 mL of a 38% aqueous hydrobromic acid solution in acetic acid. The resultant reaction mixture was allowed to react at room temperature for approximately 1 hour and then was concentrated under reduced pressure to provide a residue. This residue was slurried with toluene and then concentrated under reduced pressure to provide 61 mg of the desired subtitled intermediate. This compound was used without further purification.

$^1$H NMR ($CDCl_3$): δ 1.14 (s, 1H), 1.17-2.07 (complex, 15H), 2.66-2.87 (m, 2H), 3.21-3.25 (m, 2H), 3.75 (d, J=12 Hz, 1H), 3.85 (d, J=6 Hz, 1H), 4.36-4.47 (m, 1H), 6.73 (s, 1H), 7.39-7.90 (complex, 7H).

MS (FD): 483 ($M^+$), 483 (100).

Preparation 4

A. 2R-2-N(Benzyloxycarbonyl)amino-3-phenylthio propanoic acid

The desired subtitled intermediate was prepared substantially in accordance with the procedure detailed in Procedure 3A, using 13.1 mL (127 mmol) of thiophenol, 4.6 g (117 mmol) of a 60% sodium hydride solution and 25.6 g (116 mmol) of (L)-N(benzyloxycarbonyl)serine β-lactone in 450 mL of tetrahydrofuran to provide a residue. This residue was purified using flash chromatography (gradient eluent of 0-2% acetic acid in a 4:1 methylene chloride/ethyl acetate mixture) to provide 27.9 g of a white solid.

Yield: 72%.

$^1$H NMR ($CDCl_3$): δ 7.55-7.18 (m, 10H), 5.55 (d, J=7 Hz, 1H), 5.08 (s, 2H), 4.73-4.60 (m, 1H), 3.55-3.30 (m, 2H).

IR (KBr): 3304, 3035, 1687, 1532, 736 $cm^{-1}$.

MS (FD): m/e 332, 288, 271, 181.

| Analysis for $C_{17}H_{17}NO_4S$: | | | |
|---|---|---|---|
| Calcd: | C, 61.61; | H, 5.17; | N, 4.23; |
| Found: | C, 61.69; | H, 5.22; | N, 4.47. |

B. 3R-Benzyl, 2-aza-3-phenylthiomethyl-4-oxo-5-diazo pentanoate

The desired subtitled compound was prepared substantially in accordance with the procedure detailed in Procedure 3B, using 12.1 g (37 mmol) of the subtitled compound of Preparation 4A, 5.09 mL (37 mmol) of triethylamine, 7.13 mL (55 mmol) isobutyl chloroformate, 146 mmol of a diazomethane solution to provide a residue. The diazomethane solution was prepared using 100 mL of diethylether, 150 mL of a 5N sodium hydroxide solution and 21 g (146 mmol) of N(methyl)-N(nitro)-N(nitroso)-guanidine as described in Preparation 1B. This residue was purified using flash chromatography (gradient eluent of 0-5% ethyl acetate in methylene chloride) to provide a yellow oil.

Yield: 73%.

$^1$H NMR ($CDCl_3$) : δ 7.50-7.19 (m, 10H), 5.62 (d, J=7 Hz, 1H), 5.47 (br.s, 1H), 5.11 (s, 2H), 4.50-4.32 (m, 1H), 3.33 (d, J=6 Hz, 1H).

IR (KBr) : 3012, 2115, 1720, 1501, 1367, 1228 $cm^{-1}$.

MS (FD): m/e 356, 328, 242.

C. 3R-Benzyl, 2-aza-3-phenylthiomethyl-4-oxo-5-chloro pentanoate

The desired subtitled compound was prepared substantially in accordance with the procedure detailed in Procedure 3C, using 22.3 g (63 mmol) of the subtitled compound of Preparation 4B and small quantities of hydrochloric acid (gas) in 400 mL of diethylether to provide 21 g of a white solid. This solid was used without further purification.

$^1H$ NMR ($CDCl_3$): δ 7.50-7.15 (m, 10H), 5.56 (dd, J=2,6.7 Hz, 1H), 5.11 (s, 2H), 4.78-4.67 (m, 1H), 4.20 (d, J=15.9 Hz, 1H), 4.12 (d, J=15.9 Hz, 1H), 3.48-3.23 (m, 2H).

IR (KBr) : 3349, 1732, 1684, 1515, 1266 $cm^{-1}$.

MS (FD): m/e 363 ($M^+$).

| Analysis for $C_{18}H_{18}NO_3SCl$: | | | |
|---|---|---|---|
| Calcd: | C, 59.42; | H, 4.99; | N, 3.85; |
| Found: | C, 59.57; | H, 5.09; | N, 4.13. |

D. [3R-(*3R*,4S**)] Benzyl, 2-aza-3-phenylthiomethyl-4-hydroxy-5-chloro pentanoate

The subtitled compound was prepared substantially in accordance with the procedure detailed in Procedure 3D, using 21 g (58 mmol) of the subtitled compound of Preparation 4C, 2.4 g (63 mmol) of sodium borohydride in 300 mL of tetrahydrofuran to provide a residue. This residue was purified using flash chromatography (gradient eluent of 0-2% methanol in methylene chloride) followed by flash chromatography (gradient eluent of 0-2% ethyl acetate in chloroform) and then recrystallized from methylene chloride at -78°C to provide 8.3 g of the desired subtitled compound.

Yield: 39%.

$^1H$ NMR ($CDCl_3$): δ 7.47-7.19 (m, 10H), 5.22-5.03 (m, 1H), 5.09 (s, 2H), 4.01-3.89 (m, 2H), 3.75-3.58 (m, 2H), 3.32 (d, J=4 Hz, 2H).

IR (KBr): 3321, 2951, 1688, 1542, 1246, 738 $cm^{-1}$.

MS (FD): m/e 366 ($M^+$), 119.

| Analysis for $C_{18}H_{20}NO_3SCl$: | | | |
|---|---|---|---|
| Calcd: | C, 59.09; | H, 5.51; | N, 3.83; |
| Found: | C, 59.03; | H, 5.50; | N, 3.96. |

E. [3R-(*3R*,4S**)] Benzyl, 2-aza-3-oxiranyl-4-phenylthio butanoate

The desired subtitled compound was prepared substantially in accordance with the procedure detailed in Procedure 3E, using 8.3 g (23 mmol) of the subtitled compound of Preparation 4D, 1.4 g (25 mmol) of potassium hydroxide in 400 mL of ethanol to provide a residue. This residue was purified using flash chromatography (gradient eluent of 0-2% ethyl acetate in methylene chloride) to provide 6.4 g of a white solid.

Yield: 85%.

$^1H$ NMR ($CDCl_3$): δ 7.45-7.15 (m, 10 H), 5.12 (s, 1H), 5.08 (s, 2H), 3.77-3.62 (m, 1H), 3.21 (d, J=6 Hz, 2H), 2.99 (m, 1H), 2.77 (m, 2H).

IR (KBr): 3303 ,3067, 1694, 1538, 1257, 741 $cm^{-1}$.

MS (FD) m/e 329.

| Analysis for $C_{32}H_{45}N_3O_4S$: | | | |
|---|---|---|---|
| Calcd: | C, 65.63; | H, 5.81; | N, 4.25; |
| Found: | C, 65.48; | H, 5.82; | N, 4.29. |

F. [3R-*(3R*,4R*,3'S*,4a'S*,8a'S*)*] Benzyl, [2-aza-3-phenylthiomethyl-4-hydroxy-5-(3'-(1"-N(*t*-butyl)amino-1"- oxomethyl) decahydroisoquinolin-2'-yl)]pentanoate

The desired subtitled compound was prepared substantially in accordance with the procedure detailed in Procedure 3F, using 6.3 g (19 mmol) of the subtitled compound of Preparation 4E, 5 g (21 mmol) of 3-[N(*t*-butyl)aminocarbonyl]decahydro-(2H)-isoquinoline in 300 mL of ethanol to provide a residue. This residue was purified using flash chromatography (gradient eluent of 0-20% ethyl acetate in methylene chloride) to provide 4.3 g of a white solid.

Yield: 40%.

$^1$H NMR ($CDCl_3$): δ 7.41-7.11 (m, 10H), 5.90 (d, J=5 Hz, 1H), 5.64 (s, 1H), 5.05 (d, J=4 Hz, 2H), 4.08-3.90 (m, 2H), 3.40 (d, J= 6, 2H), 3.05 (s, 1H), 2.95-2.85 (m, 1H), 2.62-2.45 (m, 2H), 2.28-2.15 (m, 2H), 2.05-1.88 (m, 2H), 1.78-1.10 (m, 7H), 1.29 (s, 9H).

IR(KBr): 3330, 2925, 2862, 1706, 1661, 1520, 1454, 1246, 738, 694 $cm^{-1}$.

MS (FD): m/e 568 ($M^+$), 467.

| Analysis for $C_{32}H_{45}N_3O_4S$: | | | |
|---|---|---|---|
| Calcd: | C, 67.69; | H, 7.99; | N, 7.40; |
| Found: | C, 67.64; | H, 8.20; | N, 7.45. |

G. [2R-*(2R*,3R*,3'S*,4a'S*,8a'S*)*]-N(*t*-Butyl)-2'-[2-hydroxy-3-amino-4-phenylthiolbutyl decahydroisoquinoline-3'-carboxamide

The desired subtitled compound was prepared substantially in accordance with the procedure detailed in Procedure 3G using 1 g (1.8 mmol) of the subtitled compound of Preparation 4F and 40 mL of a 30% hydrobromic acid in acetic acid solution, with the exception that the crude material was dissolved in 30 mL of methanol. To the resulting solution, was added 2 mL of diethylamine and 2 mL of concentrated ammonium hydroxide and then the mixture was concentrated under reduced pressure to provide a residue. This residue was redissolved in water and ethyl acetate. The resulting layers were separated and the organic layer was washed sequentially with an aqueous sodium bicarbonate solution and brine, dried over sodium sulfate, filtered and then reduced to dryness under reduced pressure to provide a residue. This residue was purified using flash chromatography (gradient eluent of 0-10% methanol in chloroform (containing 3 drops of ammonium hydroxide per 1000 mL of chloroform)) to provide 0.54 g of a white foam.

Yield: 71%.

$^1$H NMR ($CDCl_3$): δ 7.41-7.16 (m, 5H), 6.07 (s, 1H), 3.78-3.70 (m, 1H), 3.45-3.38 (m, 1H), 3.03-2.84 (m, 3H), 2.38-2.20 (m, 3H), 2.00-1.05 (m, 12H), 1.33 (s, 9H).

IR (KBr) : 2924, 2862, 1660, 1517, 1454, 1439, 737, 691 $cm^{-1}$.

MS (FD): m/e 434 ($M^+$), 293.

Preparation 5

A. Pyrazine-2-N-(*t*-butyl)carboxamide

To a slurry of 50 g (0.403 mol) pyrazine-2-carboxylic acid in 600 mL of tetrahydrofuran and 100 mL of dimethylformamide, was added 65.9 g (0.407 mol) of carbonyldiimidazole. The resultant reaction mixture was reacted at 50°C until gas evolution ceased. After the reaction mixture cooled, 73.5 g (1.00 mol) of *t*-butylamine was slowly added. The reaction mixture was reacted for approximately thirty minutes, concentrated under reduced pressure, redissolved in 500 mL of methylene chloride and then washed sequentially with water, hydrochloric acid (pH 2), saturated sodium bicarbonate, water, 1M potassium hydroxide, and brine, dried over sodium sulfate, and concentrated to provide 68.5 g of a white solid.

Yield: 95%.

$^1$H NMR ($CDCl_3$) : δ 1.51 (s, 9H) , 7.73 (br.s, 1H), 8.49 (m, 1H), 8.72 (m, 1H), 9.38 (s, 1H).

B. (+/-)-Piperazine-2-N-(*t*-butyl)carboxamide

A mixture of 68.5 g (0.382 mol) of the subtitled compound of Preparation 5A, 70 g (0.308mol) of platinum

oxide in 186 mL of ethanol was heated overnight at 40°C under a hydrogen atmosphere (60 psi). The resultant crude material was filtered and the filtrate was concentrated to provide 65 g of white solid.
Yield: 95%.
MS (FD): m/e 185 ($M^+$, 100).

C. (+/-)-4-(Pyrid-3'-ylmethyl)piperazine-2-N-(*t*-butyl)carboxamide

To a solution of 5.0 g (0.027 mol) of the subtitled compound of Preparation 5B in 160 mL of a 1:1 mixture of water and acetonitrile, was added 18.65 g (0.135 mol) of potassium carbonate. The resultant mixture was vigorously stirred during the addition of 4.43 g (0.027 mol) of 3-chloromethylpyridine hydrochloride and then allowed to react overnight. The resultant reaction mixture was concentrated under reduced pressure, slurried in a solution of 20% isopropanol in chloroform and washed sequentially with water and brine, dried over sodium sulfate, filtered and then concentrated to provide a residue. This residue was purified using flash chromatography (eluent of 5% methanol in methylene chloride containing 1% ammonium hydroxide) to provide 1.34 g of a clear yellow oil.
Yield: 18%
$^1H$ NMR ($CDCl_3$): δ 1.10 (s, 9H), 1.89-2.01 (m, 2H), 2.35 (m, 1H), 2.57-2.74 (m, 4H), 3.09 (m, 1H), 3.27 (s, 2H), 6.71 (br.s, 1H), 7.03 (m, 1H), 7.44 (m, 1H) 8.26 (m, 2H).
IR (KBr) : 3691, 3611, 3366, 2974, 1666, 1602, 1521, 1479, 1456, 1427, 1393, 1366, 1324, 1139, 1047, 839 $cm^{-1}$.
MS (FD): m/e 276 ($M^+$, 100).

D. [2S-*(2R*,2'S*,3'R*)*]-1-[2'-Hydroxy-3'-(N-benzyloxycarbonyl)amino-4'-phenylbutyl]-4-(pyrid-3"-ylmethyl) piperazine-2-N-(*t*-butyl)carboxamide

A solution containing 0.377 g (1.27 mmol) of [1S-*(1R*,1'R*)*]-1-[(1'-N-Benzyloxycarbonyl)amino-2'-phenyl) ethyl]oxirane and 0.350 g (1.27 mmol) of the subtitled compound of Preparation 5C in 12 mL of isopropanol was reacted at 45°C for approximately forty eight hours. The reaction mixture was cooled and then concentrated under reduced pressure to provide a crude material. This material was purified using radial chromatography (6mm plate; gradient eluent of 5-10% isopropanol in methylene chloride) to provide 120 mg of isomer A and 68 mg of isomer B.
Yield: 26% overall.
Isomer A:
$^1H$ NMR ($CDCl_3$): δ 1.33 (s, 9H), 2.26-2.89 (m, 13H), 3.29 (m, 1H), 3.45 (s, 2H), 3.79-3.95 (m, 3H), 4.73 (br.s, 1H), 4.97 (br.s, 2H), 5.20 (m, 1H), 7.14-7.29 (m, 6H) 7.57 (m, 1H), 7.82 (br.s, 1H), 8.53 (m, 2H).
IR (KBr) : 3692, 3434, 2970, 2829, 1714, 1661, 1604, 1579, 1512, 1455, 1427, 1393, 1365, 1231, 1149, 1029, 909 $cm^{-1}$.
MS (FD): m/e 573 ($M^+$, 100).

E. [2S-*(2R*,2'S*,3'R*)*]-1-[2'-Hydroxy-3'-amino-4'-phenyl]butyl-4-(pyrid-3"-ylmethyl) piperazine-2-N-(*t*-butyl)carboxamide

A solution containing 0.062 g (0.11 mmol) of the subtitled compound of Preparation 5D (isomer A) was stirred for approximately ninety minutes in 1.5 mL of a solution of 30% hydrobromic acid in acetic acid. The resultant mixture was concentrated, azeotroped three times with toluene, redissolved in methanol containing 1 mL each of diethylamine and ammonium hydroxide and then concentrated under reduced pressure to provide a residue. This residue was purified using radial chromatography (2mm plate; gradient eluent of 15-25% methanol in methylene chloride containing 1% ammonium hydroxide) to provide 13 mg of a white solid.
Yield: 28%.
$^1H$ NMR ($CDCl_3$): δ 1.33 (s, 9H), 2.36-3.21 (m, 15H), 3.47 (d, 2H), 3.75 (m, 1H), 7.19-7.30 (m, 6H) 7.57 (m, 2H), 8.52 (m, 2H).
MS (FD): m/e 440 ($M^+$, 100).

Preparation 6

A. [2S-(*2R*,2'S*,3'S**)]-1-[3'-N-(Benzyloxycarbonyl)amino-2'-hydroxy-4'-phenylthiobutyl]-4-[pyrid-3"-ylmethyl] piperazine-2-N-*t*-butylcarboxamide [isomer B]

A solution of 596 mg (1.81 mmol) of [1S-*(1R*,1'S*)]*-1-[1'-N-(benzyloxycarbonyl)amino-2'-(phenylthio)ethyl]oxirane and 500 mg (1.81 mmol) of the subtitled compound of Preparation 5C in 15 mL of isopropanol were heated at 43°C for approximately forty-eight hours. The reaction was monitored using TLC (10% isopropanol in methylene chloride containing 1% ammonium hydroxide; Isomer A $R_f$ = 0.7; Isomer B $R_f$ = 0.6). When the reaction was substantially complete, the reaction mixture was concentrated under reduced pressure to provide a residue. This residue was purified using radial chromatography (6 mm plate; gradient eluent of 5-15% isopropanol in methylene chloride containing 1% ammonium hydroxide) to provide 200 mg of isomer A as a light tan foam and 119 mg of an off-white foam (isomer B).

Isomer A:
Yield: 18%.
$^1$H NMR ($CDCl_3$): δ 1.31 (s, 9H), 2.25-2.62 (m, 7H), 2.78-2.95 (m, 2H), 2.98-3.08 (m, 1H), 3.10-3.25 (m, 2H), 3.40-3.55 (m, 2H), 3.72-3.85 (m, 1H), 3.90-4.00 (m, 1H), 5.05 (s, 2H), 7.01 (br.s, 1H), 7.10-7.40 (m, 11H), 7.62 (d, J=7.8 Hz, 1H), 8.49 (s, 2H).
MS (FD): m/e 606 ($M^+$, 100).

| Analyse for $C_{33}H_{43}N_5O_4S$: | | | |
|---|---|---|---|
| Calcd: | C, 65.42; | H, 7.15; | N, 11.56; |
| Found: | C, 65.38; | H, 7.27; | N, 11.36. |

Isomer B:
Yield: 11%.
$^1$H NMR ($CDCl_3$): δ 1.33 (s, 9H), 2.25-2.85 (m, 8H), 3.20-3.32 (m, 3H), 3.47 (s, 2H), 3.78-3.95 (m, 2H), 5.06 (s, 2H), 5.30-5.38 (m, 1H), 7.10-7.42 (m, 12H), 7.55-7.85 (m, 2H), 8.50-8.60 (m, 2H).
MS (FD): m/e 606 (M), 497 (100).

| HR MS(FAB) for $C_{33}H_{44}N_5O_4S$: | |
|---|---|
| Calcd: | 606.3114; |
| Found: | 606.3141. |

B. [2S-(*2R*,2'S*,3'S**)]-1-[2'-Hydroxy-3'-amino-4'-phenylthiobutyl]-4-[pyrid-3"-ylmethyl] piperazine-2-N-*t*-butylcarboxamide

A solution of 110 mg (0.18 mmol) of isomer B from Preparation 6A in 5 mL of 30% hydrobromic acid in acetic acid was stirred at room temperature for approximately 1 hour. The reaction mixture was concentrated under reduced pressure to provide a residue. This residue was redissolved in 4 mL of ammonium hydroxide. The resultant solution was extracted four times with 10 mL portions of a 10% solution of isopropanol in chloroform. The combined organic layers were dried over sodium sulfate, filtered and concentrated under reduced pressure to provide a residue. This residue was purified using radial chromatograghy (2 mm plate; gradient eluent of 10-30% methanol in methylene chloride containing 1% ammonium hydroxide) to provide 65 mg of a light yellow foam.
Yield: 72%.
$^1$H NMR ($CDCl_3$): δ 1.25 (s, 9H), 2.25-2.78 (m, 7H), 3.00-3.32 (m, 4H), 3.47 (s, 2H), 3.60-3.75 (m, 1H), 4.18-4.35 (m, 1H), 6.90-7.65 (m, 9H), 8.40-8.60 (m, 2H).
MS (FD): m/e 473 ($M^+$, 100).

Preparation 7

3-Methoxycarbonyl-4-methyl benzoate, pentafluorophenyl ester

To a solution of 1.0 g (5.15 mmol) of 3-methoxycarbonyl-4-methyl benzoic acid and 1.9 g (10.3 mmol) of pentafluorophenol in 15 mL of methylene chloride, was added 2.0 g (10.30 mmol) of EDC. The resulting reaction mixture was reacted for approximately four hours and then was diluted with methylene chloride. The reaction mixture was washed four times with 1M potassium carbonate, dried over sodium sulfate, filtered and then concentrated under reduced pressure to provide a crude residue. This residue was purified using column chromatography (eluent of 50% hexane in ethyl acetate) to provide 1.80 g of a white solid.
Yield: 97%.

Example 1

A. N-[(1'-Oxo-1'-(3"-iodo-4"-methyl)phenyl)methyl]-1,2,3,4-tetrahydroisoquinoline

To a solution containing 5.00 g (19.1 mmol) of 3-iodo-4-methylbenzoic acid and 3.40 g (21.0 mmol) of 1,1-carbonyldiimidazole in 80 mL of tetrahydrofuran, under nitrogen, was added 2.7 mL (21.0 mmol) of 1,2,3,4-tetrahydroisoquinoline, by syringe. The resulting reaction mixture was allowed to react for approximately 2 hours and then reduced to dryness under reduced pressure to provide a residue. This residue was redissolved in 100 mL of ethyl acetate, washed sequentially with saturated sodium bicarbonate and brine solutions, dried over sodium sulfate, filtered and then reduced to dryness under reduced pressure to provide a light yellow oil. This oil was purified using flash chromatography (gradient eluent of 14-20% hexane in ethyl acetate) to provide 6.92 g of a clear oil which solidified on standing.
Yield: 96%.
$^1$H NMR ($CDCl_3$): δ 2.46 (s, 3H), 2.90 (br.s, 2H), 3.65 (br.s, 1H), 3.95 (br.s, 1H), 4.85 (br.s, 1H), 7.17-7.34 (m, 6H), 7.90 (s, 1H).
IR ($CDCl_3$) : 3010, 1624, 1586, 1547, 1497, 1370, 1300, 1253, 1108, 1050, 1035, 831 $cm^{-1}$.
MS (FD): m/e 377 ($M^+$, 100).

| Analysis for $C_{17}H_{16}NOI$: | | | | |
|---|---|---|---|---|
| Calcd: | C, 54.13; | H, 4.28; | N, 3.71; | I, 33.64; |
| Found: | C, 53.89; | H, 4.24; | N, 3.61; | I, 33.52. |

B. N-[1'-Oxo-1'-(3"-methoxycarbonyl-4"-methyl)phenyl]methyl-1,2,3,4-tetrahydroisoquinoline

A solution containing 6.35 g (16.8 mmol) of the subtitled compound of Example 1A, 3.35 mL (16.8 mmol) of dicyclohexylamine and 1.18 g (1.68 mmol) of bis(triphenylphosphine)palladium (II) chloride in 150 mL of anhydrous methanol was vigorously stirred under an atmosphere of carbon monoxide. When the reaction was substantially complete, as indicated by thin layer chromatography (TLC), the mixture was reduced to dryness under reduced pressure to provide a residue. This residue was redissolved in 200 mL of ethyl acetate and the resulting mixture filtered through celite to remove insoluble organic salts. The filtrate was then washed sequentially with saturated sodium bicarbonate and brine solutions, dried over sodium sulfate, filtered and then reduced to dryness under reduced pressure. The resultant material was purified using flash chromatography (gradient eluent of 14-20% ethyl acetate in hexane) to provide 4.54 g of the desired subtitled compound as a clear oil.
Yield: 87%.
$^1$H NMR ($CDCl_3$) : δ 2.64 (s, 3H), 2.90 (br.s, 2H), 3.89 (s, 3H), 3.98 (br.s, 1H), 4.59 (br.s, 1H), 4.87 (br.s, 1H), 7.05-7.51 (m, 6H), 8.03 (s, 1H).
IR ($CDCl_3$) 3010, 1722, 1626, 1584, 1497, 1436, 1306, 1268, 1236, 1210, 1149, 1109, 1085 $cm^{-1}$.
MS (FD): m/e 309 ($M^+$, 100).

| Analysis for $C_{19}H_{19}NO_3$: | | | |
|---|---|---|---|
| Calcd: | C, 73.77; | H, 6.19; | N, 4.53; |
| Found: | C, 73.95; | H, 6.43; | N, 4.57. |

C. N-[1'-Oxo-1'-(3"-carboxy-4"-methyl)phenyl]methyl-1,2,3,4-tetrahydroisoquinoline

To a solution containing 4.25 g (13.8 mmol) of the subtitled compound of Example 1B in 200 mL of a 3:1 tetrahydrofuran/water mixture, was added 661 mg (27.6 mmol) of lithium hydroxide. The reaction mixture was allowed to react for approximately 24 hours. The resulting reaction mixture was then concentrated under reduced pressure and acidified (pH 2-3) by the dropwise addition of 1N hydrochloric acid, resulting in the precipitation of the crude product. The resulting suspension was combined with 75 mL of ethyl acetate, and the resulting aqueous and organic layers were separated. The aqueous phase was extracted twice with ethyl acetate and the combined organic layers were then washed with brine, dried over sodium sulfate, filtered and then reduced to dryness under reduced pressure to provide a residue. This residue was filtered through silica gel using a solution of 15% methanol in methylene chloride as an eluent to provide 4.05 g of an off-white foam.

$^1H$ NMR ($CDCl_3$): δ 2.68 (s, 3H), 2.92 (br.s, 2H), 3.67 (br.s, 1H), 4.00 (br.s, 1H), 4.62 (br.s, 1H), 4.91 (br.s, 1H), 7.18-7.58 (m, 6H), 8.17 (s, 1H).

IR ($CDCl_3$): 3500-2500 (br.), 1699, 1625, 1584, 1498, 1445, 1371, 1301, 1237, 1202, 1167, 1060, 981, 934, 838 $cm^{-1}$.

MS (FD): m/e 295 ($M^+$, 100).

| Analysis for $C_{18}H_{17}NO_3$: | | | |
|---|---|---|---|
| Calcd: | C, 73.20; | H, 5.80; | N, 4.74; |
| Found: | C, 73.14; | H, 5.90; | N, 4.44. |

D. [3'''S-*(3'''R*,4'''S*)]*-N-[1'-Oxo-1'-(3"-[1'''-oxo-2'''-aza-3'''-phenylmethyl-4'''-hydroxy-5'''-(2''''-N-*t*-butylcarbamido)phenyl]pentyl-4"-methyl)phenyl]methyl-1,2,3,4-tetrahydroisoquinoline

To a cold (-10°C) solution containing 59 mg (0.2 mmol) of the subtitled compound of Example 1C, 48 mg (0.2 mmol) of the subtitled intermediate of Preparation 1D and 27 mg (0.2 mmol) of 1-hydroxybenzotriazole hydrate (HOBT·$H_2O$) in 4 mL of anhydrous tetrahydrofuran, was added 41.3 mg (0.2 mmol) of 1,3-dicyclohexylcarbodiimide (DCC). After approximately 10 minutes, the reaction mixture was warmed to room temperature and allowed to react overnight. The reaction mixture was then diluted with 50 mL of ethyl acetate, filtered through celite, and washed sequentially with water, a saturated sodium bicarbonate and a saturated brine solution. After drying over sodium sulfate, the reaction mixture was filtered and concentrated under reduced pressure to provide a crude material. This material was purified using rotary chromatography (1 mm plate, 3:2 methylene chloride/ethyl acetate) to provide 30.2 mg of the desired subtitled compound as a white foam.

Yield: 24%.

$[a]_D$ -0.99° (c=1.014, MeOH).

$^1H$ NMR ($CDCl_3$): δ 1.45 (s, 9H), 2.21 (s, 3H), 2.78-3.20 (m, 7H), 3.53 (br.s, 1H), 3.89-3.95 (m, 2H), 4.55-4.62 (m, 2H), 4.85 (br.s., 1H), 5.92 (s, 1H), 6.27 (d, J=9.7 Hz, 1H), 7.09-7.43 (m, 16H).

IR ($CDCl_3$): 3428, 3500-3100 (br.), 3010, 1644, 1514, 1455, 1395, 1368, 1300, 1237, 1223, 1210 $cm^{-1}$.

MS (FD): m/e 618 ($M^+$, 100).

| Analysis for $C_{39}H_{43}N_3O_4$: | | | |
|---|---|---|---|
| Calcd: | C, 75.82; | H, 7.02; | N, 6.80; |
| Found: | C, 75.60; | H, 6.89; | N, 6.87. |

Example 2

A. 5-Methyl-3-methoxycarbonyl benzoic acid

A solution containing 23.93 g (131 mmol) of 5-methylisopthalic acid and 18.2 mL (137 mmol) of N,N-dimethylformamide dimethyl acetal in 200 mL of methylene chloride was heated to reflux temperature and allowed to react for approximately 2 hours. The reaction mixture was then concentrated under reduced pressure and the monoacid was separated from the diacid using crude flash chromatography (7% methanol in methylene chloride: $R_f$(monoacid)=0.5; $R_f$(diacid)=0.1-0.3). The fractions containing the monoacid were combined and reduced to dryness under reduced pressure to provide a residue. This residue was then dissolved in 200 mL of ethyl acetate and extracted twice with 150 mL portions of 1M potassium carbonate. The combined aqueous extracts were acidified using 1N hydrochloric acid (pH 2) and the resulting solution was then extracted three times with 150 mL portions of ethyl acetate. The organic extracts were combined, washed with brine, dried over sodium sulfate, filtered and then reduced to dryness under reduced pressure to provide a white solid. This solid was purified by recrystallization from an ethyl acetate/hexane solution) to provide 6.65 g of small white needles.
Yield: 26%.
$^1$H NMR ($CDCl_3$): δ 2.47 (s, 3H), 3.95 (s, 3H), 8.10 (br.s, 2H), 8.57 (s, 1H).
IR ($CDCl_3$) : 3400-2200 (br.), 1721, 1606, 1441, 1272, 1214, 1129, 985, 757, 703, 584, 475 $cm^{-1}$.
MS (FD): m/e 194 ($M^+$, 100).

| Analysis for $C_{10}H_{10}O_4$: | | |
|---|---|---|
| Calcd: | C, 61.85; | H, 5.19; |
| Found: | C, 61.83; | H, 5.31. |

B. N-[(1'-Oxo-1'-(3"-methoxycarbonyl-5"-methyl)phenyl]methyl-1,2,3,4-tetrahydroisoquinoline

The subtitled compound was prepared substantially in accordance with the procedure detailed in Example 1A, using 1.0 g (5.15 mmol) of the subtitled intermediate of Example 2A, 0.88 g (5.40 mmol) of 1,1-carbonyldiimidazole and 0.68 mL (5.40 mmol) of 1,2,3,4-tetrahydroisoquinoline in 10 mL of anhydrous tetrahydrofuran to provide a residue. This residue was purified using flash chromatography (gradient eluent of 30-45% ethyl acetate in hexane) to provide 1.48 g of a clear oil.
Yield: 93%.
$^1$H NMR ($CDCl_3$): δ 2.43 (s, 3H), 2.90-3.10 (m, 2H), 3.62 (br.s, 1H), 3.91 (s, 3H), 4.01 (br.s, 1H), 4.56 (br.s, 1H), 4.89 (br.s, 1H), 7.10-7.30 (m, 4H), 7.47 (s, 1H), 7.92 (d, J = 7.0 Hz, 2H).
IR ($CDCl_3$): 3025, 3010, 1723, 1627, 1441, 1317, 1294, 1238 $cm^{-1}$.
MS (FD) m/e 309 ($M^+$, 100).

| Analysis for $C_{19}H_{19}NO_3$: | | | |
|---|---|---|---|
| Calcd: | C, 73.77; | H, 6.19; | N, 4.53; |
| Found: | C, 73.55; | H, 6.19; | N, 4.55. |

C. N-[1'-Oxo-1'-(3"-carboxy-5"-methyl)phenyl]methyl-1,2,3,4-tetrahydroisoquinoline

The subtitled compound was prepared substantially in accordance with the procedure detailed in Example 1C, using 304 mg (12.7 mmol) of lithium hydroxide and 1.31 g (4.23 mmol) of the subtitled intermediate of Example 2B in 80 mL of a 3:1 tetrahydrofuran/water mixture to provide a residue. This residue was purified by recrystallization from ethyl acetate to provide 1.03 g of a white solid.
Yield: 90%.
$^1$H NMR ($CDCl_3$) : δ 2.37 (s, 3H), 2.81 (br.s, 2H), 3.52 (br.s, 1H), 3.81 (br.s, 1H), 4.55 (br.s, 1H), 4.74 (br.s, 1H), 6.95-7.30 (m, 4H), 7.48 (s, 1H), 7.81 (s, 1H).
IR (KBr): 3600-2200 (br), 1715, 1577, 1485, 1438, 1280, 1208, 933, 885, 837, 778, 765, 748, 680, 635 $cm^{-1}$.

MS (FD): m/e 295 ($M^+$), 591 (100).

| Analysis for $C_{18}H_{17}NO_3$: | | | |
|---|---|---|---|
| Calcd: | C, 73.20; | H, 5.80; | N, 4.74; |
| Found: | C, 73.21; | H, 5.92; | N, 4.77. |

D. [3'''S-(3'''R*,4''S*)]-N-[1'-Oxo-1'-(3''-[1'''-oxo-2'''-aza-3'''-phenylmethyl-4'''-hydroxy-5'''-(2'''-N-*t*-butylcarbamido)phenyl]pentyl-5''-methyl)phenyl]methyl-1,2,3,4-tetrahydoisoquinoline

The subtitled compound was prepared substantially in accordance with the procedure detailed in Example 1D, using 439 mg (1.69 mmol) of DCC, 229 mg (1.69 mmol) of HOBT·$H_2O$, 500 mg (1.69 mmol) of the subtitled intermediate of Example 2C and 349 mg (1.22 mmol) of the subtitled intermediate of Preparation 1D in 10 mL of anhydrous tetrahydrofuran containing 0.5 mL of dimethylformamide to provide a residue. This residue was purified using rotary chromatography (6 mm plate; gradient eluent of 30-50% ethyl acetate in hexane) to provide 740 mg of a white foam.

Yield: 71%.

$[a]_D$ -18.73° (c=1.004, MeOH).

$^1H$ NMR ($CDCl_3$): δ 1.42 (s, 9H), 2.35 (s, 3H), 2.78-3.05 (m, 5H), 3.54 (br.s, 1H), 3.90-4.06 (m, 1H), 4.48-4.55 (m, 2H), 4.84 (br.s, 1H), 5.94-5.99 (m, 2H), 6.86 (br.s, 1H), 7.12-7.55 (m, 15H).

IR ($CDCl_3$) : 3600-3100 (br.), 3028, 3008, 1642, 1600, 1515, 1455, 1369, 1233, 1050 $cm^{-1}$.

MS (FD): m/e 618 ($M^+$, 100).

| Analysis for $C_{39}H_{43}N_3O_4$: | | | |
|---|---|---|---|
| Calcd: | C, 75.82; | H, 7.02; | N, 6.80; |
| Found: | C, 76.04; | H, 7.01; | N, 6.92. |

Example 3

A. N-[1'-Oxo-1'-(3''-iodo-2''-methyl)phenyl]methyl-1,2,3,4-tetrahydroisoquinoline

The subtitled compound was prepared substantially in accordance with the procedure detailed in Example 1A, using 4.93 g (18.82 mmol) 3-iodo-2-methylbenzoic acid, 3.36 g (20.70 mmol) of 1,1-carbonyldiimidazole and 2.6 mL (20.2 mmol) of 1,2,3,4-tetrahydroisoquinoline in 80 mL of anhydrous tetrahydrofuran to provide a residue. This residue was purified using flash chromatography (gradient eluent of 14-50% hexane in ethyl acetate) to provide 6.87 g of a light yellow oil which solidified on standing.

Yield: 97%.

$^1H$ NMR ($CDCl_3$): δ 2.31, 2.40 (2s, 3H (rotamers)), 2.70-3.05 (m, 2H), 3.40-3.60 (m, 1H), 4.95-4.20 (m, 1H), 4.32-4.42 (m, 1H), 4.94 (dd, J=35.6; 17.3 Hz, 1H), 6.80-7.40 (m, 6H), 7.87 (d, J=7.8 Hz, 1H).

IR ($CDCl_3$) : 3011, 1626, 1586, 1431, 1338, 1299, 1259, 1200, 1157, 1102, 1048, 1003, 932, 910 $cm^{-1}$.

MS (FD): m/e 377 ($M^+$, 100).

| Analysis for $C_{17}H_{16}NOI$: | | | |
|---|---|---|---|
| Calcd: | C, 54.13; | H, 4.54; | N, 3.71; |
| Found: | C, 54.35; | H, 4.15; | N, 3.55. |

B. N-[1'-Oxo-1'-(3''-methoxycarbonyl-2''-methyl)phenyl]methyl-1,2,3,4-tetrahydroisoquinoline

The subtitled compound was prepared substantially in accordance with the procedure detailed in Example 1B, using 6.77 g (17.9 mmol) of the subtitled intermediate of Example 3A, 3.6 mL (17.9 mmol) of dicyclohexylamine and 490 mg (0.70 mmol) of bis(triphenylphosphine)palladium (II) chloride in 230 mL of anhydrous methanol, under an atmosphere of carbon monoxide to provide a residue. This residue was purified using flash chromatography (gradient eluent of 14-25% ethyl acetate in hexane) to provide 3.15 g of a light yellow oil.
Yield: 57%.
$^1$H NMR ($CDCl_3$): δ 2.44, 2.52 (2s, 3H (rotamers)), 2.75-2.85 (m, 1H), 2.97-3.01 (m, 1H), 3.41-3.44 (m, 1H), 3.90 (s, 3H), 3.92-4.13 (m, 1H), 4.25-4.40 (m, 1H), 4.96 (dd, J=40.1; 17.4 Hz, 1H), 6.85 (d, J=7.3 Hz, 0.5H), 7.10-7.36 (m, 4.5H), 7.88-7.91 (m, 1H).
IR ($CDCl_3$) : 1723, 1626.
MS (FD): m/e 309 ($M^+$, 100).

C. N-[1'-Oxo-1'-(3"-carboxy-2"-methyl)phenyl]methyl-1,2,3,4-tetrahydroisoquinoline

The subtitled compound was prepared substantially in accordance with the procedure detailed in Example 1C, using 464 mg (19.4 mmol) of lithium hydroxide and 2.99 g (9.68 mmol) of the subtitled intermediate of Example 3B in 200 mL of a 3:1 tetrahydrofuran/water mixture to provide a residue. This residue was purified using flash chromatography (eluent of 1:1 ethyl acetate/methylene chloride solution containing 1% acetic acid) to provide 2.79 g of a light brown foam.
Yield: 98%.
$^1$H NMR ($CDCl_3$): δ 2.50, 2.58 (2s, 3H (rotamers)), 2.76-2.81 (m, 1H), 3.01 (t, J=6.1 Hz, 1H), 3.43-3.47 (m, 1H), 3.99-4.08 (m, 1H), 4.30-4.01 (m, 1H), 4.98 (dd, J=38.7; 17.4 Hz, 1H), 6.87 (d, J=7.2 Hz, 0.5H), 7.11-7.43 (m, 4.5H), 8.04-8.07 (m, 1H).
IR ($CDCl_3$): 3600-2400 (br.), 1700, 1626, 1478, 1257, 1222, 1160, 1039 $cm^{-1}$.
MS (FD): m/e 295 ($M^+$, 100).

| Analysis for $C_{18}H_{17}NO_3$: | | | |
|---|---|---|---|
| Calcd: | C, 73.20; | H, 5.80; | N, 4.74; |
| Found: | C, 73.45; | H, 5.66; | N, 4.80. |

D. [3'''S-*(3'''R*,4'''S*)*]-N-[1'-Oxo-1'-(3"-[1'''-oxo-2'''-aza-3'''-phenylmethyl-4'''-hydroxy-5'''-(2''''-N-*t*-butylcarbamido)phenyl]methyl-2"-methyl)phenyl]methyl- 1,2,3,4-tetrahydoisoquinoline

The subtitled compound was prepared substantially in accordance with the procedure detailed in Example 1D, using 176 mg (0.85 mmol) of DCC, 250 mg (0.85 mmol) of the subtitled intermediate of Example 3C, 290 mg (1.01 mmol) of the subtitled intermediate of Preparation 1D and 115 mg (0.85 mmol) of HOBT·$H_2O$ in 10 mL of anhydrous tetrahydrofuran to provide a residue. This residue was purified using rotary chromatography (4 mm plate; gradient eluent of 30-60% ethyl acetate in methylene chloride) to provide 373 mg of a white foam.
Yield: 71%.
$[a]_D$ -13.34° (c=1.004, MeOH).
$^1$H NMR ($CDCl_3$): δ 1.46 (s, 9H), 1.91, 1.97, 2.11, 2.20 (4s, 3H (rotamers)), 2.72 (br.s, 1H), 2.86-3.20 (m, 6H), 3.39 (br.s, 1H), 3.80-4.01 (m, 2H), 4.28 (m, 1H), 4.59 (br.s, 1H), 4.78 (d, J=17.2 Hz, 1H), 4.99 (d, J=17.6 Hz, 1H), 6.04 (br.s, 1H), 6.33 (br.s, 1H), 6.86-7.41 (m, 16H).
IR ($CDCl_3$) : 3600-3100 (br.), 3009, 1642, 1514, 1455, 1369, 1300 $cm^{-1}$.
MS (FD): m/e 618 ($M^+$) , 220 (100).

| Analysis for $C_{39}H_{43}N_3O_4$: | | | |
|---|---|---|---|
| Calcd: | C, 75.82; | H, 7.02; | N, 6.80; |
| Found: | C, 75.53; | H, 7.03; | N, 6.71. |

Example 4

A. N-[1'-Oxo-1'-(3"-iodo-4"-ethyl)phenyl]methyl-1,2,3,4-tetrahydroisoquinoline

The subtitled compound was prepared substantially in accordance with the procedure detailed in Example

1A, using 4.74 g (17.17 mmol) of 4-ethyl-3-iodobenzoic acid, 2.80 g (17.17 mmol) of 1,1-carbonyldiimidazole and 2.2 mL (17.17 mmol) of 1,2,3,4-tetrahydroisoquinoline in 70 mL of anhydrous tetrahydrofuran, under nitrogen to provide a residue. This residue was purified using flash chromatography (eluent of 14% ethyl acetate in hexane) to provide 4.40 g of a light yellow oil, which solidified on standing.
Yield: 65%.
$^1$H NMR ($CDCl_3$): δ 1.22 (t, J=7.5 Hz, 3H), 2.76 (q, J=7.5 Hz, 2H), 2.90 (br.s, 2H), 3.59-4.05 (m, 2H), 4.50-4.95 (m, 2H), 6.90-7.38 (m, 6H), 7.90 (s, 1H).
IR ($CDCl_3$) : 3010, 2974, 1624, 1586, 1545, 1498, 1434, 1299, 1253, 1220, 1034, 840 $cm^{-1}$.
MS (FD): m/e 391 ($M^+$, 100).

| Analysis for $C_{18}H_{18}NOI$: | | | | |
|---|---|---|---|---|
| Calcd: | C, 55.26; | H, 4.64; | N, 3.58; | I, 32.44; |
| Found: | C, 54.96; | H, 4.71; | N, 3.67; | I, 32.48. |

B. N-[1'-Oxo-1'-(3"-methoxycarbonyl-4"-ethyl)phenyl]methyl-1,2,3,4-tetrahydroisoquinoline

The subtitled compound was prepared substantially in accordance with the procedure detailed in Example 1B, using 4.13 g (10.56 mmol) of the subtitled intermediate of Example 4A, 2.2 mL (11.09 mmol) of dicyclohexylamine and 140 mg (0.20 mmol) of bis(triphenylphosphine)palladium (II) chloride in 200 mL of anhydrous methanol and under an atmosphere of carbon monoxide to provide a residue. This residue was purified using flash chromatography (gradient eluent of 11-20% ethyl acetate in hexane) to provide 1.76 g of a light yellow oil.
Yield: 52%.
$^1$H NMR ($CDCl_3$): δ 1.25 (t, J=7.4 Hz, 3H), 2.80-3.10 (m, 4H), 3.60-4.05 (m, 5H), 4.50-4.95 (m, 2H), 7.19-7.25 (m, 4H), 7.34 (d, J=7.9 Hz, 1H), 7.52 (d, J=7.9 Hz, 1H), 7.97 (s, 1H).
IR ($CDCl_3$) : 3010, 1724, 1625, 1585, 1498, 1435, 1299, 1234, 1194, 1151, 1109, 1088, 1051 $cm^{-1}$.
MS (FD) : m/e 323 ($M^+$, 100).

| Analysis for $C_{20}H_{21}NO_3$: | | | |
|---|---|---|---|
| Calcd: | C, 74.28; | H, 6.25; | N, 4.34; |
| Found: | C, 74.02; | H, 6.50; | N, 4.59. |

C. N-[1'-Oxo-1'-(3"-carboxy-4"-ethyl)phenyl]methyl-1,2,3,4-tetrahydroisoquinoline

The subtitled compound was prepared substantially in accordance with the procedure detailed in Example 1C, using 357 mg (14.91 mmol) of lithium hydroxide and 1.60 g (4.97 mmol) of the subtitled intermediate of Example 4B in 40 mL of a 3:1 tetrahydrofuran/water, with the exception that the mixture was allowed to react overnight at 50°C. The resultant residue was purified using column chromatography (activated charcoal) to provide 1.35 g of a white foam.
Yield: 88%.
$^1$H NMR ($CDCl_3$) δ 1.27 (t, J=7.5 Hz, 3H), 2.96 (br.s, 2H), 3.10 (q, J=7.4 Hz, 2H), 3.66 (br.s, 1H), 3.99 (br.s, 1H), 4.62 (br.s, 1H), 4.89 (br.s, 1H), 7.13-7.25 (m, 4H), 7.38 (d, J=7.9 Hz, 1H), 7.58 (d, J=7.2 Hz, 1H), 8.12 (d, J=1.6 Hz, 1H).
IR (KBr) : 3600-2300 (br.), 1700, 1625, 1497, 1443, 1300, 1237, 1150, 1109, 1048, 933 $cm^{-1}$.
MS (FD): m/e 309 ($M^+$, 100).

D. [3'''S-*(3'''R*,4'''S*)*]-N-[1'-Oxo-1'-(3"-[1'''-oxo-2'''-aza-3'''-phenylmethyl-4'''-hydroxy-5'''-(2'''-N-*t*-butylcarbamido)phenyl]penthyl-4"-ethyl)phenyl]methyl-1,2,3,4-tetrahydoisoquinoline

The subtitled compound was prepared substantially in accordance with the procedure detailed in Example 1D, using 206 mg (1.0 mmol) of DCC, 300 mg (0.97 mmol) of the subtitled intermediate of Example 4C, 341 mg (1.18 mmol) of the subtitled intermediate of Preparation 1D and 135 mg (1.0 mmol) of HOBT·$H_2O$, in 6 mL of cold (-10°C) anhydrous tetrahydrofuran to provide a residue. This residue was purified using rotary chromatography (6 mm plate, gradient eluent of 1-5% methanol in methylene chloride) to provide 410 mg of an off-white foam.

Yield: 67%.

$^1$H NMR ($CDCl_3$): δ 1.07 (t, J=7.6 Hz, 3H), 1.45 (s, 9H), 2.54 (q, J=7.5 Hz, 2H), 2.70-3.30 (m, 5H), 3.55 (br.s, 1H), 3.91-3.95 (m, 1H), 4.40-4.65 (m, 2H), 4.83 (br.s, 1H), 5.97 (s, 1H), 6.32 (br.d, J=9.2 Hz, 1H), 6.90-7.60 (m, 16H).

IR ($CDCl_3$) : 3009, 1645, 1514, 1455, 1236,1213 $cm^{-1}$.

MS (FD): m/e 632 ($M^+$, 100).

Example 5

A. N-[1'-Oxo-1'-(3"-methoxycarbonyl-6"-(methyl)phenyl]methyl-1,2,3,4-tetrahydroisoquinoline

The subtitled compound was prepared substantially in accordance with the procedure detailed in Example 1A, using 100 mg (0.52 mmol) of 6-methyl-3-methyl ester-1-benzenedicarboxylic acid, 84 mg (0.52 mmol) of 1,1-carbonyldiimidazole and 0.07 mL (0.52 mmol) of 1,2,3,4-tetrahydroisoquinoline in 4 mL of tetrahydrofuran to provide a residue. This residue was purified using purified using rotary chromatography (2 mm plate; gradient eluent of 25-50% hexane in ethyl acetate) to provide 109 mg of a clear oil.

Yield: 71%.

$^1$H NMR ($CDCl_3$): δ 2.27, 2.38 (2s, 3H (rotamers)), 2.80-3.00 (m, 2H), 3.46 (br.s, 1.5H), 3.90 (s, 3H), 4.10-4.40 (m, 1.5H), 5.29 (br.s, 1H), 6.85 (d, J=7.3 Hz, 0.5H), 7.11-7.34 (m, 4.5H), 7.88-7.98 (m, 2H).

IR ($CHCl_3$): 3009, 1721, 1628, 1469, 1437, 1306, 1263, 1238, 1223, 1211, 1127 $cm^{-1}$.

MS (FD): m/e 309 ($M^+$, 100).

| Analysis for $C_{19}H_{19}NO_3$: | | | |
|---|---|---|---|
| Calcd: | C, 73.77; | H, 6.19; | N, 4.53; |
| Found: | C, 73.53; | H, 6.35; | N, 4.46. |

B. N-[1'-Oxo-1'-(3"-carboxy-6"-methyl)phenyl]methyl-1,2,3,4-tetrahydroisoquinoline

The subtitled compound was prepared substantially in accordance with the procedure detailed in Example 1C, using 18 mg (0.74 mmol) of lithium hydroxide and 109 mg (0.34 mmol) of the subtitled intermediate of Example 5A in 10 mL of a 3:1 tetrahydrofuran/water mixture, with the exception that after acidifying the reaction mixture, the compound was extracted using a 3:1 methylene chloride/isopropyl alcohol solution. The resultant residue was purified using rotary chromatography (2 mm plate; eluent of 1% acetic acid in a 1:1 ethyl acetate/methylene chloride solution) to provide 107 mg of a foam.

$^1$H NMR ($CDCl_3$) δ 2.28, 2.39 (2s, 3H (rotamers)), 2.80-3.00 (m, 2H), 3.47 (br.s, 1H), 3.81 (br.s, 0.5H), 4.20-4.44 (m, 1.5H), 4.80-5.06 (br.s, 1H), 6.85 (d, J=7.3 Hz, 0.5H), 7.11-7.36 (m, 4.5H), 7.94-8.02 (m, 2H).

IR ($CHCl_3$): 3600-2400 (br.), 1698, 1628, 1498, 1447, 1300, 1240, 1159, 1129, 1048, 910 $cm^{-1}$.

MS (FD): m/e 295 ($M^+$, 100).

C. [3'"S-*(3'"R*,4'"S*)*]-N-[1'-Oxo-1'-(3"-[1'"-oxo-2'"-aza-3'"-phenylmethyl-4'"-hydroxy-5'"-(2'"-N-*t*- butylcarbamido)phenyl]pentyl-6"-methyl)phenyl]methyl-1,2,3,4-tetrahydoisoquinoline

A solution containing 90 mg (0.30 mmol) of the subtitled intermediate of Example 5B and 52 mg (0.32 mmol) of 1,1-carbonyldiimidazole in 3 mL of tetrahydrofuran was stirred for 60 minutes, under nitrogen. To this solution was added 109 mg (0.38 mmol) of the subtitled intermediate of Preparation 1D resulting in a clear yellow solution. After reacting overnight, this solution was reduced to dryness under reduced pressure to provide a residue. This residue was redissolved in 100 mL of ethyl acetate and filtered through celite. The filtrate was extracted sequentially with saturated saturated sodium bicarbonate and brine solutions, dried over sodium sulfate filtered and reduced to dryness under reduced pressure to provide a material. This material was purified using rotary chromatography (2 mm plate; gradient eluent of 50-100% ethyl acetate in hexane) to provide 100 mg of a white foam.

Yield: 54%.
[α]$_D$ -21.13° (c=1.003, MeOH).
$^1$H NMR ($CDCl_3$): δ 1.43 (s, 9H), 2.20, 2.31 (2s, 3H (rotamers)), 2.66-3.05 (m, 6H), 3.39 (br.s, 1H), 4.05-4.40 (m, 2H), 3.90 (br.s, 1H), 4.52 (br.s, 1H), 4.88 (br.s, 1H), 6.14 (br.s, 1H), 6.80-7.65 (m, 17H).
IR ($CHCl_3$): 3600-3100 (br.), 3431, 3027, 3010, 1641, 1516, 1455, 1368, 1234, 1048 $cm^{-1}$.
MS (FD): m/e 618 ($M^+$, 100).

| Analysis for $C_{39}H_{43}N_3O_4$: | | | |
|---|---|---|---|
| Calcd: | C, 75.82; | H, 7.02; | N, 6.80; |
| Found: | C, 75.58; | H, 7.27; | N, 6.71. |

## Example 6

[3S-*(3R*,4aR*,8aR*,2'S*,3'R*)*]-2-[2'-Hydroxy-3'-phenylmethyl-4'-aza-5'-oxo-5'-(3"-[1'",2'",3'",4'"-tetrahydroisoquinolin-1'"-ylcarbonyl]-6"-methyl)phenyl]pentyl-decahydroisoquinoline-3-N-*t*-butylcarboxamide

The titled compound was prepared substantially in accordance with the procedure detailed in Example 1D using 256 mg (1.24 mmol) of DCC, 168 mg (1.24 mmol) of HOBT·$H_2O$, 366 mg (1.24 mmol) of the subtitled intermediate of Example 1C and 500 mg (1.24 mmol) of the subtitled intermediate of Preparation 2B in 15 mL of cold (-10°C) anhydrous tetrahydrofuran to provide a residue. This residue was purified using rotary chromatography (6 mm plate; eluent of 3% methanol in methylene chloride) to provide 489 mg of an off-white foam.
Yield: 58%.
[a]$_D$ -69.1017° (c=1.013, MeOH).
$^1$H NMR ($CDCl_3$): δ 1.13 (s, 9H), 1.20-2.10 (m, 14H), 2.20-2.35 (m, 4H), 2.50-3.05 (m, 7H), 3.37-3.60 (m, 2H), 3.80-4.10 (m, 2H), 4.40-4.70 (m, 2H), 4.85 (br.s, 1H), 5.67 (br.s, 1H), 6.72 (br.d, J = 8.4 Hz, 1H), 6.95-7.34 (m, 12H, aromatic).
IR (KBr): 3600-3150 (br.), 3009, 1627, 1514, 1455, 1247, 1047 $cm^{-1}$.
MS (FD): m/e 679 ($M^+$), 578 (100).

| Analysis for $C_{42}H_{54}N_4O_4$: | | | |
|---|---|---|---|
| Calcd: | C, 74.30; | H, 8.02; | N, 8.25; |
| Found: | C, 74.07; | H, 8.00; | N, 8.22. |

## Example 7

A. 2-[(2'-N-(Methyl)aza-3'-oxo-3'-[3"-methoxycarbonyl-4"-methyl]phenyl)propyl]quinoline

A solution of 1.91 g (9.86 mmol) of 4-methyl-3-methoxycarbonyl benzoic acid, 2.6 mL (34.8 mmol) of thionyl chloride and 1 drop of dimethylformamide in 50 mL of benzene was heated at reflux for 2 hours, under nitrogen. The reaction mixture was then reduced to dryness under reduced pressure to provide a residue which was redissolved in 200 mL of ethyl acetate and then slowly added to a cold (-10°C) solution of 1.86 g (11.6 mmol) of 2-(2-azopropyl)quinoline in 30 mL of 1:2 pyridine/methylene chloride. The resulting mixture was gradually warmed to room temperature and then concentrated under reduced pressure to provide a residue. This residue was redissolved in 100 mL of ethyl acetate, washed sequentially with water, saturated sodium bicarbonate and brine, dried over sodium sulfate and then filtered. The desired subtitled compound was purified using flash chromatography (gradient eluent of 1-5% methanol in methylene chloride) to provide 1.29 g of a light yellow oil.
Yield: 38%.
$^1$H NMR ($CDCl_3$): δ 2.57, 2.63 (2br.s, 3H (rotamers)), 3.03, 3.15 (2br.s, 3H (rotamers)), 3.67, 3.90 (2br.s, 3H (rotamers)), 4.77, 5.05 (2br.s, 2H, (rotamers)), 7.10-8.25 (m, 9H).
IR ($CHCl_3$) : 2977, 1724, 1631, 1239, 1047 $cm^{-1}$.
MS (FD): m/e 347 ($M^+$, 100).

| Analysis for $C_{21}H_{20}N_2O_3$: | | | |
|---|---|---|---|
| Calcd: | C, 72.40; | H, 5.79; | N, 8.04; |
| Found: | C, 72.23; | H, 5.93; | N, 8.07. |

B. 2-[2'-N-(Methyl)aza-3'-oxo-3'-[3"-carboxy-4"-methyl]phenyl)propyl]quinoline

The desired subtitled compound was prepared substantially in accordance with the procedure detailed in Example 1C, using 76 mg (3.18 mmol) of lithium hydroxide and 367 mg (1.06 mmol) of the subtitled intermediate of Example 7A in 20 mL of a 2:1 tetrahydrofuran/water mixture to provide 330 mg of an off-white solid.
Yield: 94%.
$^1H$ NMR (DMSO-$d_6$): δ 2.40-2.60 (m, 3H), 2.96 (s, 3H), 4.69, 4.90 (2br.s, 2H (rotamers)), 7.20-8.00 (m, 8H), 8.35 (br.s, 1H).
IR ($CHCl_3$): 3600-2400 (br.), 1700, 1631, 1507, 1403, 1248, 1204, 1085, 830 $cm^{-1}$.
MS (FD): m/e 335 ($M^+$, 100).

C. [3S-*(3R*,4aR*,8aR*,2'S*,3'R*)*]-2-[2'-Hydroxy-3'-phenylmethyl-4'-aza-5'-oxo-5'-(3"-[1'"-oxo-2'"-N-(methyl)aza-3'"-quinolin-2""-ylpropyl]-6"- methyl)phenyl]pentyl-decahydroisoquinoline-3-N-*t*-butylcarboxamide

The subtitled compound was prepared substantially in accordance with the procedure detailed in Example 1D, using 126 mg (0.61 mmol) of DCC, 83 mg (0.61 mmol) of HOBT·$H_2O$, 202 mg (0.61 mmol) of the subtitled intermediate of Example 7B and 245 mg (0.61 mmol) of the subtitled intermediate of Preparation 2B in 5.5 mL of anhydrous tetrahydrofuran containing 0.5 mL of dimethylformamide to provide a residue. This residue was purified using rotary chromatography (2 mm plate; eluent of 5% methanol in methylene chloride) to provide 272 mg of an off-white foam.
Yield: 62%.
$[\alpha]_D$ -59.0476° (c=1.05, MeOH).
$^1H$ NMR ($CDCl_3$): δ 1.16 (s, 9H), 1.15-3.20 (m, 24H), 3.90-4.10 (m, 2H), 4.56 (br.s, 1H), 4.72, 5.03 (2br.s, 2H, (rotamers)), 6.00 (br.s, 1H), 6.55-6.80 (m, 1H), 7.00-8.25 (m, 14H).
IR ($CHCl_3$): 3650-3150 (br.), 2929, 1632 (br.), 1509, 1455, 1245, 1047 $cm^{-1}$.
MS (FD): m/e 719 ($M^+$), 617 (100).

Example 8

A. N-[1'-Oxo-1'-(3"-carboxy)phenylmethyl]-1,2,3,4-tetrahydroisoquinoline

To a cold (-5°C) solution of 22.3 mL (315 mmol) of thionyl chloride in 35 mL of benzene containing 350 μL of dimethylformamide, was added 25.0 g (150 mmol) isophthalic acid was added in one portion. The reaction mixture was refluxed for approximately 16 hours, cooled to room temperature and then reduced to dryness under reduced pressure to provide a residue. This residue was azeotroped once with benzene and then diluted with 200 mL of diethylether. The resultant solution was cooled to -10°C, followed by the dropwise addition of 18.8 mL (150 mmol) of 1,2,3,4-tetrahydroisoquinoline. The reaction was then quenched with 100 mL water. The organic and aqueous layers were separated and the organic layer was dried over sodium sulfate, filtered and then concentrated under reduced pressure to provide a residue. This residue was purified using flash chromatography to provide 477 mg of a colorless foam.
Yield: 1%.
$^1H$ NMR (DMSO-$d_6$): δ 2.69-2.87 (br.s, 2H), 3.83-4.22 (br.s, 4H), 4.73-4.80 (br.s, 1H), 7.02-7.27 (m, 4H), 7.41-7.47 (m, 2H), 7.94-8.06 (m, 2H).
MS (FAB): 282 ($M^+$), 119 (100).

B. N-[1'-Oxo-1'-3"-pentafluorophenoxycarbonyl)phenylmethyl]-1,2,3,4-tetrahydroisoquinoline

To a cold (0°C) soluton of 210 mg (0.75 mmol) of the subtitled intermediate from Example 8A and 207 mg (1.13 mmol) pentafluorophenol in 5 mL of anhydrous tetrahydrofuran, was added 173 mg (0.90 mmol) of 1-ethyl-3-[3-dimethylamino)propyl]-carbodiimide hydrochloride (EDC). The resulting reaction mixture was allowed to warm to room temperature and react for about 4 hours, at which time it was diluted with 10 mL of a

3:1 chloroform/isopropanol solution and washed sequentially with saturated sodium bicarbonate and brine solutions. The resulting layers were separated and the organic layer was dried over sodium sulfate, filtered and then concentrated under reduced pressure to provide a residue. This residue was purified using rotary chromatography to provide 74 mg of a colorless foam.

Yield: 22%.

$^1H$ NMR ($CDCl_3$): δ 2.83-3.08 (m, 2H), 3.62-3.73 (br.s, 1H), 3.92-4.13 (br.s 1H), 4.58-4.63 (br.s, 1H), 4.82-4.98 (br.s, 1H), 7.07-7.33 (m, 4H), 7.59-7.68 (m, 1H), 7.76-7.81 (m, 1H), 8.26-8.34 (m, 2H).

MS (FD): 447 ($M^+$), 447 (100).

C. [3'''S-*(3'''R*,4'''S*)*]-N-[1'-Oxo-1'-(3"-[1'''-oxo-2'''-aza-3'''-phenylmethyl-4'''-hydroxy-5'''-(2''''-N-*t*-butylcarbamido)phenyl]pentyl)phenyl]methyl-1,2,3,4-tetrahydroisoquinoline

A solution of 72 mg (0.016 mmol) of the subtitled intermediate from Example 8B in 3 mL of anhydrous methylene chloride was added via cannula to a solution of 52 mg (0.15 mmol) of the subtitled intermediate from Preparation 1D in 5 mL anhydrous methylene chloride. To this solution was added 19 μL (0.18 mmol) of 4-methylmorpholine via syringe and the resulting reaction mixture was allowed to react for approximately 16 hours. The reaction mixture was then washed sequentially with saturated sodium bicarbonate, 2N sodiumhydrogen-sulfate and brine solutions. The organic layer was dried over sodium sulfate, filtered and then concentrated under reduced pressure to provide a residue. This residue was purified using rotary chromatography to provide 27 mg of a colorless foam.

Yield: 30%.

$^1H$ NMR ($CDCl_3$) : δ 1.44 (s, 9H), 2.73-3.11 (m, 6H), 3.50-3.61 (br.s, 1H), 3.88-4.00 (m, 2H), 4.46-4.62 (m, 2H) 4.83-4.90 (br.s, 1H), 5.87 (d, J=6Hz, 1H), 6.16 (s, 1H), 7.13-7.56 (m, 16H), 7.71-7.80 (m, 2H).

MS (FD): 604 ($M^+$), 604 (100).

Example 9

A. N-(*t*-Butoxycarbonyl)methylamine

To a cold (0°C) solution of 22 g (0.33 mmol) of methylamine hydrochloride in 550 mL of anhydrous methylene chloride, was added 48 mL (0.34 mol) of triethylamine followed by 75 g (0.34 mol) of t-butoxycarbonylanhydride. The resultant reaction mixture was reacted for approximately one hour at 0°C, warmed to room temperature and allowed to react overnight. The reaction mixture was then diluted with water. The resultant layers were separated and the organic layer was washed with water, dried over sodium sulfate, filtered and concentrated to provide 47.4 g of a clear oil.

Yield:

B. 2-((N-(Methyl)-N-(*t*-butoxycarbonyl)amino)methyl)pyridine

To a cold (0°C) solution of 3.0 g (18.30 mmol) of 2-(chloromethyl)pyridine and 2.0 g (15.25 mmol) of the subtitled compound of Example 9A in 40 mL of anhydrous dimethylformamide, was added 1.12 g (27.9 mmol) of sodium hydride. After the resultant reaction mixture was warmed to room temperature, 5.5 g (36.6 mmol) of sodium iodide was slowly added. The reaction mixture was allowed to react overnight and then was poured into a saturated solution of sodium bicarbonate and the resulting emulsion was diluted with water. The resulting layers were separated and the aqueous layer was extracted twice with methylene chloride. The combined organic extracts were dried over sodium sulfate, filtered and concentrated to provide crude material. The crude material was purified using flash chromatography (gradient eluent of 20-50% ethyl acetate in hexane) to provide 69 mg of a yellow oil.

Yield: 20%.

$^1H$ NMR ($CDCl_3$): δ 1.20-1.75 (m, 9H), 2.75-3.10 (m, 3H), 4.40-4.62 (m, 2H), 7.13-7.31 (m, 2H), 7.60-7.73 (m, 1H), 8.53 (d, J=4.38 Hz, 1H).

IR ($CHCl_3$): 3010, 2980, 2935, 1687, 1594, 1573, 1479, 1455, 1421, 1394, 1369, 1305, 1249, 1172, 1152, 1050, 879 $cm^{-1}$.

MS (FD): m/e 223 ($M^+$, 100).

C. 2-((N-(Methyl)amino)methyl)pyridine hydrochloride

A solution of 300 mg (1.35 mmol) and 5 mL of 5N aqueous hydrochloric acid in 5 mL of dioxane was reacted overnight. The resultant reaction mixture was concentrated under reduced pressure to provide a residue. This residue was azeotroped with toluene and then reduced to dryness under reduced pressure to provide an amorphous yellow solid. This solid was used without further purification.
$^1H$ NMR (DMSO-$d_6$) : δ 2.50-2.63 (m, 3H), 4.25-4.45 (m, 2H), 7.61-7.65 (m, 1H), 7.86 (d, J=7.84 Hz, 1H), 8.12-8.14 (m, 1H), 8.71 (d, J=4.90 Hz, 1H).

D. 2-[(2'-N-(Methyl)aza-3'-oxo-3'-[3"-methoxycarbonyl-4"- methyl]phenyl)propyl]pyridine

A solution containing 260 mg (1.33 mmol) of the subtitled compound of Example 9C, 238 mg (0.66 mmol) of the titled compound of Preparation 7, and 0.5 mL of triethylamine in 5 mL of anhydrous methylene chloride was reacted overnight. The resultant reaction mixture was diluted with methylene chloride and then extracted three times with 1M potassium carbonate. The combined organic extracts were dried over sodium sulfate, filtered and then concentrated under reduced pressure to provide crude material. This material was purified using radial chromatography (4mm; gradient eluent of 0-5% methanol in methylene chloride) to provide 154 mg of a yellow oil.
Yield: 40%.
$^1H$ NMR ($CDCl_3$): δ 2.50-2.80 (m, 3H), 2.90-3.20 (m, 3H), 3.70-4.00 (m, 3H), 4.50-4.95 (m, 2H), 7.05-7.77 (m, 6H), 8.05 (s, 1H), 8.57 (d, J=3.91 Hz, 1H).
IR ($CHCl_3$) : 3027, 3008, 2954, 1723, 1631, 1594, 1573, 1477, 1437, 1404, 1303, 1249, 1088, 1077 $cm^{-1}$.
MS (FD): m/e 298 ($M^+$, 100).

| Analysis for $C_{17}H_{18}N_2O_3$: | | | |
|---|---|---|---|
| Calcd: | C, 68.44; | H, 6.08; | N, 9.39; |
| Found: | C, 68.64; | H, 6.21; | N, 9.27. |

E. 2-[(2'-N-(Methyl)aza-3'-oxo-3'-[3"-carboxy-4"-methyl]phenyl)propyl]pyridine hydrochloride

The desired subtitled compound was prepared substantially in accordance with the procedure detailed in Example 1C, using 99 mg (0.33 mmol) of the subtitled compound of Example 9D and 16 mg (0.66 mmol) of lithium hydroxide in 8 mL of a 3:1 mixture of tetrahydrofuran and water to provide 94 mg of a solid which was used without further purification.
$^1H$ NMR (DMSO-$d_6$): δ 2.40-2.60 (m, 3H), 2.85-3.10 (m, 3H), 4.60-5.05 (m, 2H), 7.20-8.85 (m, 7H).
IR ($CHCl_3$) : 3700-1800 (br.), 1713, 1635, 1537, 1518, 1484, 1470, 1404, 1356, 1306, 1236, 1085, 997, 981 $cm^{-1}$.
MS (FD): m/e 285 ($M^+$, 100).

F. [2'R-*(2'R*,3'S*)*]-2-(2'-Hydroxy-3'-phenylmethyl-4'-aza-5'-oxo-5'-(3"-[1'"-oxo-2'"-N-(methyl)aza-3'"-pyrid-2""-ylpropyl]-6"-methyl)-phenyl]pentyl -N-*t*-butylbenzamide

The subtitled compound was prepared substantially in accordance with the procedure detailed in Example 1D, using 95 mg (0.28 mmol) of the subtitled compound of Preparation 1D, 93 mg (0.29 mmol) of the subtitled compound of Example 9E, 38 mg (0.28 mmol) of HOBT·$H_2O$ and 58 mg (0.28 mmol) of DCC in 5 mL of tetrahydrofuran. The resultant crude matrial was purified using radial chromatography (2mm; gradient eluent of 2-4% methanol in methylene chloride) to provide 50 mg of a white solid.
Yield: 28%
$^1H$ NMR ($CDCl_3$) : δ 1.46 (s, 9H), 2.10-2.22 (m, 3H), 2.80-3.22 (m, 7H), 3.80-3.98 (m, 1H), 4.48-4.65 (m, 2H), 4.70-4.95 (m, 1H), 5.95-6.40 (m, 3H), 7.05-7.78 (m, 15H), 8.50-8.62 (m, 1H).
IR ($CHCl_3$) : 3600-3100 (br.), 2974, 2931, 1643, 1573, 1514, 1485, 1455, 1437, 1395, 1368, 1311, 1233, 1077, 1049, 910, 882 $cm^{-1}$.
MS (FD): m/e 607 ($M^+$, 100).

Example 10

[3S-*(3R*,4aR*,8aR*,2'S*,3'R*)*]-2-(2'-Hydroxy-3'-phenylmethyl-4'-aza-5'-oxo-5'-(3"-[1'"-oxo-2'"-N-(methyl)aza-3'"-pyrid-2""-ylpropyl]-6"-methyl)phenyl]pentyl decahydroisoquinoline-3-N-*t*-butylcarboxamide

The subtitled compound was prepared substantially in accordance with the procedure detailed in Example 2D, using 124 mg (0.31 mmol) of the subtitled compound of Preparation 2B, 44 mg (0.33 mmol) of the subtitled compound of Example 9E, 42 mg (0.31 mmol) of HOBT·$H_2O$ and 64 mg (0.31 mmol) of DCC in 5 mL of anhydrous tetrahydrofuran and 0.5 mL of anhydrous dimethylformamide. The resultant crude material was purified using radial chromatography (2mm; gradient eluent of 0-5% methanol in methylene chloride) to provide 111 mg of an off-white foam.
Yield: 50%.
$^1H$ NMR ($CDCl_3$): δ 1.13 (s, 9H), 1.20-2.35 (m, 19H), 2.50-2.70 (m, 2H), 2.90-3.10 (m, 4H), 3.25-3.60 (m, 1H), 3.92-4.10 (m, 2H), 4.50-4.66 (m, 2H), 4.80-4.92 (m, 1H), 5.60-5.76 (m, 1H), 6.50-6.78 (m, 1H), 7.06-7.44 (m, 9H), 7.60-7.80 (m, 1H), 8.54-8.61 (m, 1H).
IR ($CHCl_3$): 3600-3100 (br.), 3000, 2929, 2865, 1669, 1632, 1572, 1512, 1455, 1437, 1394, 1368, 1305, 1277, 1148, 1077, 983, 909 $cm^{-1}$.
MS (FD): m/e 667 ($M^+$, 100).

| Analysis for $C_{40}H_{53}N_5O_4$: | | | |
|---|---|---|---|
| Calcd: | C, 71.93; | H, 8.00; | N, 10.49; |
| Found: | C, 72.17; | H, 7.71; | N, 10.40. |

Example 11

A. 3-((N-(Methyl)-N-(*t*-butoxycarbonyl)amino)methyl)pyridine

The subtitled compound was prepared substantially in accordance with the procedure detailed in Example 9B, using 6.0 g (45.7 mmol) of the subtitled compound of Example 9A, 9.02 g (55.0 mmol) of 3-(chloromethyl)pyridine, 10.3 g (68.5 mmol) of sodium iodide, 4.1 g (103 mmol) of 60% sodium hydride in 100 mL of dimethylformamide. The crude material was purified using radial chromatography (6mm; 20-100% ethyl acetate in methylene chloride) to provide 760 mg of an orange oil.
$^1H$ NMR ($CDCl_3$): δ 1.48 (s, 9H), 2.85 (br.s, 3H), 4.44 (s, 2H), 7.25-7.32 (m, 1H), 7.50-7.65 (m, 1H), 8.50-8.60 (m, 2H).
MS (FD) : m/e 285 ($M^+$, 100).

B. 3-((N-(Methyl)amino)methyl)pyridine

The subtitled compound was deprotected substantially in accordance with the procedure detailed in Example 9C.

C. 3-[(2'-N-(Methyl)aza-3'-oxo-3'-[3"-methoxycarbonyl-4"-methyl]phenyl)propyl]pyridine

The subtitled compound was prepared substantially in accordance with the procedure detailed in Example 9D using 488 mg (4.0 mmol) of the subtitled compound of Example 11B, 700 mg (2.0 mmol) of the titled compound of Preparation 7 in 8 mL of methylene chloride, with the exception that triethylamine was not added to the reaction mixture. The crude material was purified using radial chromatography (4mm; 2-4% methanol in methylene chloride) to provide 495 mg of a clear oil.
Yield: 83%.
$^1H$ NMR ($CDCl_3$): δ 2.64 (s, 3H), 2.90-3.15 (s, 3H), 3.90 (s, 3H), 4.50-4.90 (m, 2H), 7.25-7.40 (m, 2.5H), 7.40-7.55 (m, 1H), 7.70-7.85 (m, 0.5H), 8.03 (s, 1H), 8.50-9.00 (m, 1H).
IR ($CHCl_3$): 3008, 1724, 1632, 1580, 1503, 1481, 1437, 1425, 1405, 1306, 1260, 1224, 1192, 1163, 1081, 1076, 1030, 836 $cm^{-1}$.
MS (FD): m/e 298 ($M^+$, 100).

| Analysis for $C_{17}H_{18}N_2O_3$: | | | |
|---|---|---|---|
| Calcd: | C, 68.44; | H, 6.08; | N, 9.39; |
| Found: | C, 68.16; | H, 6.35; | N, 9.42. |

D. 3-[(2'-N-(Methyl)aza-3'-oxo-3'-[3"-carboxy-4"-methyl]phenyl)propyl]pyridine hydrochloride

The desired subtitled compound was prepared substantially in accordance with the procedure detailed in Example 1C, using 189 mg (0.63 mmol) of the subtitled compound of Example 11C and 46 mg (1.9 mmol) of lithium hydroxide in 10 mL of a 3:1 mixture of tetrahydrofuran and water, with the exception that the reaction mixture was acidified to pH 2 using 5N hydrochloric acid followed by the addition of lithium chloride. The resultant mixture was washed several times with isopropanol, dried over sodium sulfate, filtered and reduced to dryness under reduced pressure to provide 150 mg of an off-white foam.

$^1$H NMR ($CDCl_3$) : δ 2.68 (s, 3H), 3.03 (s, 3H), 4.60-5.00 (m, 2H), 7.10-7.70 (m, 5H), 7.90-8.10 (m, 1H), 8.50-9.00 (m, 1H).

MS (FD): m/e 285 ($M^+$, 100).

E. [3S-*(3R*,4aR*,8aR*,2'S,3'R*)*]-2-(2'-Hydroxy-3'-phenylmethyl-4'-aza-5'-oxo-5'-(3"-[1'"-oxo-2'"-N-(methyl)aza-3'"-pyrid-3""-ylpropyl]-6"-methyl)phenyl]pentyl decahydroisoquinoline-3-N-*t*-butylcarboxamide

The subtitled compound was prepared substantially in accordance with the procedure detailed in Example 1D, using 177 mg (0.44 mmol) of the subtitled compound of Preparation 2B, 143 mg (0.45 mmol) of the subtitled compound of Example 11D, 59 mg (0.44 mmol) of HOBT·$H_2O$ and 91 mg (0.44 mmol) of DCC in 5 mL of tetrahydrofuran. The resultant crude matrial was purified using radial chromatography (2mm; gradient eluent of 2-5% methanol in methylene chloride) to provide 119 mg of a white foam.

Yield: 40%.

$^1$H NMR ($CDCl_3$): δ 1.14 (s, 9H), 1.20-2.35 (m, 18H), 2.48-3.10 (m, 6H), 3.35-3.55 (m, 1H), 3.95-4.18 (m, 2H), 4.55 (m, 3H), 5.72 (br.s, 1H), 6.70-6.85 (m, 1H), 7.04-7.38 (m, 10.5H), 7.65-7.82 (m, 0.5H), 8.55-8.65 (m, 1H).

IR ($CHCl_3$): 3691, 3600-3100 (br.), 3429, 3008, 2929, 1670, 1634, 1512, 1481, 1455, 1394, 1368, 1246, 1075, 1046, 910 $cm^{-1}$.

MS (FD): m/e 668 ($M^+$), 567 (100).

| Analysis for $C_{40}H_{53}N_5O_4$: | | | |
|---|---|---|---|
| Calcd: | C, 71.93; | H, 8.00; | N, 10.49. |
| Found: | C, 71.63; | H, 7.85; | N, 10.34. |

Example 12

A. 4-[(N-(*t*-butoxycarbonyl)amino)methyl]pyridine

To a solution of 10.0 g (92 mmol) of 4-aminomethylpyridine in 550 mL of methylene chloride, was added 21 g (97 mmol) of *t*-butoxycarbonylanhydride. The resultant reaction mixture was reacted for approximately thirty minutes. When the reaction mixture was substantially complete, as indicated by TLC, the reaction mixture was washed sequentially with saturated sodium bicarbonate, and brine, dried over sodium sulfate, filtered and then concentrated to provide a crude material. This material was purified using flash chromatography (gradient eluent of 50-100% ethyl acetate in methylene chloride) to provide 15.23 g of a light yellow solid.

Yield: 80%

$^1$H NMR ($CDCl_3$) : δ 1.46 (s, 9H), 4.33 (d, J=5.91 Hz, 2H), 4.98 (br.s, 1H), 7.18-7.26 (m, 2H), 8.54-8.56 (m, 2H).

IR ($CHCl_3$): 3458, 2982, 1715, 1605, 1565, 1507, 1455, 1418, 1396, 1369, 1348, 1275, 1248, 1166, 1069, 1050, 996, 862 $cm^{-1}$.

MS (FD): m/e 209 ($M^+$, 100).

| Analysis for $C_{11}H_{16}N_2O_2$: | | | |
|---|---|---|---|
| Calcd: | C, 63.44; | H, 7.74; | N, 13.45; |
| Found: | C, 63.44; | H, 7.71; | N, 13.49. |

B. 4-((N-(Methyl)-N-(*t*-butoxycarbonyl)amino)methyl)pyridine

To a cold (0°C) solution of 8.0 g (38 mmol) of the subtitled compound of Example 12A in 50 mL of anhydrous dimethylformamide, was added 1.00 g (41.8 mmol) of sodium hydride. The resultant reaction mixture was stirred for approximately thirty minutes and then 5.72 g (40.3 mmol) of methyl iodide was slowly added. The reaction mixture was poured into a methylene chloride/water mixture and then refrigerated. The resulting layers were separated and the organic layer was washed four times with 1N sodium hydroxide, dried over sodium sulfate, filtered and then reduced to dryness under reduced pressure (with heat) to provide 7.54 g of a dark orange oil.
Yield: 88%.
$^1H$ NMR ($CDCl_3$): δ 1.30-1.70 (m, 9H), 2.75-3.00 (m, 3H), 4.42 (br.s, 2H), 7.12 (d, J=4.73 Hz, 2H), 8.55 (d, J=5.54 Hz, 2H).

C. 4-((N-(Methyl)amino)methyl)pyridine dihydrochloride

The subtitled compound was prepared substantially in accordance with the procedure detailed in Example 9C, using 1.15 g (5.17 mmol) of the subtitled compound of Example 12B and 15 mL of 5N hydrochloric acid in 15 mL of dioxane to provide a crude material. This material was used without further purification.
$^1H$ NMR (DMSO-$d_6$) : δ 2.58 (s, 3H), 4.40 (s, 2H), 8.15-8.25 (m, 1H), 8.90-9.90 (m, 1H), 10.22 (br.s, 2H).
IR (KBr): 3800-1800 (br.), 1638, 1599, 1503, 1428, 1233, 1193, 1145, 1067, 1002, 887, 809, 766, 593, 478 $cm^{-1}$.

D. 4-[(2'-N-(Methyl)aza-3'-oxo-3'-[3"-methoxycarbonyl-4"-methyl]phenyl)propyl]pyridine

The subtitled compound was prepared substantially in accordance with the procedure detailed in Example 9D, using the subtitled compound of Example 12C (5.17 mmol), 1.12 g (3.23 mmol) of the titled compound of Preparation 7, and 2 mL of triethylamine in 20 mL of anhydrous methylene chloride. The resultant material was purified using radial chromatography (6mm; gradient eluent of 2-4% methanol in methylene chloride) to provide 617 mg of the desired compound.
Yield: 64%.
$^1H$ NMR ($CDCl_3$): δ 2.62 (s, 3H), 2.80-3.20 (m, 3H), 3.70-4.00 (m, 3H), 4.40-4.85 (m, 2H), 7.00-7.60 (m, 4H), 8.03 (s, 1H), 8.55-8.70 (m, 2H).
IR ($CHCl_3$) : 3009, 2955, 1724, 1634, 1604, 1565, 1503, 1483, 1449, 1437, 1404, 1363, 1305, 1261, 1089, 1077, 1069 $cm^{-1}$.
MS (FD): m/e 298 ($M^+$, 100).

| Analysis for $C_{17}H_{18}N_2O_3$: | | | |
|---|---|---|---|
| Calcd: | C, 68.44; | H, 6.08; | N, 9.39; |
| Found: | C, 68.65; | H, 6.20; | N, 9.41. |

E. 4-[(2'-N-(Methyl)aza-3'-oxo-3'-[3"-carboxy-4"-methyl]phenyl)propyl]pyridine hydrochloride

The desired subtitled compound was prepared substantially in accordance with the procedure detailed in Example 11D, using 124 mg (0.42 mmol) of the subtitled compound of Example 12D and 30 mg (1.25 mmol) of lithium hydroxide in 10 mL of a 3:1 mixture of tetrahydrofuran and water to provide 120 mg of a white foam which was used without further purification.
$^1H$ NMR (DMSO-$d_6$): δ 2.54 (s, 3H), 2.98 (s, 3H), 4.65-4.95 (m, 2H), 7.20-8.00 (m, 5H), 8.76-8.90 (m, 2H).
IR ($CHCl_3$) : 3400-3200 (br.), 1711, 1636, 1506, 1484, 1452, 1401, 1203, 1087, 1005, 925, 792, 744

$cm^{-1}$.
MS (FD): m/e 285 ($M^+$, 100).

F. [3S-*(3R*,4aR*,8aR*,2'S*,3'R*)*]-2-(2'-Hydroxy-3'-phenylmethyl-4'-aza-5'-oxo-5'-(3"-[1"'-oxo-2"'-N-(methyl)aza-3"'-pyrid-4""-ylpropyl]-6"-methyl)phenyl]pentyl decahydroisoquinoline-3-N-*t*-butylcarboxamide

The subtitled compound was prepared substantially in accordance with the procedure detailed in Example 1D, using 109 mg (0.272 mmol) of the subtitled compound of Preparation 2B, 92 mg (0.287 mmol) of the subtitled compound of Example 12E, 37 mg (0.272 mmol) of HOBT·$H_2O$ and 56 mg (0.272 mmol) of DCC in 3 mL of tetrahydrofuran. The resultant crude matrial was purified using radial chromatography (2mm; gradient eluent of 2-6% methanol in methylene chloride) to provide 40 mg of the desired compound.
Yield: 22%.
$^1H$ NMR ($CDCl_3$): δ 1.14 (s, 9H), 1.20-2.35 (m, 18H), 2.45-2.70 (m, 2H), 2.80-3.10 (m, 5H), 3.25-3.55 (m, 1H), 3.95-4.10 (m, 2H), 4.38-4.78 (m, 3H), 5.79 (br.s, 1H), 6.75-7.40 (m, 10H), 8.60 (br.s, 2H).
IR ($CHCl_3$) : 3600-3100, 3008, 2929, 2865, 1728, 1669, 1635, 1604, 1513, 1482, 1455, 1447, 1393, 1367, 1275, 1117, 1076, 910 $cm^{-1}$.
MS (FD): m/e 668 ($M^+$, 100).

| Analysis for $C_{40}H_{53}N_5O_4$: | | | |
|---|---|---|---|
| Calcd: | C, 71.93; | H, 8.00; | N, 10.49. |
| Found: | C, 72.10; | H, 8.11; | N, 10.37. |

Example 13

A. 2-[(3'-N-(Methyl)aza-4'-oxo-4'-[3"-methoxycarbonyl-4"-methyl]phenyl)butyl]pyridine

The subtitled compound was prepared substantially in accordance with the procedure detailed in Example 9D, using 803 mg (3.84 mmol) of 2-[2-(N-(methyl)amino)ethyl)pyridine dihydrochloride, 500 mg (1.44 mmol) of the titled compound of Preparation 7, and 2 mL of triethylamine in 10 mL of methylene chloride. The resultant material was purified using radial chromatography (4mm; gradient eluent of 50-100% ethyl acetate in methylene chloride) to provide 497 mg of the desired compound.
Yield: 64%.
$^1H$ NMR ($CDCl_3$): δ 2.62 (s, 3H), 2.84-3.25 (m, 5H), 3.62-4.00 (m, 5H), 6.95-7.98 (m, 6H), 8.38-8.62 (m, 1H).
IR ($CHCl_3$) : 2954, 1723, 1626, 1594, 1571, 1504, 1478, 1436, 1405, 1302, 1259, 1153, 1100, 1088, 1052, 995, 919, 834 $cm^{-1}$.
MS (FD) : m/e 312 ($M^+$, 100).

| Analysis for $C_{18}H_{20}N_2O_3$: | | | |
|---|---|---|---|
| Calcd: | C, 69.21; | H, 6.45; | N, 8.97; |
| Found: | C, 68.97; | H, 6.34; | N, 8.95. |

B. 2-[(3'-N-(Methyl)aza-4'-oxo-4'-[3"-carboxy-4"-methyl]phenyl)butyl]pyridine hydrochloride

The desired subtitled compound was prepared substantially in accordance with the procedure detailed in Example 11D, using 153 mg (0.49 mmol) of the subtitled compound of Example 13A and 36 mg (1.47 mmol) of lithium hydroxide in 10 mL of a 3:1 mixture of tetrahydrofuran and water to provide 134 mg of a hydroscopic white foam which was used without further purification.
$^1H$ NMR (DMSO-$d_6$) : δ 2.40-2.55 (s, 3H), 2.85-3.90 (m, 8H), 7.20-8.85 (m, 7H).
IR ($CHCl_3$): 3800-1800 (br.), 1715, 1631, 1540, 1506, 1474, 1451, 1406, 1242, 1166, 1085, 1044, 1001, 841 $cm^{-1}$.
MS (FD): m/e 299 ($M^+$, 100).

C. [3S-*(3R*,4aR*,8aR*,2'S*,3'R*)*]-2-(2'-Hydroxy-3'-phenylmethyl-4'-aza-5'-oxo-5'-3"-[1'"-oxo-2'"-N-(methyl)aza-4'"-pyrid-2""-ylpropyl]-6"-methyl)phenyl]pentyl decahydroisoquinoline-3-N-*t*-butylcarboxamide

The subtitled compound was prepared substantially in accordance with the procedure detailed in Example 1D, using 69 mg (0.17 mmol) of the subtitled compound of Preparation 2B, 60 mg (0.18 mmol) of the subtitled compound of Example 13B, 23 mg (0.17 mmol) of HOBT·$H_2O$ and 35 mg (0.17 mmol) of DCC in 3 mL of anhydrous tetrahydrofuran to provide 60 mg of the desired compound.
Yield: 53%.
$^1H$ NMR ($CDCl_3$): δ 0.8-3.55 (m, 38H), 3.70-4.10 (m, 2H), 4.35-4.65 (m, 1H), 5.78-5.98 (m, 1H), 6.70-7.40 (m, 10H), 7.42-7.70 (m, 1H), 8.28 (s, 0.5H), 8.55 (s, 0.5H).
IR ($CHCl_3$) : 3691, 3600-3100, 3028, 3005, 2929, 1670, 1627, 1512, 1455, 1395, 1368, 1243, 1049, 910 $cm^{-1}$.
MS (FD): m/e 682 ($M^+$, 100).

| Analysis for $C_{41}H_{55}N_5O_4$: | | | |
|---|---|---|---|
| Calcd: | C, 72.22; | H, 8.13; | N, 10.27. |
| Found: | C, 72.00; | H, 8.15; | N, 10.18. |

Example 14

A. 2-[(2'-N-(Methyl)aza-3'-oxo-3'-[3"-methoxycarbonyl-4"-methyl]phenyl)propyl]pyrazine

The subtitled compound was prepared substantially in accordance with the procedure detailed in Example 11C, using 204 mg (1.66 mmol) of 2-[2-(N-(methyl)amino)methyl)pyrazine dihydrochloride, and 300 mg (0.83 mmol) of the titled compound of Preparation 7 in 10 mL of methylene chloride. The resultant material was purified using radial chromatography (4mm; gradient eluent of 10-30% acetone in methylene chloride) followed by radial chromatography (2mm; gradient eluent of 10-30% acetone in methylene chloride) to provide 212 mg of a light orange oil.
Yield: 85%.
$^1H$ NMR ($CDCl_3$) : δ 2.61 (s, 3H), 3.07 (s, 3H), 3.88 (s, 3H), 4.55-4.95 (m, 2H), 7.20-7.38 (m, 1H), 7.40-7.60 (m, 1H), 8.04 (s, 1H), 8.35-8.80 (m, 1H).
IR ($CHCl_3$): 3022, 3007, 2954, 1724, 1634, 1503, 1483, 1449, 1437, 1402, 1303, 1261, 1238, 1152, 1089, 1077, 1057, 1020 $cm^{-1}$.
MS (FD): m/e 299 ($M^+$, 100).

B. 2-[(2'-N-(Methyl)aza-3'-oxo-3'-[3"-carboxy-4"-methyl]phenyl)propyl]pyrazine

The desired subtitled compound was prepared substantially in accordance with the procedure detailed in Example 11D, using 150 mg (0.50 mmol) of the subtitled compound of Example 14A and 36 mg (1.50 mmol) of lithium hydroxide in 8 mL of a 3:1 mixture of tetrahydrofuran and water to provide 121 mg of an off-white foam.
Yield: 85%.
$^1H$ NMR ($CDCl_3$) : δ 2.65 (s, 3H), 3.11 (s, 3H), 4.55-4.98 (m, 2H), 7.20-7.38 (m, 1H), 7.50-7.62 (m, 1H), 8.18 (s, 1H), 8.40-8.85 (m, 3H).
IR ($CHCl_3$): 3700-2400 (br.), 1700, 1634, 1530, 1504, 1483, 1450, 1402, 1304, 1176, 1152, 1086, 1020, 923 867, 837 $cm^{-1}$.
MS (FD) : m/e 286 ($M^+$, 100).

C. [3S-*(3R*,4aR*,8aR*,2'S*,3'R*)*]-2-(2'-Hydroxy-3'-phenylmethyl-4'-aza-5'-oxo-5'-(3"-[1'"-oxo-2'"-N-(methyl)aza-3'"-pyrazin-2""-ylpropyl]-6"-methyl)phenyl]pentyl decahydroisoquinoline-3-N-*t*-butylcarboxamide

The subtitled compound was prepared substantially in accordance with the procedure detailed in Example 1D, using 75.1 mg (0.187 mmol) of the subtitled compound of Preparation 2B, 59.3 mg (0.208 mmol) of the subtitled compound of Example 14B, 25.3 mg (0.187 mmol) of HOBT·$H_2O$ and 38.6 mg (0.187 mmol) of DCC in 2 mL of anhydrous tetrahydrofuran to provide 89 mg of a white foam.

Yield: 71%.
$^1H$ NMR ($CDCl_3$): δ 1.13 (s, 9H), 1.15-2.30 (s, 19H), 2.47-2.70 (m, 2H), 2.90-3.08 (m, 5H), 3.30-3.50 (m, 1H), 3.95-4.05 (m, 1H), 4.50-4.65 (m, 2H), 4.83 (br.s, 1H), 5.77 (m, 1H), 6.70-6.90 (m, 1H), 7.02-7.38 (m, 8H), 8.48-8.58 (m, 2.5H), 8.71 (s, 0.5H).
IR ($CHCl_3$): 3690, 3618, 3600-3200 (br.), 3025, 3009, 2977, 2929, 1670, 1636, 1513, 1455, 1395, 1368, 1077, 1047, 1020, 879 $cm^{-1}$.
MS (FD): m/e 669 ($M^+$, 100).

Example 15

A. 2-[2-(N-(*t*-butoxycarbonyl)azopropyl]naphthalene

To a solution of 2.0 g (15.25 mmol) of the subtitled compound of Example 9A in 40 mL of anhydrous dimethylformamide, was added 622 mg (15.30 mmol) of 60% sodium hydride. After approximately one hour, 3.38 g (15.30 mmol) of 2-(bromomethyl)naphthalene was added and the resulting reaction mixture was reacted for 5 hours and then poured into a mixture of methylene chloride and water. The resultant layers were separated and the organic phase was washed twice with water, once with saturated sodium carbonate, once with brine, and then dried over sodium sulfate and filtered. The resultant crude material was purified using radial chromatography (6mm; gradient eleunt of 0-5% ethyl acetate in hexane) to provide 2.25 g of a clear oil.
Yield: 54%.
$^1H$ NMR ($CDCl_3$): δ 1.50 (s, 9H), 2.75-2.95 (m, 3H), 4.58 (s, 2H), 7.30-7.90 (m, 7H).
IR ($CHCl_3$) : 3010, 2980, 2932, 1686, 1510, 1482, 1455, 1425, 1394, 1368, 1244, 1147, 1049, 875, 818 $cm^{-1}$.
MS (FD): m/e 271 ($M^+$, 100).

| Analysis for $C_{17}H_{21}NO_2$: | | | |
|---|---|---|---|
| Calcd: | C, 75.25; | H, 7.80; | N, 5.16. |
| Found: | C, 75.42; | H, 7.98; | N, 5.08. |

B. 2-(2-Azopropyl)naphthalene

A solution of 505 mg (1.86 mmol) of the subtitled compound of Example 15A in 10 mL of a 10% solution of trifluoroacetic acid in methylene chloride was reacted for approximately one hour. The resulting reaction mixture was then concentrated to provide a residue. This residue was redissolved in methylene chloride, washed with saturated sodium carbonate, dried over sodium sulfate, filtered, and then concentrated to provide 300 mg of a yellow oil which was used without further purification.
Yield: 94%.
$^1H$ NMR ($CDCl_3$): δ 2.49 (s, 3H), 3.91 (s, 2H), 7.38-7.55 (m, 3H), 7.70-7.90 (m, 4H) .
IR ($CHCl_3$) : 3059, 3011, 2974, 2941, 2852, 2798, 1681, 1602, 1509, 1475, 1440, 1272, 1126, 1095, 909, 894, 857, 819 $cm^{-1}$.
MS (FD): m/e 171 ($M^{+}$' 100) .

C. 2-[(2'-N-(Methyl)aza-3'-oxo-3'-[3"-methoxycabonyl-4"-methyl]phenyl)propyl]naphthalene

The subtitled compound was prepared substantially in accordance with the procedure detailed in Example 11C, using 71 mg (0.42 mmol) of the subtitled compound of Example 15B, and 100 mg (0.28 mmmol) of the titled compound of Preparation 7 in 5 mL of anhydrous methylene chloride. The resultant crude material was purified using radial chromatography (1mm; gradient eluent of 10-35% ethyl acetate in hexane) to provide 79 mg of an oil.
Yield: 81%.
$^1H$ NMR ($CDCl_3$): δ 2.61 (s, 3H), 2.75-3.25 (m, 3H), 3.60-4.00 (m, 3H), 4.55-5.00 (m, 2H), 7.08-8.18 (m, 10H).
IR ($CHCl_3$) : 3029, 3011, 2954, 2930, 1724, 1628, 1503, 1483, 1437, 1404, 1305, 1272, 1247, 1166, 1151, 1089, 1076, 817 $cm^{-1}$.

| Analysis for $C_{22}H_{21}NO_3$: | | | |
|---|---|---|---|
| Calcd: | C, 76.06; | H, 6.09; | N, 4.03; |
| Found: | C, 76.31; | H, 6.37; | N, 3.77. |

D. 2-[(2'-N-(Methyl)aza-3'-oxo-3'-[3"-carboxy-4"-methyl]phenyl)propyl]naphthalene

The subtitled compound was prepared using the procedure detailed in Example 1C, using 51 mg (0.15 mmol) of the subtitled compound of Example 15C and 8 mg (0.30 mmol) of lithium hydroxide in 5 mL of a 3:1 mixture of tetrahydrofuran and water to provide 48 mg of an off-white solid which was used without further purification. Yield: 96%.

$^1H$ NMR (DMSO-$d_6$): δ 2.38-2.60 (m, 3H), 2.76-3.00 (m, 3H), 4.50-4.90 (m, 2H), 7.18-8.00 (m, 10H).

MS (FD): m/e 347 ($M^+$, 100).

E. [3S-*(3R*,4aR*,8aR*,2'S*,3'R*)*]-2-(2'Hydroxy-3'-phenylmethyl-4'-aza-5'-oxo-5'-(3"-[3"-[1'"-oxo-2'"-N-(methyl)aza-3'"-naphth-2""-ylpropyl]-6"-methyl)phenyl]pentyl decahydroisoquinoline-3-N-*t*-butylcarboxamide

The subtitled compound was prepared substantially in accordance with the procedure detailed in Example 1D, using 46 mg (0.11 mmol) of the subtitled compound of Preparation 2B, 38 mg (0.11 mmol) of the subtitled compound of Example 15D, 15 mg (0.11 mmol) of HOBT·$H_2O$ and 23 mg (0.11 mmol) of DCC in 2 mL of anhydrous tetrahydrofuran and 0.2 mL of anhydrous dimethylformamide. The resultant crude matrial was purified using radial chromatography (2mm; gradient eluent of 0-5% methanol in methylene chloride) to provide 54 mg of a white foam.

Yield: 68%.

$[\alpha]_D$ -65.35 (c = 1.01, MeOH).

$^1H$ NMR ($CDCl_3$): δ 1.13 (s, 9H), 1.15-2.35 (m, 15H), 2.45-3.10 (m, 6H), 3.20-3.55 (m, 1H), 3.82-4.10 (m, 2H), 4.50-4.65 (m, 2H), 4.88 (br.s, 1H), 5.69 (br.s, 1H), 6.50-6.83 (m, 1H), 6.95-7.90 (m, 15H).

IR ($CHCl_3$): 3622, 3600-3100 (br.), 3024, 3008, 2976, 2929, 1670, 1630, 1512, 1455, 1394, 1368, 1246, 1200, 1076, 1047, 910, 819 $cm^{-1}$.

MS (FD): m/e 718 ($M^+$ +1), 616 (100).

| Analysis for $C_{45}H_{56}N_4O_4$: | | | |
|---|---|---|---|
| Calcd: | C, 75.39; | H, 7.87; | N, 7.81; |
| Found: | C, 75.32; | H, 7.88; | N, 8.04. |

## Example 16

A. 1-[(2'-N-(Methyl)aza-3'-oxo-3'-[3"-methoxycarbonyl-4"-methyl]phenyl)propyl]naphthalene

The subtitled compound was prepared substantially in accordance with the procedure detailed in Example 9D, using 57 mg (0.56 mmol) of triethylamine, 87 mg (0.42 mmol) of N-methyl-1-naphthalene methylamine hydrochloride, and 100 mg (0.28 mmol) of the titled compound of Preparation 7 in 5 mL of anhydrous methylene chloride. The resultant crude material was purified using radial chromatography (2mm; gradient eluent of 30-50% ethyl acetate in hexane) to provide 97 mg of a light yellow oil.

Yield: 100%.

$^1H$ NMR ($CDCl_3$): δ 2.56 (br.s, 3H), 2.70-3.30 (m, 3H), 3.60-4.00 (m, 3H), 4.85-5.40 (m, 2H), 7.10-8.30 (m, 10H).

IR ($CHCl_3$): 3029, 3010, 1723, 1623, 1511, 1483, 1436, 1405, 1304, 1252, 1222, 1212, 1210, 1192, 1090, 1076, 804 $cm^{-1}$.

MS (FD): m/e 347 ($M^+$, 100).

| Analysis for $C_{22}H_{21}NO_3$: | | | |
|---|---|---|---|
| Calcd: | C, 76.06; | H, 6.09; | N, 4.03; |
| Found: | C, 76.33; | H, 6.28; | N, 4.14. |

B. 1-[(2'-N-(Methyl)aza-3'-oxo-3'-[3"-carboxy-4"-methyl]phenyl)propyl]naphthalene

The subtitled compound was prepared using the procedure detailed in Example 1C, using 89 mg (0.26 mmol) of the subtitled compound of Example 16A and 13 mg (0.52 mmol) of lithium hydroxide in 8 mL of a 3:1 mixture of tetrahydrofuran and water, to provide 86 mg of a white foam which was used without further purification.

Yield: 100%.

$^1H$ NMR ($CDCl_3$) : δ 2.64 (s, 3H), 2.70-3.25 (m, 3H), 4.80-5.30 (m, 2H), 7.05-8.35 (m, 10H), 8.70 (br.s, 1H).

IR ($CHCl_3$) : 3700-2300 1700, 1623, 1504, 1489, 1450, 1404, 1263, 1199, 1078, 1045, 878 $cm^{-1}$.

MS (FD): m/e 333 ($M^+$, 100) .

| Analysis for $C_{21}H_{19}NO_3$: | | | |
|---|---|---|---|
| Calcd: | C, 75.66; | H, 5.74; | N, 4.20; |
| Found: | C, 75.91; | H, 5.96; | N, 4.22. |

C. [3S-*(3R*,4aR*,8aR*,2'S*,3'R*)*]-2-[2'-Hydroxy-3'-phenylmethyl-4'-aza-5'-oxo-5'-(3"-[1"'-oxo-2"'-N-(methyl)aza-3"'-naphth-1""-ylpropyl]-6"-methyl)phenyl]pentyl decahydroisoquinoline-3-N-*t*-butylcarboxamide

The titled compound was prepared substantially in accordance with the procedure detailed in Example 1D, using 80 mg (0.20 mmol) of the subtitled compound of Preparation 2B, 66 mg (0.20 mmol) of the subtitled compound of Example 16B, 27 mg (0.20 mmol) of HOBT·$H_2O$ and 42 mg (0.20 mmol) of DCC in 5 mL of anhydrous tetrahydrofuran and 0.5 mL of anhydrous dimethylformamide. The resultant crude material was purified using radial chromatography (2mm; gradient eluent of 0-4% methanol in methylene chloride) to provide 86 mg of a white foam.

Yield: 86%.

$^1H$ NMR ($CDCl_3$ ): δ 1.13 (s, 9H), 1.10-4.60 (m, 28H), 4.80-5.40 (m, 2H), 5.65 (m, 1H), 6.60-7.60 (m, 13H), 7.75-7.95 (m, 2H), 8.10-8.25 (m, 1H).

IR ($CHCl_3$) : 3600-3100 (br.), 3028, 3009, 2929, 1670, 1631, 1512, 1455, 1394, 1368, 1259, 1199, 1075, 1047, 910, 837 $cm^{-1}$.

MS (FD): m/e 718 ($M^+$ +1), 616 (100).

| Analysis for $C_{45}H_{56}N_4O_4$: | | | |
|---|---|---|---|
| Calcd: | C, 75.39; | H, 7.87; | N, 7.81; |
| Found: | C, 75.59; | H, 7.79; | N, 7.63. |

Example 17

A. [(2'-N-(Methyl)aza-3'-oxo-3'-(3"-methoxycarbonyl-4"-methylphenyl)propyl]benzene

The subtitled compound was prepared substantially in accordance with the procedure detailed in Example 9D, using N-methyl-N-benzylamine, and the titled compound of Preparation 7 in anhydrous methylene chloride.

$^1H$ NMR ($CDCl_3$): δ 2.59 (s, 3H), 2.75-3.15 (m, 3H), 3.70-3.95 (m, 3H), 4.40-4.80 (m, 2H), 7.00-7.55 (m, 7H), 8.02 (s, 1H).

IR ($CHCl_3$): 3030, 3010, 1723, 1628, 1497, 1454, 1437, 1404, 1359, 1303, 1242, 1195, 1165, 1089, 836

cm$^{-1}$.
MS (FD): m/e 297 ($M^+$, 100).

| Analysis for $C_{18}H_{19}NO_3$: | | | |
|---|---|---|---|
| Calcd: | C, 72.71; | H, 6.44; | N, 4.71; |
| Found: | C, 72.72; | H, 6.49; | N, 4.88. |

B. [(2'-N-(Methyl)aza-3'-oxo-3'-(3"-carboxy-4"-methylphenyl)propyl]benzene

The subtitled compound was prepared substantially in accordance with the procedure detailed in Example 1C, using 110 mg (0.37 mmol) of the subtitled compound of Example 17A, and 18 mg (0.74 mmol) of lithium hydroxide in 10 mL of a 3:1 mixture of tetrahydrofuran and water with the exception that when the reaction was substantially complete, the reaction mixture was partitioned between 10 mL of 1N hydrochloric acid and 10 mL of ethyl acetate. The resultant layers were separated and the aqueous phase was extracted with 10 mL of ethyl acetate. The ethyl acetate extracts were then washed with brine, dried over sodium sulfate and filtered. The resultant crude material was purified using radial chromatography (4mm; gradient eluent of 10-50% methanol in methylene chloride) to provide 87 mg of a white solid.
Yield: 83%.
$^1H$ NMR ($CD_3OD$): δ 2.51 (s, 3H), 2.75-3.10 (m, 3H), 4.40-4.85 (m, 2H), 6.90-7.90 (m, 8H).
IR ($CHCl_3$): 3800-2300 (br.), 1616, 1573, 1497, 1454, 1402, 1379, 1261, 1101, 1077, 833 cm$^{-1}$.
MS (FD): m/e 283 ($M^+$, 100).

C. [3S-*(3R*,4aR*,8aR*,2'S*,3'R*)*]-2-(2'-Hydroxy-3'-phenylmethyl-4'-aza-5'-oxo-5'-(3"-[1'"-oxo-2'"-N- (methyl)aza-3'"-phenylpropyl]-6"-methyl)phenyl]pentyl decahydroisoquinoline-3-N-*t*-butylcarboxamide

The subtitled compound was prepared substantially in accordance with the procedure detailed in Example 1D, using 99 mg (0.25 mmol) of the subtitled compound of Preparation 2B, 70 mg (0.25 mmol) of the subtitled compound of Example 17B, 33 mg (0.25 mmol) of HOBT·$H_2O$, and 51 mg (0.25 mmol) of DCC in 3 mL of anhydrous tetrahydrofuran and 1 mL of anhydrous dimethylformamide. The resultant crude material was purified using radial chromatography (4mm; eluent of 2% methanol in methylene chloride) to provide 108 mg of a white foam.
Yield: 66%.
$^1H$ NMR ($CDCl_3$): δ 1.17 (s, 9H), 1.20-3.10 (m, 26H), 3.25-3.55 (m, 1H), 3.90-4.17 (m, 2H), 4.35-4.80 (m, 2H), 5.71 (br.s, 1H), 6.55-6.80 (m, 1H), 7.00-7.42 (m, 11H), 8.00 (s, 1H).
IR ($CHCl_3$): 3600-3100 (br.), 3011, 2931, 2861, 1672, 1631, 1515, 1455, 1391, 1368, 1250, 1079, 1047, 910 cm$^{-1}$.
MS (FD) : m/e 668 ($M^+$, 100).

| Analysis for $C_{41}H_{54}N_4O_4$: | | | |
|---|---|---|---|
| Calcd: | C, 73.84; | H, 8.16; | N, 8.40; |
| Found: | C, 73.81; | H, 8.39; | N, 8.35. |

Example 18

A. [(2'-Aza-3'-oxo-3'-(3"-methoxycarbonyl-4"-methylphenyl)propyl]benzene

The subtitled compound was prepared substantially in accordance with the procedure detailed in Example 11C, using 150 mg (0.416 mmol) of the titled compound of Preparation 7, and 67 mg (0.62 mmol) of benzylamine in 5 mL of methylene chloride. The resultant material was purified using radial chromatography (1mm; eluent of 50% hexane in ethyl acetate) to provide 99 mg of the subtitled compound.
Yield: 84%.
IR ($CHCl_3$) : 3452, 3031, 3012, 2954, 2954, 1724, 1660, 1612, 1522, 1491, 1454, 1437, 1400, 1309, 1249, 1191, 1152, 1083, 1029 cm$^{-1}$.

MS (FD): m/e 283 ($M^+$, 100).

B. [(2'-Aza-3'-oxo-3'-(3"-carboxy-4"-methylphenyl)propyl]benzene

The subtitled compound was prepared using the procedure detailed in Example 1C, using 66 mg (0.23 mmol) of the subtitled compound of Example 18A and 17 mg (0.70 mmol) of lithium hydroxide in 5 mL of a 3:1 mixture of tetrahydrofuran and water, to provide 64 mg of a white solid which was used without further purification.

Yield: 100%.

$^1H$ NMR ($CDCl_3$): δ 2.60 (s, 3H), 4.50-4.60 (m, 2H), 7.15-7.40 (m, 6H), 7.80-7.90 (m, 1H), 8.39 (s, 1H), 8.98-9.10 (m, 1H).

IR ($CHCl_3$): 4000-2000 (br.), 3296, 3070, 3031, 2977, 2930, 1700, 1636, 1610, 1570, 1545, 1497, 1416, 1310, 1264, 917, 698, 671 $cm^{-1}$.

MS (FD): m/e 269 ($M^+$, 100).

| Analysis for $C_{16}H_{15}NO_3$: | | | |
|---|---|---|---|
| Calcd: | C, 71.36; | H, 5.61; | N, 5.20; |
| Found: | C, 71.42; | H, 5.99; | N, 5.12. |

C. [3S-(*3R*,4aR*,8aR*,2'S*,3'R**)]-2-(2'-Hydroxy-3'-phenylmethyl-4'-aza-5'-oxo-5'-(3"-[1'"-oxo-2'"-aza-3'"-phenylpropyl]-6"-methyl)phenyl]pentyl decahydroisoquinoline-3-N-*t*-butylcarboxamide

The subtitled compound was prepared substantially in accordance with the procedure detailed in Example 1D, using 72 mg (0.18 mmol) of the subtitled compound of Preparation 2B, 49 mg (0.18 mmol) of the subtitled compound of Example 18B, 24 mg (0.18 mmol) of HOBT.$H_2O$, and 37 mg (0.18 mmol) of DCC in 3 mL of anhydrous tetrahydrofuran. The resultant crude material was purified using radial chromatography (1mm; gradient eluent of 0-5% methanol in methylene chloride) to provide 72 mg of a white powder.

$^1H$ NMR ($CDCl_3$): δ 1.06 (s, 9H), 1.15-2.70 (m, 21H), 2.85-3.05 (m, 2H), 3.48-3.55 (m, 1H), 3.75-4.00 (m, 2H), 4.50-4.70 (m, 3H), 5.68 (s, 1H), 6.75-7.42 (m, 12H), 7.76 (m, 1H).

IR ($CHCl_3$): 3429, 3600-3100 (br.), 3009, 2929, 2865, 1657, 1606, 1517, 1497, 1455, 1394, 1368, 1307, 1277, 1145, 1046, 910 $cm^{-1}$.

| Analysis for $C_{40}H_{52}N_4O_4$: | | | |
|---|---|---|---|
| Calcd: | C, 73.59; | H, 8.03; | N, 8.58; |
| Found: | C, 73.31; | H, 7.92; | N, 8.43. |

Example 19

A. 1-(N-(Methyl)-N-(*t*-butoxycarbonyl)amino)methyl-4-fluoro benzene

The subtitled compound was prepared substantially in accordance with the procedure detailed in Example 15A, using 3.0 (22.9 mmol) of the subtitled compound of Example 9A, 3.97 g (27.4 mmol) of 4-fluoro benzylchloride, 604 mg (25.2 mmol) of sodium hydride, 8.2 mg (25.2 mmol) of sodium iodide in 6.0 mL of anhydrous dimethylformamide. The resultant crude material was purified using flash chromatography (gradient eluent of 5-10% ethyl acetate in hexane) to provide 3.74 g of a clear oil.

Yield: 68%.

$^1H$ NMR ($CDCl_3$): δ 1.47 (s, 9H), 2.80 (br.s, 2H), 4.38 (s, 2H), 6.90-7.25 (m, 4H).

IR ($CHCl_3$): 3010, 2981, 2932, 1686, 1607, 1510, 1481, 1455, 1428, 1394, 1368, 1314, 1300, 1294, 1173, 1147, 878, 822 $cm^{-1}$.

MS (FD): m/e 239 ($M^+$), 469 (100).

| Analysis for $C_{13}H_{18}NO_2F$: | | | | |
|---|---|---|---|---|
| Calcd: | C, 65.25; | H, 7.58; | N, 5.85; | F, 7.94; |
| Found: | C, 65.10; | H, 7.48; | N, 5.81; | F, 7.81. |

B. 1-(N-(Methyl)amino)methyl-4-fluoro benzene hydrochloride

The subtitled compound was prepared substantially in accordance with the procedure detailed in Example 9C, using 2.0 g (8.36 mmol) of the subtitled compound of Example 19A, 10 mL of 5N hydrochloric acid in 10 mL of dioxane to provide 1.38 g of a white solid.

Yield: 95%.

MS (FD) m/e 139 ($M^+$ +1, 100).

IR ($CHCl_3$) : 2989, 2934, 2857, 2776, 2711, 2432, 1603, 1516, 1477, 1440, 1422, 1236, 1167, 838, 825, 772, 548, 501 $cm^{-1}$.

C. 1-[(2'-N(Methyl)aza-3'-oxo-3'-(3"-methoxycarbonyl-4"-methylphenyl)propyl]-4-fluorobenzene

The subtitled compound was prepared substantially in accordance with the procedure detailed in Example 9D, using 520 mg (1.44 mmol) of the titled compound of Preparation 7, and 351 mg (2.17 mmol) of the subtitled compound of Example 19B, and 0.5 mL of triethylamine in 5 mL of methylene chloride. The resultant material was purified using flash chromatography (gradient eluent of 20-30% ethyl acetate in hexane) to provide 376 mg of the desired compound.

Yield: 83%.

$^1H$ NMR ($CDCl_3$) : δ 2.61 (s, 3H), 2.80-3.10 (m, 3H), 3.87 (br.s, 3H), 4.40-4.80 (m, 2H), 7.00-7.55 (m, 6H), 8.02 (s, 1H).

IR ($CHCl_3$) : 3029, 3009, 2954, 2931, 1724, 1629, 1511, 1484, 1437, 1404, 1355, 1305, 1157, 1089, 1076, 970, 834, 824 $cm^{-1}$.

MS (FD): m/e 315 ($M^+$, 100).

| Analysis for $C_{18}H_{18}NO_3F$: | | | | |
|---|---|---|---|---|
| Calcd: | C, 68.56; | H, 5.75; | N, 4.44; | F, 6.02; |
| Found: | C, 68.36; | H, 5.89; | N, 4.61; | F, 6.10. |

D. 1-[(2'-N(Methyl)aza-3'-oxo-3'-(3"-carboxy-4"-methylphenyl)propyl]-4-fluorobenzene

The subtitled compound was prepared using the procedure detailed in Example 1C, using 269 mg (0.85 mmol) of the subtitled compound of Example 19C and 102 mg (4.03 mmol) of lithium hydroxide in 10 mL of a 3:1 mixture of tetrahydrofuran and water, to provide 254 mg of a clear amorphous solid.

Yield: 99%.

$^1H$ NMR ($CDCl_3$) : δ 2.68 (s, 3H), 2.80-3.10 (m, 3H), 4.40-4.80 (m, 2H), 7.00-7.60 (m, 6H), 8.17 (s, 1H).

IR ($CHCl_3$): 3700-2300 (br.), 1699, 1628, 1609, 1511, 1484, 1450, 1403, 1355, 1302, 1158, 1097, 1076, 924, 851, 823 $cm^{-1}$.

MS (FD): m/e 301 ($M^+$), 603 (100).

E. [3S-*(3R*,4aR*,8aR*,2'S*,3'R*)*]-2-(2'-Hydroxy-3'-phenylmethyl-4'-aza-5'-oxo-5'-(3"-[1'"-oxo-2'"-N-(methyl)aza-3'"-(4""-fluorophenyl)propyl]-6"-methyl)phenyl]pentyl decahydroisoquinoline-3-N-*t*-butylcarboxamide

The subtitled compound was prepared substantially in accordance with the procedure detailed in Example 1D, using 76 mg (0.19 mmol) of the subtitled compound of Preparation 2B, 57 mg (0.19 mmol) of the subtitled compound of Example 18B, 26 mg (0.19 mmol) of HOBT·$H_2O$, and 39 mg (0.19 mmol) of DCC in anhydrous tetrahydrofuran and anhydrous dimethylformamide. The resultant crude material was purified using flash chromatography (gradient eluent of 1-4% methanol in methylene chloride) to provide 96 mg of the desired com-

pound.
Yield: 74%.
$^1H$ NMR ($CDCl_3$): δ 1.14 (s, 9H), 1.20-2.36 (m, 19H), 2.50-3.10 (m, 6H), 3.35-3.60 (m, 1H), 3.90-4.10 (m, 2H), 4.35-4.75 (m, 3H), 5.68 (s, 1H), 6.65-6.82 (m, 1H), 6.98-7.40 (m, 11H).
IR ($CHCl_3$): 3613, 3600-3100 (br.), 3011, 2977, 2929, 2866, 1670, 1632, 1607, 1511, 1455, 1394, 1368, 1158, 1075, 1047, 879, 823 $cm^{-1}$.
MS (FD): m/e 686 ($M^+$, +1), 584 (100).

Example 20

A. 3-N,N-(Dimethyl)carbamoyl-6-methyl benzoate, methyl ester

The subtitled compound was prepared substantially in accordance with the procedure detailed in Example 9D, using 388 mg (1.08 mmol) of the titled compound of Preparation 7, and 176 mg (2.16 mmol) of dimethylamine hydrochloride,and 0.5 mL of triethylamine in 5 mL of methylene chloride. The resultant material was purified using flash chromatography (eluent of 20% ethyl acetate in hexane) to provide 226 mg of a clear oil.
Yield: 95%.
$^1H$ NMR ($CDCl_3$): δ 2.61 (s, 3H), 2.90-3.20 (m, 6H), 3.88 (s, 3H), 7.24-7.30 (m, 1H), 7.43-7.48 (m, 1H), 7.98 (d, J=1.63 Hz, 1H).
IR ($CHCl_3$): 3029, 3007, 2954, 1723, 1628, 1509, 1487, 1437, 1401, 1304, 1258, 1151, 1099, 1081, 1007, 978, 919, 835 $cm^{-1}$.
MS (FD): m/e 221 ($M^+$, 100).

| Analysis for $C_{12}H_{15}NO_3$: | | | |
|---|---|---|---|
| Calcd: | C, 65.14; | H, 6.83; | N, 6.33; |
| Found: | C, 65.37; | H, 6.73; | N, 6.09. |

B. (3-N,N-Dimethylcarbamoyl-6-methyl)benzoic acid

The subtitled compound was prepared using the procedure detailed in Example 1C, using 186 mg (0.84 mmol) of the subtitled compound of Example 20A and 101 mg (4.20 mmol) of lithium hydroxide in 10 mL of a 3:1 mixture of tetrahydrofuran and water, to provide 158 mg of a white solid.
Yield: 91%.
$^1H$ NMR ($CDCl_3$): δ 2.67 (s, 3H), 2.90-3.40 (m, 6H), 7.32 (d, J=7.83 Hz, 1H), 7.45-7.65 (m, 1H), 8.12 (d, J=1.36 Hz, 1H).
IR ($CHCl_3$): 3700-2300 (br.), 1699, 1628, 1567, 1509, 1487, 1452, 1401, 1302, 1265, 1152, 1099, 1062, 922, 834 $cm^{-1}$.
MS (FD): m/e 208 ($M^+$ +1), 415 (100).

| Analysis for $C_{11}H_{13}NO_3$: | | | |
|---|---|---|---|
| Calcd: | C, 63.76; | H, 6.32; | N, 6.76; |
| Found: | C, 63.89; | H, 6.28; | N, 6.50. |

C. [3S-*(3R*,4aR*,8aR*,2'S*,3'R*)*]-2-(2'-Hydroxy-3'-phenylmethyl-4'-aza-5'-oxo-5'-(3"-[1'"-oxo-1'"-N,N- (dimethyl)amino-methyl]-6"-methyl)phenyl]pentyl decahydroisoquinoline-3-N-*t*-butylcarboxamide

The subtitled compound was prepared substantially in accordance with the procedure detailed in Example 1D, using 165 mg (0.41 mmol) of the subtitled compound of Preparation 2B, 85 mg (0.41 mmol) of the subtitled compound of Example 20B, 56 mg (0.41 mmol) of HOBT·$H_2O$, and 85 mg (0.41 mmol) of DCC in 2 mL of anhydrous tetrahydrofuran. The resultant crude material was purified using radial chromatography (1mm; gradient eluent of 1-5% methanol in methylene chloride) to provide 76 mg of the desired compound.
Yield: 31%.
$^1H$ NMR ($CDCl_3$): δ 1.16 (s, 9H), 1.20-3.50 (m, 29H), 3.95-4.22 (m, 2H), 4.55-4.70 (m, 1H), 5.83 (m, 1H),

6.80-7.55 (m, 8H).

IR ($CHCl_3$) : 3650-3100, 2930, 2866, 1669, 1628, 1513, 1455, 1394, 1368, 1306, 1265, 1147, 1116, 1086, 1048, 910, 837 $cm^{-1}$.

MS (FD): m/e 591 ($M^+$), 490 ($M^+$, 100).

| Analysis for $C_{35}H_{50}N_4O_4$: | | | |
|---|---|---|---|
| Calcd: | C, 71.15; | H, 8.53; | N, 9.48; |
| Found: | C, 71.45; | H, 8.74; | N, 9.44. |

Example 21

[3S-*(3R*,4aR*,8aR*,2'S*,3'S*)*]-2-(2'-Hydroxy-3'-phenylthiomethyl-4'-aza-5'-oxo-5'-(3"-[1'''-oxo-2'''-N-(methyl)aza-3'''-pyrid-2''''-ylpropyl]-6"-methyl)phenyl]pentyl decahydroisoquinoline-3-N-*t*-butylcarboxamide

The subtitled compound was prepared substantially in accordance with the procedure detailed in Example 1D, using 103 mg (0.244 mmol) of the subtitled compound of Preparation 4G, 86 mg (0.27 mmol) of the subtitled compound of Example 9E, 33 mg (0.24 mmol) of HOBT·$H_2O$, 50 mg (0.24 mmol) of DCC, and 102 μL of triethylamine in anhydrous tetrahydrofuran. The resultant crude material was purified using radial chromatography (2mm; gradient eluent of 0-6% methanol in methylene chloride) to provide 147 mg of the desired compound.

Yield: 86%.

$^1H$ NMR ($CDCl_3$): δ 1.05 (s, 9H), 1.10-2.24 (m, 16H), 2.40-2.60 (m, 4H), 2.85-2.96 (m, 1H), 3.04 (s, 3H), 3.37-3.45 (m, 1H), 3.50-3.95 (m, 2H), 4.0-4.10 (m, 1H), 4.35-4.65 (m, 2H), 4.70-5.00 (m, 1H), 5.49 (s, 1H), 7.14-7.50 (m, 9H), 7.53-7.63 (m, 2H), 8.56 (d, J=4.66 Hz, 1H).

IR ($CHCl_3$): 3600-3100 (br.), 3028, 3007, 2976, 2929, 1670, 1633, 1573, 1514, 1481, 1455, 1439, 1394, 1368, 1276, 1247, 1077, 1047 $cm^{-1}$.

MS (FD): m/e 700 ($M^+$, 100).

| Analysis for $C_{35}H_{50}N_4O_4$: | | | |
|---|---|---|---|
| Calcd: | C, 71.15; | H, 8.53; | N, 9.48; |
| Found: | C, 71.45; | H, 8.74; | N, 9.44. |

Example 22

[3S-*(3R*,4aR*,8aR*,2'S*,3'S*)*]-2-(2'-Hydroxy-3'-phenylthiomethyl-4'-aza-5'-oxo-5'-(3"-[1'''-oxo-2'''-N-(methyl)aza-3'''-pyrid-4''''-ylpropyl]-6"-methyl)phenyl]pentyl decahydroisoquinoline-3-N-*t*-butylcarboxamide

The subtitled compound was prepared substantially in accordance with the procedure detailed in Example 1D, using 56 mg (0.13 mmol) of the subtitled compound of Preparation 4G, 50.0 mg (0.16 mmol) of the subtitled compound of Example 12E, 18 mg (0.13 mmol) of HOBT·$H_2O$, 27 mg (0.13 mmol) of DCC, and 54 μL of triethylamine in 2 mL of tetrahydrofuran. The resultant crude matrial was purified using radial chromatography (1mm; eluent of 5% methanol in methylene chloride) to provide 56 mg of an off-white foam.

Yield: 50%.

$^1H$ NMR ($CDCl_3$) : δ 1.03 (s, 9H), 1.10-2.60 (m, 20H), 2.85-3.05 (m, 4H), 3.35-3.45 (m, 1H), 3.55-3.95 (m, 2H), 4.00-4.10 (m, 1H), 4.38-4.80 (m, 3H), 5.54 (s, 1H), 7.05-7.75 (m, 10H), 8.52-8.60 (m, 2H).

MS (FD): m/e 700 ($M^+$) 599 (100).

Example 23

[3S-*(3R*,4aR*,8aR*,2'S*,3'S*)*]-2-(2'-Hydroxy-3'-naphth-2-ylthiomethyl-4'-aza-5'-oxo-5'-(3"-[1'''-oxo-2'''-N-(methyl)aza-3'''-pyrid-2''''-ylpropyl]-6"-methyl)phenyl]pentyl decahydroisoquinoline-3-N-*t*-butylcarboxamide

The subtitled compound was prepared substantially in accordance with the procedure detailed in Example

1D, using 77 mg (0.16 mmol) of the subtitled compound of Preparation 3G, 38 mg (0.18 mmol) of the subtitled compound of Example 9E, 22 mg (0.16 mmol) of HOBT·$H_2O$, 34 mg (0.16 mmol) of DCC, and 68 µL of triethylamine in 3 mL of anhydrous tetrahydrofuran. The resultant crude material was purified using radial chromatography (2mm; gradient eluent of 0-6% methanol in methylene chloride) followed by radial chromatography (2mm; gradient eluent of 1-4% methanol in methylene chloride) to provide 81.6 mg of the desired compound.
Yield: 67%.
$^1H$ NMR ($CDCl_3$): δ 1.06 (s, 9H), 1.10-2.65 (m, 19H), 2.65-3.05 (m, 4H), 3.42-4.18 (m, 5H), 4.40-4.90 (m, 3H), 5.63 (s, 1H), 7.10-7.90 (m, 13H), 8.53 (d, J=3.13 Hz, 1H).
IR ($CHCl_3$) : 3600-3100 (br.), 3025, 3008, 2929, 1670, 1632, 1572, 1515, 1455, 1394, 1368, 1247, 1147, 1077, 1047, 944, 816 $cm^{-1}$.
MS (FD): m/e 751 ($M^+$ +1, 100).

| Analysis for $C_{44}H_{55}N_5O_4S$: | | |
|---|---|---|
| Calcd: | C, 70.46; | H, 7.39; |
| Found: | C, 70.46; | H, 7.19. |

Example 24

[2S-*(2R*,2'S*,3'R*)*]-1-[2'-Hydroxy-3'-phenylmethyl-4'-aza-5'-oxo-5'-(3"-[1'"-oxo-2'"-N-(methyl)aza-3'"-pyrid-2""-ylpropyl]-6"-methyl)phenyl]pentyl-(pyrid-3"-ylmethyl) piperazine-2-N-(*t*-butyl)carboxamide

The subtitled compound was prepared substantially in accordance with the procedure detailed in Example 1D, using 67 mg (0.18 mmol) of the subtitled compound of Preparation 5E, 59 mg (0.15 mmol) of the subtitled compound of Example 9E, 21 mg (0.15 mmol) of HOBT·$H_2O$, 31 mg (0.15 mmol) of DCC, and 64 µL of triethylamine in 3 mL of anhydrous tetrahydrofuran and 0.5 mL of dimethylformamide. The resultant crude material was purified using radial chromatography (1mm; gradient eluent of 5-8% methanol in methylene chloride) to provide 93 mg of the desired compound.
Yield: 87%.
$^1H$ NMR ($CDCl_3$) : δ 1.33 (s, 9H), 2.10-3.10 (m, 16H), 3.32-3.55 (m, 3H), 3.90-4.03 (m, 1H), 4.38-4.57 (m, 2H), 4.65-4.92 (m, 2H), 6.23 (d, J=9.26 Hz, 1H), 7.03-7.43 (m, 11H), 7.56-7.91 (m, 3H), 8.50-8.60 (m, 2H).
IR ($CHCl_3$): 3691, 3700-3100 (br.), 3025, 3007, 2976, 1634, 1553, 1512, 1480, 1455, 1427, 1405, 1366, 1237, 1151, 1077, 1048 $cm^{-1}$.

Example 25

[3S-*(3R*,4aR*,8aR*,2'S*,3'R*)*]-2-(2'-Hydroxy-3'- phenylmethyl-4'-aza-5'-oxo-5'-(3"-[1'"-oxo-2'"-N' (methyl)aza-3'"-pyrid-2""-ylpropyl]-6"-methyl)phenyl]pentyl decahydroisoquinoline-3-N-*t*-butylcarboxamide dimethanesulfonate

To a cold (0°C) solution of 100 mg (0.15 mmol) of the titled compound of Example 10 in 2 mL of anhydrous methylene chloride, was added 0.3 mL (0.30 mmol) of 1M methanesulfonic acid (in methylene chloride). The resultant reaction mixture was reduced to dryness under reduced pressure to provide 122.7 mg of the desired compound.

Example 26

[3S-*(3R*,4aR*,8aR*,2'S*,3'S*)*]-2-(2'-Hydroxy-3'-phenylthiomethyl-4'-aza-5'-oxo-5'-(3"-[1'"-oxo-2'"-N- (methyl)aza-3'"-pyrid-2""-ylpropyl]-6"-methyl)phenyl]pentyl decahydroisoquinoline-3-N-*t*-butylcarboxamide dimethanesulfonate

The titled compound was prepared substantially in accordance with the procedure detailed in Example 25, using 30 mg (0.043 mmol) of the titled compound of Example 21 and 172 µL (0.085 mmol) of 0.5M methanesulfonic acid (methylene chloride) in 2 mL of methylene chloride to provide 38 mg of the desired compound.

Example 27

[3S-(*3R*,4aR*,8aR*,2'S*,3'S**)]-2-(2'-Hydroxy-3'-naphth-2-ylthiomethyl-4'-aza-5'-oxo-5'-(3"-[1'''-oxo-2'''-N-(methyl)aza- 3'''-pyrid-2''''-ylpropyl]-6"-methyl)phenyl]pentyl decahydroisoquinoline-3-N-*t*-butylcarboxamide dimethanesulfonate

The titled compound was prepared substantially in accordance with the procedure detailed in Example 25, using 35 mg (0.047 mmol) of the titled compound of Example 21 and 193 µL (0.096 mmol) of 0.5M methanesulfonic acid (methylene chloride) in 2 mL of methylene chloride to provide 35 mg of the desired compound.

Example 28

[2S-(*2R*,2'S*,3'R**)]-1-[2'-Hydroxy-3'-phenylmethyl-4'-aza-5'-oxo-5'-(3"-[1'''-oxo-2'''-N-(methyl)aza-3'''-pyrid-2''''-ylpropyl]-6"-methyl)phenyl]pentyl-(pyrid-3"-ylmethyl) piperazine-2-N-(*t*-butyl)carboxamide trimethanesulfonate

The titled compound was prepared substantially in accordance with the procedure detailed in Example 25, using 35.9 mg (0.051 mmol) of the titled compound of Example 21 and 312 µL (0.156 mmol) of 0.5M methanesulfonic acid (methylene chloride) in 1 mL of methylene chloride to provide 50 mg of the desired compound.

As noted above, the compounds of the present invention are useful for inhibiting HIV protease, which is an enzyme associated with viral component production and assembly. An embodiment of the present invention is a method of treating or preventing HIV infection comprising administering to a primate in need thereof an effective amount of a compound of formula I or a pharmaceutically acceptable salt thereof. Another embodiment of the present invention is a method of treating or preventing AIDS comprising administering to a primate in need thereof an effective amount of a compound of formula I or a pharmaceutically acceptable salt thereof. A further embodiment of the present invention is a method of inhibiting HIV replication comprising administering to an HIV infected cell, a cell susceptible to HIV infection or a primate in need thereof, an effective amount of a compound of formula I or a pharmaceutically acceptable salt thereof.

The term "effective amount" as used herein, means an amount of a compound of formula I which is capable of inhibiting the HIV protease mediated viral component production and assembly. The HIV protease inhibition contemplated by the present method includes either therapeutic or prophylactic treatment, as appropriate. The specific dose of compound administered according to this invention to obtain therapeutic or prophylactic effects will, of course, be determined by the particular circumstances surrounding the case, including, for example, the compound administered, the route of administration, the condition being treated and the individual being treated. A typical daily dose will contain a dosage level of from about 0.01 mg/kg to about 50 mg/kg of body weight of an active compound of this invention. Preferred daily doses generally will be from about 0.05 mg/kg to about 20 mg/kg and ideally from about 0.1 mg/kg to about 10 mg/kg.

The compounds can be administered by a variety of routes including oral, rectal, transdermal, subcutaneous, intravenous, intramuscular and intranasal. The compounds of the present invention are preferably formulated prior to administration. Therefore, another embodiment of the present invention is a pharmaceutical formulation comprising an effective amount of a compound of formula I or a pharmaceutically acceptable salt thereof and a pharmaceutically acceptable carrier, diluent or excipient therefor.

The active ingredient in such formulations comprises from 0.1% to 99.9% by weight of the formulation. By "pharmaceutically acceptable" it is meant that the carrier, diluent or excipient is compatible with the other ingredients of the formulation and not deleterious to the recipient thereof.

The present pharmaceutical formulations can be prepared by known procedures using known and readily available ingredients. In making the compositions of the present invention, the active ingredient will usually be admixed with a carrier, or diluted by a carrier, or enclosed within a carrier which may be in the form of a capsule, sachet, paper or other container. When the carrier serves as a diluent, it may be a solid, semi-solid or liquid material which acts as a vehicle, excipient or medium for the active ingredient. Thus, the compositions can be in the form of tablets, pills, powders, lozenges, sachets, cachets, elixirs, suspensions, emulsions, solutions, syrups, aerosols, (as a solid or in a liquid medium), ointments containing, for example, up to 10% by weight of the active compound, soft and hard gelatin capsules, suppositories, sterile injectable solutions, sterile packaged powders and the like.

The following formulation examples are illustrative only and are not intended to limit the scope of the invention in any way. The term "active ingredient" represents a compound of formula I or a pharmaceutically acceptable salt thereof.

Formulation 1

Hard gelatin capsules are prepared using the following ingredients:

| | Quantity (mg/capsules) |
|---|---|
| Active ingredient | 250 |
| Starch, dried | 200 |
| Magnesium stearate | 10 |
| Total | 460 mg |

Formulation 2

A tablet is prepared using the ingredients below:

| | Quantity (mg/capsule) |
|---|---|
| Active ingredient | 250 |
| Cellulose, microcrystalline | 400 |
| Silicon dioxide, fumed | 10 |
| Stearic acid | 5 |
| Total | 665 mg |

The components are blended and compressed to form tablets each weighing 665 mg.

Formulation 3

An aerosol solution is prepared containing the following components:

| | Weight |
|---|---|
| Active ingredient | 0.25 |
| Methanol | 25.75 |
| Propellant 22 (Chlorodifluoromethane) | 74.00 |
| Total | 100.00 |

The active compound is mixed with ethanol and the mixture added to a portion of the propellant 22, cooled to -30°C and transferred to a filling device. The required amount is then fed to a stainless steel container and diluted with the remainder of the propellant. The valve units are then fitted to the container.

Formulation 4

Tablets, each containing 60 mg of active ingredient, are made as follows:

| | Quantity (mg/tablet) |
|---|---|
| Active ingredient | 60 |
| Starch | 45 |
| Microcrystalline cellulose | 35 |
| Polyvinylpyrrolidone (as 10% solution in water) | 4 |
| Sodium carboxymethyl starch | 4.5 |
| Magnesium stearate | 0.5 |
| Talc | 1 |
| Total | 150 |

The active ingredient, starch and cellulose are passed through a No. 45 mesh U.S. sieve and mixed thoroughly. The aqueous solution containing polyvinylpyrrolidone is mixed with the resultant powder, and the mixture then is passed through a No. 14 mesh U.S. sieve. The granules so produced are dried at 50°C and passed through a No. 18 mesh U.S. sieve. The sodium carboxymethyl starch, magnesium stearate and talc, previously passed through a No. 60 mesh U.S. sieve, are then added to the granules which, after mixing, are compressed on a tablet machine to yield tablets each weighing 150 mg.

Formulation 5

Capsules, each containing 80 mg of active ingredient, are made as follows:

| | Quantity (mg/capsule) |
|---|---|
| Active ingredient | 80 mg |
| Starch | 59 mg |
| Microcrystalline cellulose | 59 mg |
| Magnesium stearate | 2 mg |
| Total | 200 mg |

The active ingredient, cellulose, starch and magnesium stearate are blended, passed through a No. 45 mesh U.S. sieve, and filled into hard gelatin capsules in 200 mg quantities.

Formulation 6

Suppositories, each containing 225 mg of active ingredient, are made as follows:

| | |
|---|---|
| Active ingredient | 225 mg |
| Saturated fatty acid glycerides | 2,000 mg |
| Total | 2,225 mg |

The active ingredient is passed through a No. 60 mesh U.S. sieve and suspended in the saturated fatty acid glycerides previously melted using the minimum heat necessary. The mixture is then poured into a suppository mold of nominal 2 g capacity and allowed to cool.

Formulation 7

Suspensions, each containing 50 mg of active ingredient per 5 mL dose, are made as follows:

| | |
|---|---|
| Active ingredient | 50 mg |
| Sodium carboxymethyl cellulose | 50 mg |
| Syrup | 1.25 mL |
| Benzoic acid solution | 0.10 mL |
| Flavor | q.v. |
| Color | q.v. |
| Purified water to total | 5 mL |

The active ingredient is passed through a No. 45 mesh U.S. sieve and mixed with the sodium carboxymethyl cellulose and syrup to form a smooth paste. The benzoic acid solution, flavor and color are diluted with a portion of the water and added, with stirring. Sufficient water is then added to produce the required volume.

Formulation 8

An intravenous formulation may be prepared as follows:

| | |
|---|---|
| Active ingredient | 100 mg |
| Isotonic saline | 1,000 mL |

The solution of the above ingredients generally is administered intravenously to a subject at a rate of 1 mL per minute.

The following experiment (Fluorescence HIV-1 Protease Inhibitor Assay) was carried out to demonstrate the ability of the compounds of the present invention to inhibit HIV protease.

As used herein, the abbreviations are defined as follows:

BSA - bovine serum albumin
BOC - *t*-butoxycarbonyl
BrZ - 2-bromobenzyloxycarbonyl
2-ClZ - 2-chlorobenzyloxycarbonyl
DCC - dicyclohexylcarbodiimide
DIEA - diisopropylethylamine
DTT - dithiothreitol
EDTA - ethylenediaminetetraacetic acid
FITC - fluorescein isothiocarbamyl
HEPES - 4-(2-hydroxyethyl)-1-piperazineethanesulfonic acid
MES - 4 morpholineethanesulfonic acid
PAM - phenylacetimidomethyl
TAPS - 3-[tris(hydroxymethyl)methyl]amino-1-sulfonic acid
TRIS - tris(hydroxymethyl)aminomethane
TOS - *p*-toluenesulfonyl (tosyl)

I. Preparation of Protease and Gag Fractions

A. Culture of E. coli K12 L507/pHP10D

Lyophils of E. coli K12 L507/pHP10D were obtained from the Northern Regional Research Laboratory, Peoria, Illinois 61604, under the accession number NRRL B-18560 (deposited November 14, 1989). The lyophils were decanted into tubes containing 10 mL LB medium (10 g Bacto-tryptone, 5 g Bacto-yeast extract, and 10 g aqueous sodium chloride per liter; the pH was adjusted to 7.5 and incubated at 32°C, overnight).

A small portion of the overnight culture was placed on LB-agar (LB medium with 15 g/L Bacto-agar) plates containing 12.5 µg/mL tetracycline in a manner so as to obtain a single colony isolate of E. coli K12 L507/pHP10D. The single colony obtained was inoculated into 10 mL of LB medium containing 12.5 µg/mL tetracycline and incubated overnight at 32°C with vigorous shaking. The 10 mL overnight culture was inoculated

into LB medium containing 12.5 μg/mL tetracycline and incubated at 32°C with vigorous shaking until the culture reached mid-log phase.

B. Culture of E. coli K12 L507/pHGAG

Lyophils of E. coli K12 L507/pHGAG were obtained from the NRRL under the accession number NRRL B-18561 (deposited November 14, 1989). A purified colony of E. coli K 12 L507/pHGAG was isolated, and used as an inoculum for a culture which was grown to mid-log phase in substantial accordance with the teaching of Step A, above, for E. Coli K12 L507/pHP10D.

C. Preparation of Protease Fraction

A culture of E. coli K12 L507/pHP10D was grown to mid-log phase at 32°C in LB media containing 12.5 μg/mL tetracycline. The cultivation temperature was quickly elevated to 40°C to induce gene expression, and the cells were allowed to grow for 2.5 hours at this temperature before the culture was quickly chilled on ice. The cells were centrifuged and the cell pellet was resuspended in 20 mL of 50 mmol MES buffer (pH 6.0) containing 1 mmol EDTA, 1 mmol DTT, 1 mmol PMSF and 10% glycerol ("Buffer A"). Cells were lysed by sonication using a Fischer Model 300 Dismembrator and a microtip probe. Following centrifugation at 27,000 x g, the supernatant was diluted to a total volume of 60 mL with Buffer A and loaded onto a 2.0x19 cm QAE-Sepharose column (1 mL/min, 4°C), that had been equilibrated in Buffer A. The column was washed isocratically for 180 min and then eluted with a gradient eluent of 0-1.0M aqueous sodium chloride in Buffer A over 120 min. Enzymatic activity was measured by HPLC using the synthetic peptide Ser-Gln-Asn-Tyr-Pro-Ile-Val as described in Margolin et al., Biochem. Biophys. Res. Commun., 167, 554-560 (1990); the production of the pl peptide (Ser-Gln-Asn-Tyr) was measured.

The active fractions were combined, made 1.2M in ammonium sulfate, and applied to a 2.0x18 cm hexyl agarose column that had been equilibrated in Buffer A containing 1.2M ammonium sulfate. The sample was loaded at a flow rate of 1 mL/min at 4°C, washed with the equilibration buffer for 240 min (1 mL/min) and then eluted using a reverse linear gradient of 1.2-0M ammonium sulfate in Buffer A for 120 min at the same flow rate. The column was then washed isocratically in Buffer A for 120 min.

The active fractions were combined, concentrated to 10 mL using an Amicon stirred cell with a YM-10 membrane and then applied to a MonoS cation exchange column (1.0x10 cm) that had been equilibrated in Buffer A. The sample was loaded at a flow rate of 1 mL/min at 25°C. After washing isocratically for 30 min, the protease was eluted using a linear gradient of 0-0.45M aqueous sodium chloride in Buffer A over 40 min.. The column was washed isocratically in Buffer A containing 0.45M aqueous sodium chloride for 30 minutes.

The active fractions were combined and concentrated to 200 μL using an Amicon stirred cell and a YM-10 membrane and then the protease was applied to a Superose 6 size exclusion column equilibrated in Buffer A containing 0.1M aqueous sodium chloride. The column was washed isocratically in this buffer at a flow rate of 0.5 ml/min, following which the HIV protease was eluted as a single peak.

QAE-Sepharose, and hexyl agarose were purchased from Sigma Chemical Company. Superose 6 and MonoS were were purchased from Pharmacia. Buffers and reagents were obtained from Sigma.

D. Preparation of Gag Fraction

In an analogous manner, a culture of E. coli K12 507/pHGAG was grown to mid-log phase at 32°C then shifted to 40°C for about 4 to 5 hours. The culture was chilled on ice and centrifuged, then the pellet was resuspended in 8 mL lysis buffer containing 5 mg/mL lysozyme. Lysis buffer was comprised of 50 mM Tris-HCl (pH 7.8), 5 mM EDTA, 1 mM DTT, 100 mM NaCl, 1 μg/mL E64 and 2 μg/mL aprotinin. The culture was incubated about 30 to 60 minutes at 4°C, then briefly sonicated in a Branson® Cell Disrupter at 60% power, for three 20 second bursts with chilling between each burst. The culture was then centrifuged at 15,000 x g. The supernatant, which contains the unprocessed gag protein, was partially purified by size exclusion chromatography on a Sephadex G-50 column and stored at -20°C in 50% glycerol and lysis buffer.

II. Preparation of Substrate: $N^{\alpha}$-Biotin-Gly-Ser-Gln-Asn-Tyr-Pro-Ile-Val-Gly-Lys($N^{\varepsilon}$-FITC)-OH SEQ ID NO:1.

A. Preparation of $N^{\alpha}$-Biotin-Gly-Ser-Gln-Asn-Tyr-Pro-IleVal-Gly-Lys-OH SEQ ID NO:3.

The protected peptide-resin $N^{\alpha}$-Boc-Gly-Ser-Gln-Asn-Tyr(BrZ)-Pro-Ile-Val-Gly-Lys(2-ClZ)-$OCH_2$-PAM-resin SEQ ID NO:2. was synthesized on an Advanced Chemtech Model 200 peptide synthesizer at 1.5 mmol

scale using the standard double-couple protocol. The amino terminal Boc group was removed with 50% trifluoroacetic acid in methylene chloride and the resulting resin neutralized with 5% diisopropylethylamine (DIEA) in methylene chloride. Then, 1.1 g (4.5 mmol) of biotin in 20 mL of dimethylsulfoxide was added to the peptide resin, followed by 4.5 mmol of dicyclohexyl-carbodiimide (DCC) in 9 mL of methylene chloride. The resulting reaction mixture was diluted to 40 mL total volume using 11 mL of methylene chloride, and then allowed to react for approximately 5 hours. The reaction solution was concentrated, the resin washed sequentially with dimethyl sulfoxide, dimethylformamide and methylene chloride and then neutralized with 5% DIEA in methylene chloride. This reaction was repeated twice, with the reaction time being extended to 12 hours per reaction. Ninhydrin analysis of the resin indicated complete reaction of the biotin with the glycine amine group. The final peptide resin was washed extensively with dimethylformamide and methylene chloride and dried to provide 4.3 g (98%).

B. Deprotection

The peptide was deprotected and cleaved from the resin using 50 mL of a hydrofluoric acid/*m*-cresol solution, 0°C, 1 hour. After removal of the hydrofluoric acid by vacuum distillation, the *m*-cresol was extracted from the reaction mixture using 100 mL of diethylether. The peptide was then solubilized in 50% aqueous acetic acid, frozen and lyophilized to provide 2.14 g.

C. Purification

The crude $N^{\alpha}$-Biotin-Gly-Ser-Gln-Asn-Tyr-Pro-Ile-Val-Gly-Lys-OH SEQ ID NO:3. was dissolved in 200 mL of a 5% acetonitrile in water solution containing 0.1% trifluoroacetic acid and then filtered through a 0.22 micron filter. The resulting solution was applied to a 2.2x25 cm. reverse phase column of octadecyl-silica (Vydac C-18) which had been equilibrated with the same buffer. The peptide was eluted using an 855 minute linear gradient of 7.5-25% acetonitrile, at 2 mL/minute, with collection of fractions. These fractions were analyzed using Analytical HPLC was performed on a 4.6x250 mm Vydac C-18 column using similar buffer conditions. The fractions containing the desired material were combined, frozen and lyophilized to provide 1.206 g (62%).

Amino acid analysis of the isolated $N^{\alpha}$-Biotin-Gly-Ser-Gln-Asn-Tyr-Pro-Ile-Val-Gly-Lys-OH SEQ ID NO:3. gave the following ratios: Asn 1.1; Ser 0.96; Gln 1.1; Pro 1.1; Gly 2.1; Val 0.80; Ile 0.78; Tyr 1.1; Lys 1.1; in agreement with theory. Fast-atom bombardment mass spectrometry gave a molecular ion mass peak of 1288, in agreement with theory.

D. Labeling

The purified peptide was labeled with a fluorescent marker at the C-terminal end for use in the Pandex assay. $N^{\alpha}$-Biotin-Gly-Ser-Gln-Asn-Tyr-Pro-Ile-ValGly-Lys-OH SEQ ID NO:3 (1.206 g, 0.936 mmol) was dissolved in 100 mL of 0.1<u>M</u> sodium borate, pH 9.5. Then, a solution of 3 g (7.7 mmol) of fluorescein isothiocyanate in 15 mL of dimethylsulfoxide was added to the reaction mixture in 10 equal portions over two hours. The resulting mixture was allowed to react for one hour after the final addition. The solution was adjusted to pH 3 using 5<u>N</u> hydrochloric acid, resulting in the formation of a precipitate which was removed by centrifugation.

The peptide solution was then adjusted to pH 7.8 using 5<u>N</u> sodium hydroxide and then diluted to 200 mL total volume by the addition of 0.1<u>M</u> ammonium acetate, pH 7.5. The resulting solution was then filtered through a 0.22 micron filter and loaded onto a 2.2x25 cm column of Vydac C-18 which had been equilibrated with of 5% acetonitrile in 0.1<u>M</u> ammonium acetate (pH 7.5). The peptide was eluted from the column using an 855 minute linear gradient of 5-25% acetonitrile, at 2 mL/minute, with collection of fractions. Analytical HPLC was used to analyze the fractions. The fractions containing the desired product were then combined, frozen and lyophilized to provide 190.2 mg (12%).

Amino acid analysis of the purified peptide gave the following: Asn 1.1; Ser 1.0; Gln 1.1: Pro 1.1; Gly 2.1; Val 0.8; Ile 0.8; Tyr 1.1; Lys 1.0; in agreement with theory. Fast-atom bombardment mass spectrometry gave amolecular ion mass peak of 1678, in agreement with theory.

E. Fluorescence HIV-1 Protease Inhibitor Assay

The following buffers and solutions are used in the Fluorescence HIV-1 Protease Inhibitor Assay:

| | |
|---|---|
| MES-ALB Buffer: | 0.05<u>M</u> 4-morpholineethane sulfonic acid, pH 5.5<br>0.02<u>M</u> NaCl |

| | |
|---|---|
| | 0.002M EDTA |
| | 0.001M DTT |
| | 1.0 mg/mL BSA |
| TBSA Buffer: | 0.02M TRIS |
| | 0.15M NaCl |
| | 1.0 mg/mL BSA |
| Avidin Coated Beads Solution: | 0.1% solution of Fluoricon Avidin Assay Particles (Avidin conjugated to solid polystyrene beads, 0.6-0.8 microns in diameter in TBSA Buffer |
| Enzyme Solution: | 27 IU/mL of purified HIV-1 protease in MES-ALB buffer (1 IU equals the amount of enzyme required to hydrolyze 1 μmole of substrate per minute at 37°C |

To each well of a round bottom, 96-well plate is added 20 μL of the Enzyme Solution followed by 10 μL of the compound to be evaluated in a 20% aqueous dimethylsulfoxide solution. Purified HIV-1 protease was obtained as described above. The resulting solution is incubated for one hour at room temperature and then 20 μL of a solution containing the substrate, $N^{\alpha}$-Biotin-Gly-Ser-Gln-Asn-Tyr-Pro-Ile-Val-Gly-Lys($N^{\epsilon}$-FITC)-OH SEQ ID NO:1, in MES-ALB buffer (1.5 μl/mL) is added to each well. The solutions are then incubated for 16 hours at room temperature and then each well is diluted with 150 μL of MES-ALB buffer.

To each well of a second round bottom, 96-well Pandex plate is added 25 uL of the Avidin Coated Beads Solution. Then, to each well is added 25 μL of the diluted incubation solutions, prepared above. The solutions are mixed thoroughly and the plates are loaded into a Pandex® machine, washed, evacuated and read. Sample detection was performed by excitation at 485 nm, reading the resulting epifluorescence at 535 nm.

The $IC_{50}$ results obtained in the Fluorescence Assay for the compounds of the present invention are set forth below in Table 1. All values have been normalized to a positive control which is [1*S*- *(1R*, 4R*, 5S*)*]-*N*-(1-(2-amino-2-oxoethyl)-2-oxo-3-aza-4-phenylmethyl-5-hydroxy-6-(2-(1-*t*-butylamino-1-oxomethyl)phenyl)hexyl)-2-quinolinyl carboxamide.

Table 1

Inhibitory Activity of Formula I Compounds

| Example No. | Fluorescence Assay $IC_{50}$ in ng/mL |
|---|---|
| Control | 1.0 |
| 1 | 3.6 |
| 2 | 300 |
| 3 | 1000 |
| 4 | 35 |
| 5 | 400 |
| 6 | 2.7 |
| 7 | 0.7 |
| 8 | 28 |
| 9 | 19.1* |
| 10 | 5.7* |
| 11 | 4.5 |
| 12 | 5 |
| 13 | 13.4 |
| 14 | 16.1 |
| 15 | 6.6* |
| 16 | 8.4* |
| 17 | 5.6* |
| 18 | 278* |
| 19 | 5.3 |
| 20 | 185 |
| 21 | 14.8* |
| 22 | 2.8 |
| 23 | 11.3 |
| 24 | 4.6 |

* Average $IC_{50}$.
N.T. Not Tested

Table 1, continued

Inhibitory Activity of Formula I Compounds

| Example No. | Fluorescence Assay $IC_{50}$ in ng/mL |
|---|---|
| 25 | N.T. |
| 26 | N.T. |
| 27 | N.T. |
| 28 | N.T. |

* Average $IC_{50}$.
N.T. Not Tested

**Claims**

1. A compound of formula I

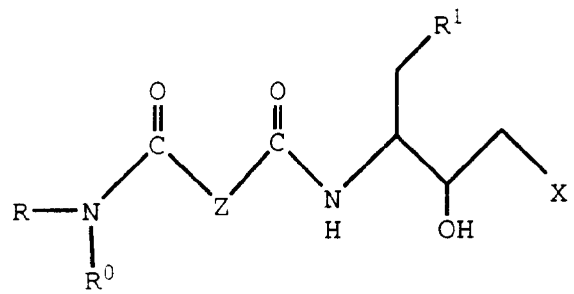

I

wherein:

R and $R^0$ are independently selected from hydrogen, $C_1$-$C_6$ alkyl, aryl($C_1$-$C_4$)alkyl, heterocycle($C_1$-$C_4$)-alkyl or unsaturated heterocycle($C_1$-$C_4$)alkyl;

or R and $R^0$ are combined with the nitrogen atom to which they are attached to form a heterocycle or unsaturated heterocycle with the proviso that the nitrogen atom may not be quaternized;

Z is a group having the structure:

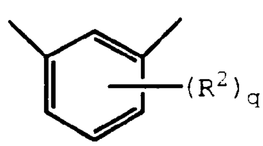 , 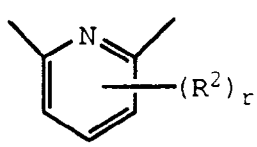 , 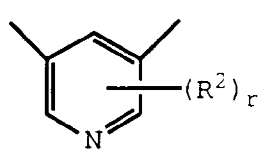 ,

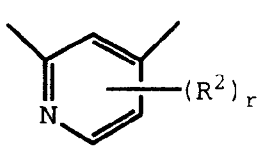 , 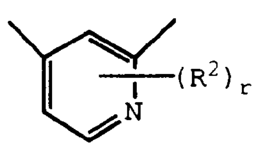 , 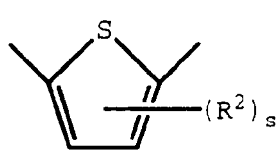 ,

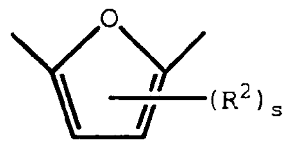 or 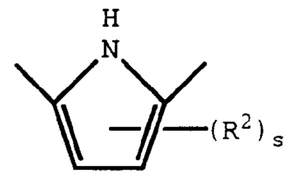 ;

q is 0, 1, 2, 3 or 4;
r is 0, 1, 2 or 3;
s is 0, 1 or 2;

$R^2$ is hydrogen, halo, $C_1$-$C_6$ alkyl, aryl($C_1$-$C_4$)alkyl, $C_1$-$C_6$ alkoxy, $C_1$-$C_6$ alkylthio, amino, $C_1$-$C_4$ alkylamino, di($C_1$-$C_4$)alkylamino, carbamoyl or N-($C_1$-$C_4$)alkylcarbamoyl;

$R^1$ is aryl, heterocycle, unsaturated heterocycle, $C_5$-$C_7$ cycloalkyl or -S-$R^{1a}$, where $R^{1a}$ is aryl, heterocycle, unsaturated heterocycle or $C_5$-$C_7$ cycloalkyl;

X is a group having the structure:

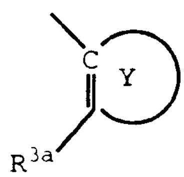 , 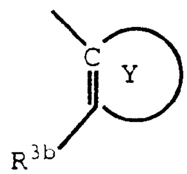 or 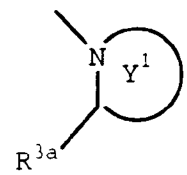 ;

Y is aryl or unsaturated heterocycle;
$Y^1$ is heterocycle;
$R^{3a}$ is a group having the structure:
1) -C(O)-$NR^4R^4$,

2) 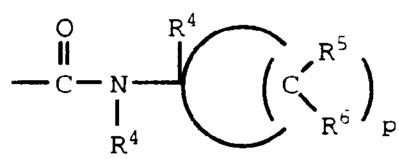 ,

or

3) 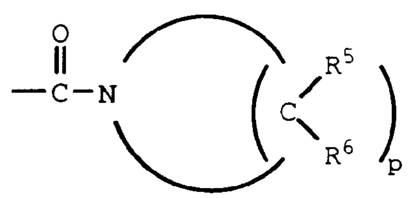 ;

$R^{3b}$ is a group having the structure:

1) 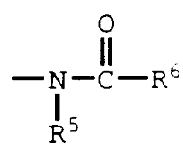 ,

2) 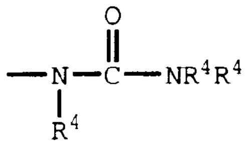 ,

or

3) 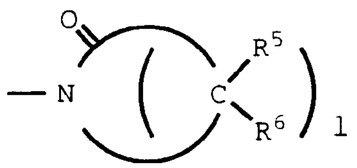 ;

p is 4 or 5;
l is 3, 4 or 5;
$R^4$ at each occurrence is independently hydrogen, $C_1$-$C_6$ alkyl or hydroxy($C_1$-$C_4$)alkyl; and
$R^5$ and $R^6$ are independently selected from hydrogen, hydroxy, $C_1$-$C_6$ alkyl, $C_1$-$C_6$ alkoxy, amino, $C_1$-$C_4$ alkylamino, hydroxy($C_1$-$C_4$)alkyl, carboxy, $C_1$-$C_4$ alkoxycarbonyl, carbamoyl, N-($C_1$-$C_4$)alkylcarbamoyl, aryl, heterocycle or unsaturated heterocycle; or a pharmaceutically acceptable salt thereof.

**2.** A compound according to claim 1 wherein:
Z is

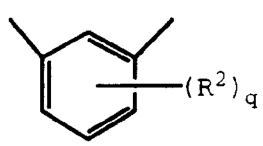 ;

$R^1$ is aryl or -S-$R^{1a}$ where $R^{1a}$ is aryl;
$R^2$ is hydrogen or $C_1$-$C_6$ alkyl;
q is 0 or 1;
X is

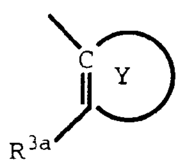 or 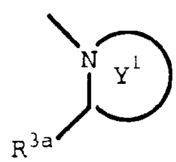 ;

and
$R^{3a}$ is a group having the structure -C(O)-NR$^4$R$^4$, where $R^4$ is as defined above;
or a pharmaceutically acceptable salt thereof.

**3.** A compound according to claim 2 wherein:
Y is phenyl;
$Y^1$ is decahydro-(4aS, 8aS)-isoquinolinyl; and
$R^{3a}$ is -C(O)NH(*t*-butyl);
or a pharmaceutically acceptable salt thereof.

**4.** [3S- *(3R*, 4aR*, 8aR*, 2'S*, 3'R*)*]-2-[2'-Hydroxy-3'-phenylmethyl-4'-aza-5'-oxo-5'-(3"-[1'"-oxo-2'"-N-(methyl)aza-3'"-quinolin-2""-ylpropyl]-6"-methyl)phenyl]pentyl-decahydroisoquinoline-3-N-*t*-butylcarboxamide; [3'"S- *(3'"R*, 4'"S*)]*-N-[1'-oxo-1'-(3"-[1'"-oxo-2'"-aza-3'"-phenylmethyl-4'"-hydroxy-5'"-(2""-

N-*t*-butylcarbamido)-phenyl]pentyl-4"-methyl)phenyl]methyl-1,2,3,4-tetrahydroisoquinoline; or [3S-*(3R*,4aR*,8aR*,2'S*,3'R*)]*-2-[2'-hydroxy-3'-phenylmethyl-4'- aza-5'-oxo-5'-(3"-[1''',2''',3''',4'''-tetrahydroisoquinolin-1'''-ylcarbonyl]-6"-methyl)-phenyl]pentyl decahydroisoquinoline-3-N-*t*-butylcarboxamide; or a pharmaceutically acceptable salt thereof.

**5.** A pharmaceutical formulation comprising a compound of formula, or a pharmaceutically acceptable salt thereof, as claimed in any one of Claims 1 to 4, associated with one or more pharmaceutically acceptable carriers, diluents or excipients therefor.

**6.** A compound of formula I, or a pharmaceutically acceptable salt thereof, as claimed in any one of claims 1 to 4, for use as an antiviral.

**7.** A compound of formula I, or a pharmaceutically acceptable salt thereof, as claimed in any one of claims 1 to 4, for use in the inhibition of HIV replication.

**8.** A process for preparing a compound of formula I

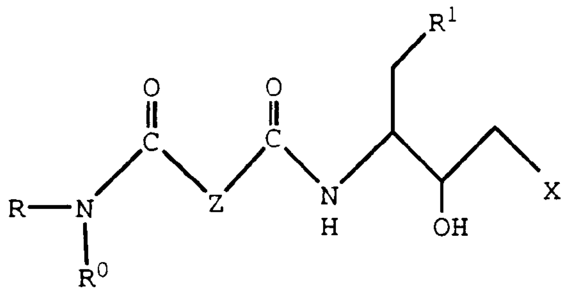

I

or a pharmaceutically acceptable salt thereof, as claimed in any one of claims 1 to 4, comprising:

a) reacting a compound of formula IA

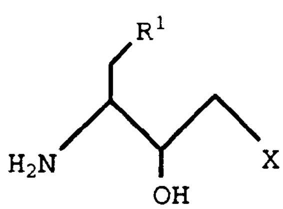

(IA)

with a compound of the formula of formula IB

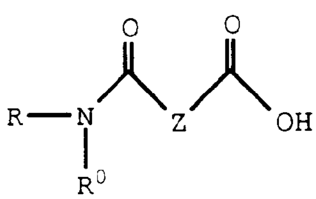

(IB)

or an activated derivative therof;

where R, $R^0$, $R^1$, X, and Z are as defined above; and

b) optionally converting the resulting product into a pharmaceutically acceptable salt thereof.

European Patent Office

# EUROPEAN SEARCH REPORT

Application Number
EP 93 31 0355

## DOCUMENTS CONSIDERED TO BE RELEVANT

| Category | Citation of document with indication, where appropriate, of relevant passages | Relevant to claim | CLASSIFICATION OF THE APPLICATION (Int.Cl.5) |
|---|---|---|---|
| A | EP-A-0 432 695 (F.HOFFMANN-LA ROCHE A.G.)<br>* the whole document * | 1-8 | C07D217/06<br>C07D401/12<br>C07D217/14<br>C07D215/12<br>C07D241/42<br>C07D213/40<br>A61K31/50<br>A61K37/02 |
| A | EP-A-0 361 341 (MOLECULAR THERAPEUTICS, INC.)<br>* the whole document * | 1-8 | |
| A | WO-A-91 08221 (WISCONSIN ALUMNI RESEARCH FOUNDATION)<br>* examples 5.1- 3, 6.1, 9.1- 10.2 * | 1-8 | |
| A | SCIENCE,<br>vol.248, no.4953, 1990, LANCASTER, PA US<br>pages 358 - 361<br>N.A. ROBERTS ET AL.<br>* table 1 * | 1-8 | |
| A | CHEMICAL ABSTRACTS, vol. 114, no. 15, 15 April 1991, Columbus, Ohio, US;<br>abstract no. 143998q,<br>D.H. RICH ET AL.<br>* abstract *<br>& J. MED. CHEM.,<br>vol.34, no.3, 1991<br>pages 1222 - 1225 | 1-8 | TECHNICAL FIELDS SEARCHED (Int.Cl.5)<br>C07D<br>A61K<br>C07K |

The present search report has been drawn up for all claims

| Place of search | Date of completion of the search | Examiner |
|---|---|---|
| BERLIN | 22 April 1994 | Frelon, D |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)